# EUROPÄISCHE PATENTSCHRIFT

(11) **EP 2 697 225 B1**
(45) Veröffentlichungstag und Bekanntmachung des Hinweises auf die Patenterteilung: **07.10.2015**
(21) Anmeldenummer: 12710445.3
(22) Anmeldetag: 15.03.2012
(51) Int. Cl.: C07D 471/14, C07D 487/04, C07D 487/22, C07D 498/04, C07D 513/04, H01L 51/00, H01L 51/50

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 13.04.2011 EP 11003106
(43) Veröffentlichungstag der Anmeldung: 19.02.2014
(73) Patentinhaber: Merck Patent GmbH, 64293 Darmstadt (DE)
(72) Erfinder: MUJICA-FERNAUD, Teresa, 64289 Darmstadt (DE); PFLUMM, Christof, 60316 Frankfurt am Main (DE); MARTYNOVA, Irina, 64347 Griesheim (DE)
(86) Internationale Anmeldenummer: PCT/EP2012/001156
(87) Internationale Veröffentlichungsnummer: WO 2012/139692

(56) Entgegenhaltungen:
- US-A1- 2010 051 928
- DATABASE REGISTRY [Online] CHEMICAL ABSTRACTS SERVICE, COLUMBUS, OHIO, US; 14. März 2001 (2001-03-14), XP002676517, gefunden im STN Database accession no. 327080-21-7
- DATABASE REGISTRY [Online] CHEMICAL ABSTRACTS SERVICE, COLUMBUS, OHIO, US; 13. Dezember 2000 (2000-12-13), XP002676518, gefunden im STN Database accession no. 308298-97-7
- LLOYD J. DOLBY ET AL: "2-Alkylidene-2H-indole intermediates. Thermolysis of 2-hydroxydiphenylmethylindole", THE JOURNAL OF ORGANIC CHEMISTRY, Bd. 34, Nr. 10, 1. Oktober 1969 (1969-10-01), Seiten 2988-2993, XP55027856, ISSN: 0022-3263, DOI: 10.1021/jo01262a042
- YU. M. VOLOVENKO ET AL: "Synthesis of condensed pyrrolo[b]pyrazines", CHEMISTRY OF HETEROCYCLIC COMPOUNDS, Bd. 35, Nr. 9, 1. September 1999 (1999-09-01), Seiten 1089-1093, XP55027759, ISSN: 0009-3122, DOI: 10.1007/BF02251802
- FURSY TAMARA ET AL: "Synthesis of novel heterocyclic systems from a-(2-R-5-halogenopyrimidin-4-yl)-2-azaheta rylacetonitriles. New potential anticancer agents", ELECTRONIC CONFERENCE ON HETEROCYCLIC CHEMISTRY : ECHET '98 ; 29 JUNE 19980629; 19980629 - 19980724 LONDON : IMPERIAL COLLEGE PRESS, GB, 29. Juni 1998 (1998-06-29), Seiten 1-3, XP009159618, ISBN: 981-02-3594-1
- DATABASE CA [Online] CHEMICAL ABSTRACTS SERVICE, COLUMBUS, OHIO, US; SHMEISS, N. A. M. M. ET AL: "Antimitotic agents: synthesis of new heterocyclic and other derivatives of 1,2,3,4-tetrahydro-9H-carbazole", XP002676987, gefunden im STN Database accession no. 1999:653553 & SHMEISS, N. A. M. M. ET AL: "Antimitotic agents: synthesis of new heterocyclic and other derivatives of 1,2,3,4-tetrahydro-9H-carbazole", AL-AZHAR JOURNAL OF PHARMACEUTICAL SCIENCES , 22, 82-99 CODEN: AAJPFT; ISSN: 1110-1644, 1998,
- Kenya Kawashima ET AL: "Synthesis of dibenzo[b,f]cycloprop[d]azepine derivatives. II. Introduction of a cyclopropane ring by the dichloromethylene transfer reaction", Chemical & Pharmaceutical Bulletin, 1. Januar 1978 (1978-01-01), Seiten 942-950, XP55027853, Gefunden im Internet: URL:http://ci.nii.ac.jp/els/110003623070.p df?id=ART0004128622&type=pdf&lang=en&host= cinii&order_no=&ppv_type=0&lang_sw=&no=133 7860423&cp= [gefunden am 2012-05-22]
- BARBERO N ET AL: "Divergent synthesis of isoindolo[2,1-a]indole and indolo[1,2-a]indole through copper-catalysed C- and N-arylations", 6. Mai 2009 (2009-05-06), TETRAHEDRON LETTERS, ELSEVIER, AMSTERDAM, NL, PAGE(S) 2129 - 2131, XP026011897, ISSN: 0040-4039 [gefunden am 2009-02-26] in der Anmeldung erwähnt Seite 2130; Verbindung 2
- DATABASE PUBCHEM COMPOUNDS [Online] CID 9928634, 25 Oktober 2006 Database accession no. CID 9928634

## Beschreibung

Die vorliegende Erfindung betrifft eine elektronische Vorrichtung enthaltend eine oder mehrere Verbindungen einer unten definierten Formel (I) oder (II). Weiterhin umfasst die Erfindung die Verwendung einer Verbindung gemäß Formel (I) oder (II) in einer elektronischen Vorrichtung, sowie die Bereitstellung bestimmter, unten definierter Verbindungen gemäß Formel (I) oder (II).

Die Entwicklung neuartiger funktioneller Verbindungen zur Verwendung in elektronischen Vorrichtungen ist aktuell Gegenstand intensiver Forschung. Ziel ist hierbei die Entwicklung und Untersuchung von Verbindungen, welche bislang noch nicht in elektronischen Vorrichtungen eingesetzt wurden, sowie die Entwicklung von Verbindungen, welche ein verbessertes Eigenschaftsprofil der Vorrichtungen ermöglichen.

Unter dem Begriff elektronische Vorrichtung werden gemäß der vorliegenden Erfindung unter anderem organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Feld-Quench-Devices (OFQDs), organische lichtemittierende elektrochemische Zellen (OLECs), organische Laserdioden (O-Laser) und organische Elektrolumineszenzvorrichtungen (OLEDs) verstanden.

Der Aufbau organischer Elektrolumineszenzvorrichtungen (OLEDs), in denen die Verbindungen gemäß Formel (I) oder (II) bevorzugt als funktionelle Materialien eingesetzt werden können, ist dem Fachmann bekannt und unter anderem in US 4539507, US 5151629, EP 0676461 und WO 1998/27136 beschrieben.

Betreffend die Leistungsdaten der organischen Elektrolumineszenzvorrichtungen sind, insbesondere in Hinblick auf eine breite kommerzielle Verwendung, noch weitere Verbesserungen erforderlich. Von besonderer Bedeutung sind in diesem Zusammenhang die Lebensdauer, die Effizienz und die Betriebsspannung der organischen Elektrolumineszenzvorrichtungen sowie die realisierten Farbwerte. Insbesondere bei blau emittierenden Elektrolumineszenzvorrichtungen besteht Verbesserungspotential bezüglich der Lebensdauer der Vorrichtungen.

Zudem ist es wünschenswert, dass die Verbindungen zur Verwendung als organische Halbleitermaterialien eine hohe thermische Stabilität und eine hohe Glasübergangstemperatur aufweisen und sich unzersetzt sublimieren lassen.

Es besteht in diesem Zusammenhang unter anderem Bedarf an alternativen Matrixmaterialien zur Verwendung in elektronischen Vorrichtungen. Insbesondere besteht Bedarf an Matrixmaterialien für phosphoreszierende Emitter, die gleichzeitig zu guter Effizienz, hoher Lebensdauer und geringer Betriebsspannung führen. Gerade die Eigenschaften der Matrixmaterialien sind häufig limitierend für die Lebensdauer und die Effizienz der organischen Elektrolumineszenzvorrichtung.

Gemäß dem Stand der Technik werden häufig Carbazolderivate, z. B. Bis(carbazolyl)biphenyl, als Matrixmaterialien verwendet. Hier besteht noch Verbesserungspotential insbesondere in Bezug auf die Lebensdauer und die Glasübergangstemperatur der Materialien. Weiterhin besteht Verbesserungsbedarf in Bezug auf die Betriebsspannung der elektronischen Vorrichtungen enthaltend die betreffenden Materialien.

Auch hoch-kondensierte Stickstoff-enthaltend Heterocyclen werden als Matrixmaterialien verwendet wie in US 2010/0051928.

Weiterhin werden Ketone (WO 2004/093207), Phosphinoxide, Sulfone (WO 2005/003253) sowie Triazinverbindungen wie Triazinylspirobifluoren (vgl. die Anmeldungen WO 2005/053055 und WO 2010/015306) als Matrixmaterialien für phosphoreszierende Emitter verwendet. Hier besteht noch Verbesserungspotential insbesondere in Bezug auf die Effizienz und die Kompatibilität mit Metallkomplexen, welche Ketoketonat-Liganden enthalten, beispielsweise Acetylacetonat.

Weiterhin werden Metallkomplexe, beispielsweise BAlq oder Bis[2-(2-benzothiazol)phenolat]-zink(II), als Matrixmaterialien für phosphoreszierende Emitter verwendet. Hier besteht noch Verbesserungsbedarf insbesondere in Bezug auf die Betriebsspannung und die chemische Stabilität. Rein organische Verbindungen sind häufig stabiler als diese Metallkomplexe. So sind einige dieser Metallkomplexe hydrolyseempfindlich, was die Handhabung der Komplexe erschwert.

Von besonderem Interesse ist weiterhin die Bereitstellung von alternativen Materialien als Matrixkomponenten von Mixed-Matrix-Systemen. Unter einem Mixed-Matrix-System wird im Sinne dieser Anmeldung ein System verstanden, in dem zwei oder mehr verschiedene Matrixverbindungen zusammen mit einer (alternativ auch mehreren) Dotandverbindungen gemischt als emittierende Schicht verwendet werden. Diese Systeme sind insbesondere von Interesse bei phosphoreszierenden organischen Elektrolumineszenzvorrichtungen. Für detailliertere Informationen wird auf die Anmeldung WO 2010/108579 verwiesen. Als im Stand der Technik bekannte Verbindungen als Matrixkomponenten in Mixed-Matrix-Systemen sind unter anderem CBP (Biscarbazolylbiphenyl) und TCTA (Triscarbazolyltriphenylamin) zu nennen. Es besteht jedoch weiterhin Bedarf an alternativen Verbindungen zur Verwendung als Matrixkomponenten in Mixed-Matrix-Systemen. Insbesondere besteht Bedarf an Verbindungen, welche eine Verbesserung der Betriebsspannung, der Leistungseffizienz und der Lebensdauer der elektronischen Vorrichtungen bewirken.

Weiterhin besteht Bedarf an alternativen Lochtransportmaterialien zur Verwendung in elektronischen Vorrichtungen. Bei Lochtransportmaterialien gemäß dem Stand der Technik steigt im Allgemeinen die Spannung mit der Schichtdicke der Lochtransportschicht an. In der Praxis wäre häufig eine höhere Schichtdicke der Lochtransportschicht wünschenswert, dies hat jedoch oftmals eine höhere Betriebsspannung und schlechtere Leistungsdaten zur Folge. In diesem Zusammenhang besteht Bedarf an neuen Lochtransportmaterialien, die eine hohe Ladungsträgerbeweglichkeit aufweisen, so dass dickere Lochtransportschichten mit lediglich geringem Anstieg der Betriebsspannung realisiert werden können.

In den Anmeldungen WO 2010/136109 und WO 2011/000455 werden Indenocarbazol- und Indolocarbazolderivate mit unterschiedlicher Verknüpfungsgeometrie der Inden- bzw. Indol- und der Carbazoleinheit offenbart. Die Verbindungen eignen sich zur Verwendung als Funktionsmaterialien in organischen Elektrolumineszenzvorrichtungen, insbesondere als Matrixmaterialien für phosphoreszierende Emitter sowie als Elektronentransportmaterialien. Es besteht jedoch weiterhin Bedarf an alternativen Verbindungen, insbesondere solchen, mit denen eine Senkung der Betriebsspannung, eine Erhöhung der Leistungseffizienz sowie eine Erhöhung der Lebensdauer erreicht werden kann.

Im Rahmen der vorliegenden Erfindung wurde nun gefunden, dass sich Verbindungen der unten angegebenen Formel (I) oder (II) hervorragend zur Verwendung in elektronischen Vorrichtungen eignen.

Gegenstand der vorliegenden Erfindung ist somit eine elektronische Vorrichtung, enthaltend Anode, Kathode sowie mindestens eine organische Schicht, wobei die organische Schicht mindestens eine Verbindung der Formel (I) oder (II) enthält 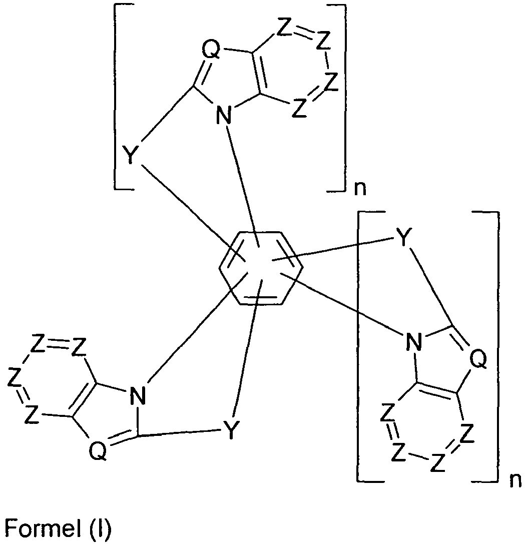 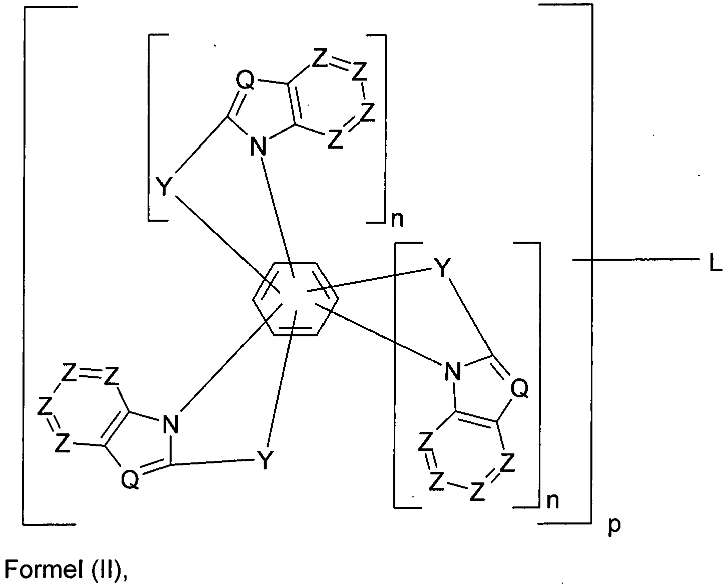 wobei gilt:
- Y: gleich C(R¹)₂;
- Q, Z: sind CR¹;
- L: ist ausgewählt aus C=O, C=NR¹, Si(R¹)₂, NR¹, P(=O)(R¹), O, S, SO, SO₂, Alkylengruppen mit 1 bis 20 C-Atomen oder Alkenylen- oder Alkinylengruppen mit 2 bis 20 C-Atomen, wobei bei den genannten Gruppen eine oder mehrere CH₂-Gruppen durch Si(R¹)₂, O, S, C=O, C=NR¹, C(=O)O, (C=O)NR¹, NR¹, P(=O)(R¹), SO oder SO₂ ersetzt sein können und wobei ein oder mehrere H-Atome in den genannten Gruppen durch D, F, Cl, Br, I, CN oder NO₂ ersetzt sein können, und aromatischen oder heteroaromatischen Ringsystemen mit 5 bis 60 aromatischen Ringatomen, die jeweils durch einen oder mehrere Reste R¹ substituiert sein können, und beliebigen Kombinationen aus 1, 2, 3, 4 oder 5 gleichen oder verschiedenen Gruppen ausgewählt aus den oben genannten Gruppen; oder L ist eine Einfachbindung, wobei p in diesem Fall gleich 2 ist;
- R¹: ist bei jedem Auftreten gleich oder verschieden H, D, F, Cl, Br, I, B(OR²)₂, CHO, C(=O)R², CR²=C(R²)₂, CN, C(=O)OR², C(=O)N(R²)₂, Si(R²)₃, N(R²)₂, NO₂, P(=O)(R²)₂, OSO₂R², OR², S(=O)R², S(=O)₂R², eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch -R²C=CR²-, -C=C-, Si(R²)₂, Ge(R²)₂, Sn(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy-, Heteroaryloxy-, Aralkyl- oder Heteroaralkylgruppe mit 5 bis 60 aromatischen Ringatomen, die durch einen oder mehrere Reste R² substituiert sein kann und wobei in der Alkylgruppe auch einzelne H-Atome und/oder CH₂-Gruppen durch die oben genannten Gruppen ersetzt sein können , wobei zwei oder mehr Reste R¹ miteinander verknüpft sein können und einen Ring oder ein Ringsystem bilden können;
- R²: ist bei jedem Auftreten gleich oder verschieden H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C=C-, Si(R³)₂, Ge(R³)₂, Sn(R³)₂, C=O, C=S, C=Se, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die durch einen oder mehrere Reste R² substituiert sein kann und wobei in der Alkylgruppe auch einzelne H-Atome und/oder CH₂-Gruppen durch die oben genannten Gruppen ersetzt sein können, wobei zwei oder mehr Reste R² miteinander verknüpft sein können und einen Ring oder ein Ringsystem bilden können;
- R³: ist bei jedem Auftreten gleich oder verschieden H, D, F oder ein aliphatischer, aromatischer und/oder heteroaromatischer organischer Rest mit 1 bis 20 C-Atomen, in dem auch ein oder mehrere H-Atome durch D oder F ersetzt sein können; dabei können zwei oder mehr Substituenten R³ auch miteinander verknüpft sein und einen Ring oder ein Ringsystem bilden;
- n: ist bei jedem Auftreten gleich oder verschieden 0 oder 1, wobei für den Fall n=0 die in Klammern stehende Gruppe nicht vorliegt und an den zentralen Benzolring gegebenenfalls stattdessen Gruppen R¹ gebunden sind;
- p: ist gleich 2, 3, 4, 5 oder 6; und
wobei die Gruppe Y und das Stickstoffatom an beliebigen benachbarten Positionen am aromatischen Sechsring binden können;
wobei in Formel (II) die an L bindenden Molekülteile in eckigen Klammern mit Index p gleich oder verschieden sein können; und
wobei in Formel (II) die Gruppe L an einer beliebigen Position des in eckigen Klammern gesetzten Molekülteils mit Index p gebunden sein kann.

Eine Arylgruppe im Sinne dieser Erfindung enthält 6 bis 60 C-Atome; eine Heteroarylgruppe im Sinne dieser Erfindung enthält 1 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Eine Aralkylgruppe im Sinne dieser Erfindung ist eine mit einer Arylgruppe substituierte Alkylgruppe, wobei der Begriff Arylgruppe wie oben definiert zu verstehen ist und die Alkylgruppe 1 bis 20 C-Atome aufweist, wobei in der Alkylgruppe auch einzelne H-Atome und/oder CH₂-Gruppen durch die oben bei der Definition von R¹ und R² genannten Gruppen ersetzt sein können und wobei die Alkylgruppe diejenige Gruppe darstellt, welche an den Rest der Verbindung bindet. Entsprechend stellt eine Heteroaralkylgruppe eine mit einer Heteroarylgruppe substituierte Alkylgruppe dar, wobei der Begriff Heteroarylgruppe wie oben definiert zu verstehen ist und die Alkylgruppe 1 bis 20 C-Atome aufweist, wobei in der Alkylgruppe auch einzelne H-Atome und/oder CH₂-Gruppen durch die oben bei der Definition von R¹ und R² genannten Gruppen ersetzt sein können und wobei die Alkylgruppe diejenige Gruppe darstellt, welche an den Rest der Verbindung bindet.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis-oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neo-Pentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung verknüpft sind. Dies wird durch das folgende Schema verdeutlicht:

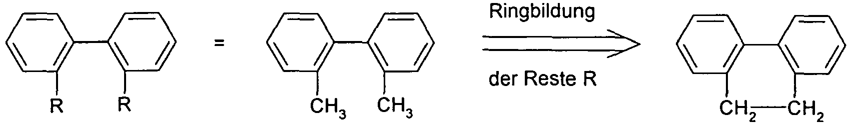

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

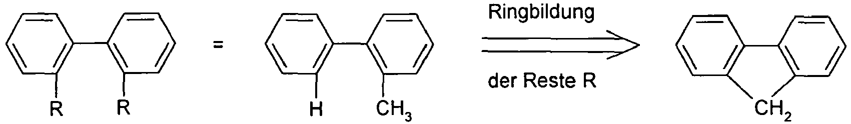

Weiterhin soll hervorgehoben werden, dass die in den zentralen Benzolring gezeichneten Bindungen ausgehend von N und Y von jeder beliebigen freien Position des Benzol ausgehen können. Dabei gilt jedoch, dass es sich um benachbarte Positionen handeln muss, wie oben in der Definition der erfindungsgemäßen Verbindungen angegeben.

Aus der Art der Darstellung in Formel (I) und Formel (II) soll nicht abgeleitet werden, dass N oberhalb von Y am Benzolring binden muss. Die räumliche Anordnung des Indolderivats mit der Brücke Y kann daher im Rahmen der Erfindung frei gewählt werden, solange N und die Gruppe Y in benachbarten Positionen binden.

Bevorzugte Ausführungsformen für die Bindungspositionen von Y und N lassen sich den in einem folgenden Abschnitt abgebildeten bevorzugten Strukturen von Verbindungen der Formel (I) oder (II) entnehmen.

Erfindungsgemäß kann die Gruppe L in Verbindungen der Formel (II) an einer beliebigen Position des in eckigen Klammern gesetzten Molekülteils mit Index p gebunden sein kann. Bevorzugt erfolgt die Anbindung an gleichen Positionen, d. h. symmetrisch. Weiterhin bevorzugt erfolgt die Bindung über die folgenden in untenstehender Formel (II) mit einem Kreis markierten Positionen, wobei im Fall einer Bindung über den zentralen Benzolring maximal einer der beiden Indices n gleich 1 sein kann. Alternativ kann auch eine Anbindung von L über eine Gruppe Y erfolgen.

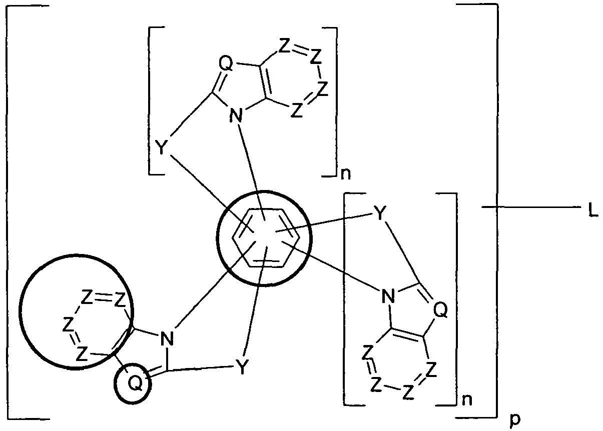

Bevorzugte Ausführungsformen von Formel (II) entsprechen somit den Formeln (II-A) bis (II-C)

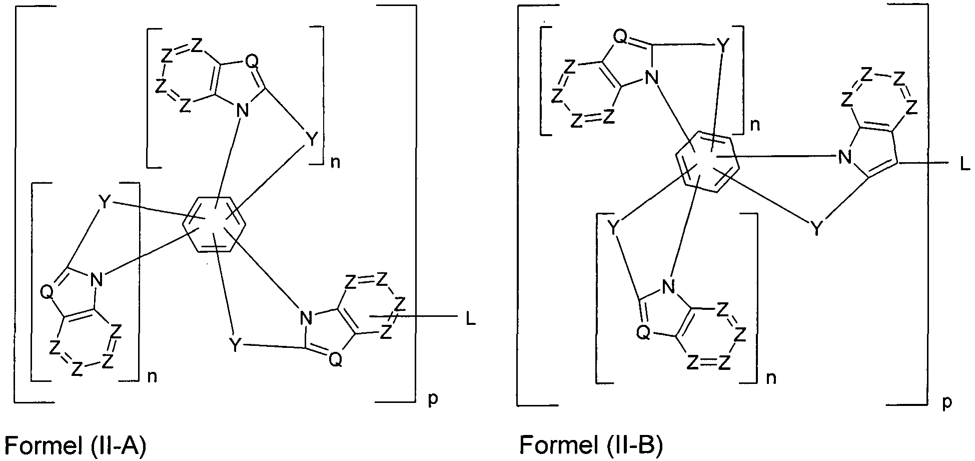

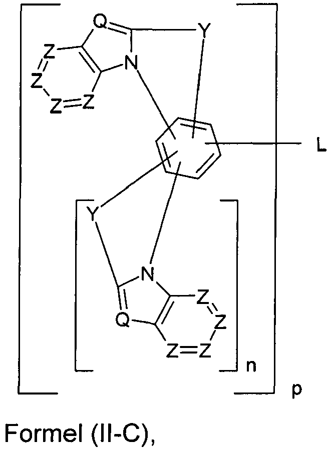

Gemäß einer weiteren bevorzugten Ausführungsform sind die an L bindenden Molekülteile in eckigen Klammern mit Index p in Formel (II) gleich gewählt, d. h. die Verbindung ist symmetrisch.

Weiterhin ist der Index p, der die Anzahl der an L bindenden Molekülteile wiedergibt, bevorzugt gleich 2 oder 3 und besonders bevorzugt gleich 2. Gemäß einer bevorzugten Ausführungsform ist die Summe der Werte für n in Formel (I) gleich 0 oder 1, besonders bevorzugt ist sie gleich 0. Gemäß einer bevorzugten Ausführungsform ist die Summe der Werte für n pro Einheit in eckigen Klammern mit Index p für Formel (II) gleich 0 oder 1, besonders bevorzugt gleich 0.

Bevorzugt ist L ausgewählt aus einer Einfachbindung, wobei in diesem Fall p = 2 ist.

Gleichfalls bevorzugt ist L ausgewählt aus C=O, NR¹, O oder S, wobei in diesen Fällen p=2 ist, oder aus Alkylengruppen mit 1 bis 10 C-Atomen, Alkenylengruppen mit 2 bis 10 C-Atomen, wobei bei den genannten Gruppen eine oder mehrere CH₂-Gruppen durch C=O, NR¹, P(=O)(R¹), O oder S ersetzt sein können, oder aus Arylen- oder Heteroarylengruppen mit 5 bis 20 aromatischen Ringatomen, welche mit einem oder mehreren Resten R¹ substituiert sein können. Der Index p ist in diesen Fällen bevorzugt gleich 2, kann aber auch größer sein, beispielsweise 3.

Gleichfalls bevorzugt ist L ein divalentes aromatisches oder heteroaromatisches Ringsystem der Formel (L-1) 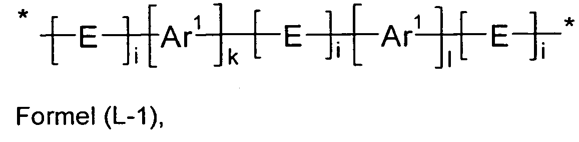 wobei p in diesem Fall gleich 2 ist und wobei weiterhin gilt:
- Ar¹: ist bei jedem Auftreten gleich oder verschieden eine Aryl- oder Heteroarylgruppe mit 5 bis 20 aromatischen Ringatomen, welche jeweils mit einem oder mehreren Resten R¹ substituiert sein kann;
- E: ist bei jedem Auftreten gleich oder verschieden eine Einfachbindung, C=O, NAr¹, P(=O)(R¹), O, S, SO oder SO₂;
- i: ist bei jedem Auftreten gleich oder verschieden 0 oder 1;
- k,l: sind bei jedem Auftreten gleich oder verschieden 0, 1, 2 oder 3, wobei die Summe der Werte von k und I größer als 0 ist; und
wobei weiterhin die Gruppen Ar¹ untereinander über eine oder mehrere divalente Gruppen T verbunden sein können, wobei
- T: bei jedem Auftreten gleich oder verschieden ausgewählt ist aus einer Einfachbindung, BR¹, C(R')₂, C=O, C=S, C=NR¹, C=C(R¹)₂, CR¹=CR¹, Si(R¹)₂, NR¹, PR¹, P(=O)R¹, O, S, S=O und S(=O)₂; und
die Symbole * Bindungen der Gruppe L an den Rest der Verbindung markieren.

Besonders bevorzugt ist L eine Einfachbindung oder eine Arylen- oder Heteroarylengruppe mit 5 bis 18 aromatischen Ringatomen, welche mit einem oder mehreren Resten R¹ substituiert sein kann, oder ein divalentes aromatisches oder heteroaromatisches Ringsystem der Formel (L-1), wobei der Index p gleich 2 ist und wobei einschränkend zu den oben angegebenen Definitionen für Formel (L-1) gilt, dass
- E: bei jedem Auftreten gleich oder verschieden eine Einfachbindung, C=O, NAr¹, O oder S ist;
- k,l: bei jedem Auftreten gleich oder verschieden 0 oder 1 ist, wobei die Summe der Werte von k und l größer als 0 ist; und
- T: bei jedem Auftreten gleich oder verschieden ausgewählt ist aus einer Einfachbindung, C(R¹)₂, C=O, NR¹, O und S.

Bevorzugt ist
- R¹: bei jedem Auftreten gleich oder verschieden ausgewählt aus H, D, F, CN, Si(R²)₃, N(R²)₂ oder einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen, wobei die oben genannten Gruppen jeweils mit einem oder mehreren Resten R² substituiert sein können und wobei in den oben genannten Gruppen eine oder mehrere CH₂-Gruppen durch -C=C-, -R²C=CR²-, Si(R²)₂, C=O, C=NR², -NR²-, -O-, -S-, -C(=O)O- oder -C(=O)NR²- ersetzt sein können, oder ein aromatisches oder heteroaromatisches Ringsystem mit 5 bis 20 aromatischen Ringatomen, das jeweils mit einem oder mehreren Resten R² substituiert sein kann, wobei zwei oder mehr Reste R¹ miteinander verknüpft sein können und einen Ring oder ein Ringsystem bilden können.

Besonders bevorzugt ist
- R¹: bei jedem Auftreten gleich oder verschieden ausgewählt aus H, D, F, N(R²)₂, einer geradkettigen Alkylgruppe mit 1 bis 8 C-Atomen oder einer verzweigten oder cyclischen Alkylgruppe mit 3 bis 8 C-Atomen, wobei die oben genannten Gruppen jeweils mit einem oder mehreren Resten R² substituiert sein können und wobei in den oben genannten Gruppen eine oder mehrere CH₂-Gruppen durch -R²C=CR²-, -NR²-, -O- oder -S- ersetzt sein können, oder eine Aryl- oder Heteroarylgruppe mit 5 bis 20 aromatischen Ringatomen, die jeweils mit einem oder mehreren Resten R² substituiert sein kann.

Bevorzugt ist
- R²: bei jedem Auftreten gleich oder verschieden ausgewählt aus H, D, F, CN, Si(R³)₃, N(R³)₂ oder einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen, wobei die oben genannten Gruppen jeweils mit einem oder mehreren Resten R³ substituiert sein können und wobei in den oben genannten Gruppen eine oder mehrere CH₂-Gruppen durch -C=C-, -R³C=CR³-, Si(R³)₂, C=O, C=NR³, -NR³-, -O-, -S-, -C(=O)O- oder -C(=O)NR³- ersetzt sein können, oder ein aromatisches oder heteroaromatisches Ringsystem mit 5 bis 20 aromatischen Ringatomen, das jeweils mit einem oder mehreren Resten R³ substituiert sein kann, wobei zwei oder mehr Reste R² miteinander verknüpft sein können und einen Ring oder ein Ringsystem bilden können.

Besonders bevorzugt ist
- R²: bei jedem Auftreten gleich oder verschieden ausgewählt aus H, D, F, N(R³)₂, einer geradkettigen Alkylgruppe mit 1 bis 8 C-Atomen oder einer verzweigten oder cyclischen Alkylgruppe mit 3 bis 8 C-Atomen, wobei die oben genannten Gruppen jeweils mit einem oder mehreren Resten R³ substituiert sein können und wobei in den oben genannten Gruppen eine oder mehrere CH₂-Gruppen durch -R³C=CR³-, -NR³-, -O- oder -S- ersetzt sein können, oder eine Aryl- oder Heteroarylgruppe mit 5 bis 20 aromatischen Ringatomen, die jeweils mit einem oder mehreren Resten R³ substituiert sein kann.

Es ist weiterhin bevorzugt, dass die erfindungsgemäßen Verbindungen als Substituent R¹ mindestens eine Gruppe tragen, welche ausgewählt ist aus Gruppen R¹-I, R¹-II und R¹-III, für die gilt:
- R¹-I: ist eine Heteroarylgruppe mit 5 bis 20 aromatischen Ringatomen oder eine Ketogruppe oder eine Phosphoroxidgruppe oder eine Schwefeloxidgruppe, welche jeweils direkt oder über eine oder mehrere divalente Aryl- oder Heteroarylgruppen gebunden ist und welche mit einem oder mehreren Resten R² substituiert sein kann;
- R¹-II: ist ein aromatisches oder heteroaromatisches Ringsystem mit 5 bis 24 aromatischen Ringatomen, welches mit einem oder mehreren Resten R² substituiert sein kann, und
- R¹-III: ist eine Arylamingruppe, welche mit einem oder mehreren Resten R² substituiert sein kann.

Unter einer Ketogruppe, welche direkt oder über eine oder mehrere divalente Arylgruppen gebunden ist und welche mit einem Rest R² substituiert sein kann, wird im Rahmen der vorliegenden Erfindung eine Gruppe der folgenden Formel verstanden 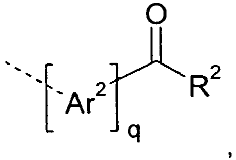 wobei die gestrichelte Bindung die Anbindungsstelle der Ketogruppe darstellt,
- q: gleich 0, 1, 2, 3, 4 oder 5 sein kann,
- Ar²: bei jedem Auftreten gleich oder verschieden eine Aryl- oder Heteroarylgruppe mit 5 bis 20 aromatischen Ringatomen darstellt, welche mit einem oder mehreren Resten R² substituiert sein kann, wobei die Gruppen Ar² untereinander über eine oder mehrere Gruppen U verbunden sein können; und
- U: bei jedem Auftreten gleich oder verschieden ausgewählt ist aus einer Einfachbindung, BR², C(R²)₂, C=O, C=S, C=NR², C=C(R²)₂, CR²=CR², Si(R²)₂, NR², PR², P(=O)R², O, S, S=O und S(=O)₂; und
- R²: wie oben definiert ist.

Unter einer Phosphoroxidgruppe, welche direkt oder über eine oder mehrere divalente Arylgruppen gebunden ist und welche mit einem Rest R² substituiert sein kann, wird im Rahmen der vorliegenden Erfindung eine Gruppe der folgenden Formel verstanden 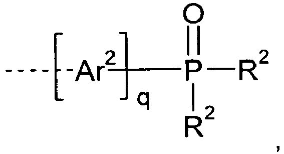 wobei die gestrichelte Bindung die Anbindungsstelle der Phosphoroxidgruppe darstellt und R², q und Ar² wie oben definiert sind, wobei die Gruppen Ar² untereinander über eine oder mehrere Gruppen U, wie oben definiert, verbunden sein können.

Unter einer Schwefeloxidgruppe, welche direkt oder über eine oder mehrere divalente Arylgruppen gebunden ist und welche mit einem Rest R² substituiert sein kann, wird im Rahmen der vorliegenden Erfindung eine Gruppe der folgenden Formel verstanden 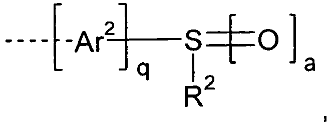 wobei die gestrichelte Bindung die Anbindungsstelle der Schwefeloxidgruppe darstellt,
- a: gleich 1 oder 2 sein kann,
und R², q und Ar² wie oben definiert sind, wobei die Gruppen Ar² untereinander über eine oder mehrere Gruppen U, wie oben definiert, verbunden sein können.

Die oben genannten Gruppen R¹-I stellen bevorzugt Gruppen der folgenden Formel dar 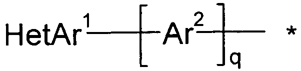 wobei das Symbol * die Bindung zum Rest der Verbindung markiert und weiterhin
q und Ar² wie oben definiert sind, wobei die Gruppen Ar² untereinander über eine oder mehrere Gruppen U, wie oben definiert, verbunden sein können; und
- HetAr¹: eine Heteroarylgruppe mit 5 bis 20 aromatischen Ringatomen darstellt, welche mit einem oder mehreren Resten R² substituiert sein kann.

Bevorzugt ist HetAr¹ ausgewählt aus Pyridin, Pyrimidin, Pyridazin, Pyrazin, Triazin und Benzimidazol, die jeweils mit einem oder mehreren Resten R² substituiert sein können.

Die oben genannten Gruppen R¹-II sind bevorzugt ausgewählt aus Phenyl, Naphthyl, Anthracenyl, Phenanthrenyl, Benzanthracenyl, Pyrenyl, Biphenyl, Terphenyl und Quaterphenyl, welche mit einem oder mehreren Resten R² substituiert sein können.

Die oben genannten Gruppen R¹-III stellen bevorzugt Gruppen der folgenden Formel dar 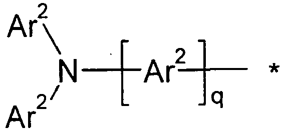 wobei das Symbol * die Bindung zum Rest der Verbindung markiert und weiterhin
q und Ar² wie oben definiert sind, wobei die Gruppen Ar² untereinander über eine oder mehrere Gruppen U, wie oben definiert, verbunden sein können.

Besonders bevorzugte Ausführungsformen von Verbindungen der Formel (I) sind die im Folgenden dargestellten Formeln: 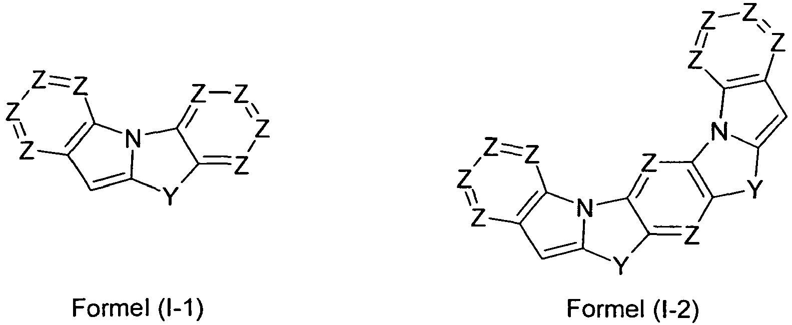 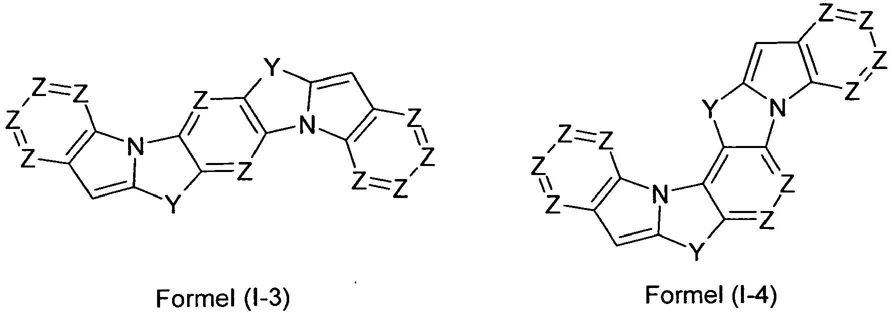 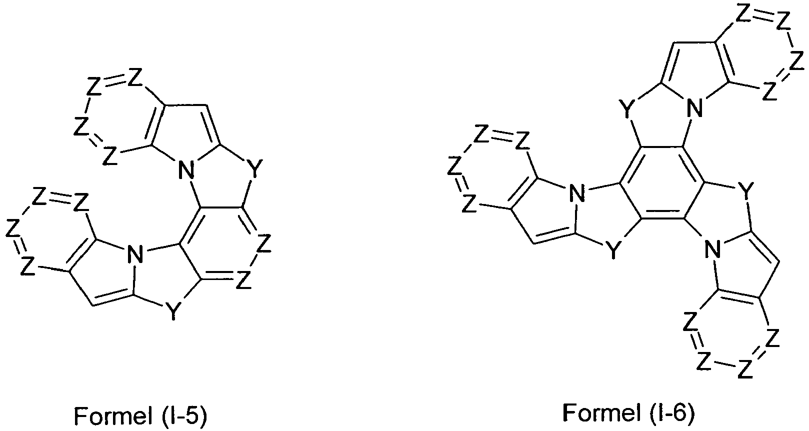 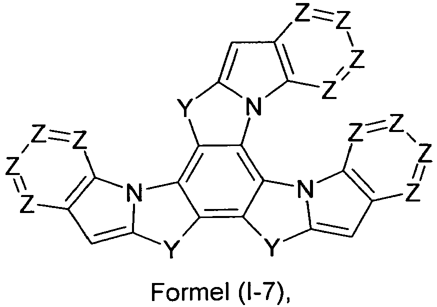 wobei die auftretenden Symbole wie oben definiert sind und weiterhin die Verbindungen an allen unsubstituierten Positionen mit Resten R¹ substituiert sein können.

Ganz besonders bevorzugt sind Verbindungen der Formeln (I-1), (I-2) und (I-3).

Besonders bevorzugte Ausführungsformen von Verbindungen der Formel (II-A), (II-B) und (II-C) sind die im Folgenden dargestellten Formeln: 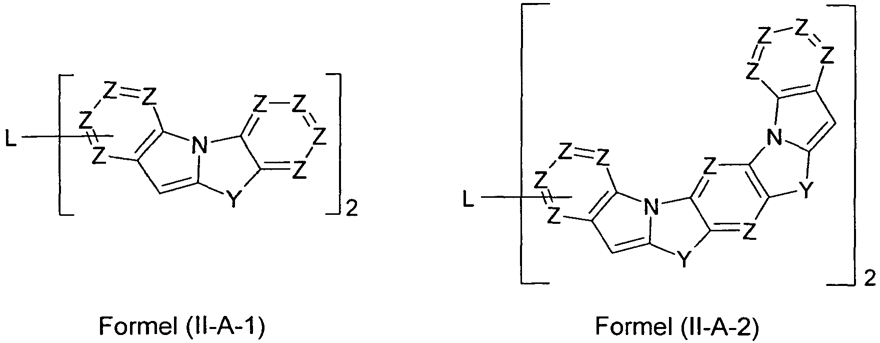 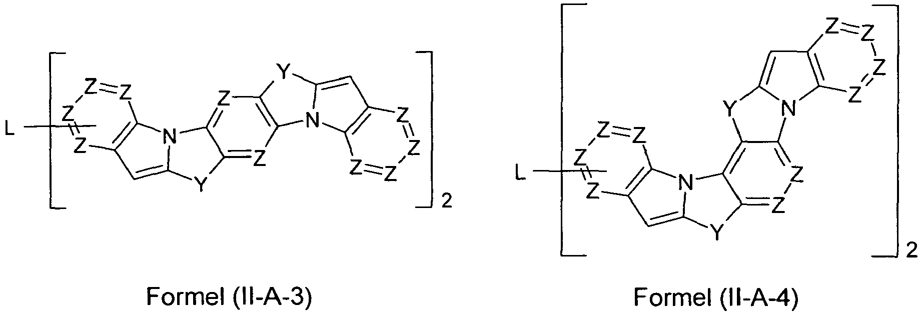 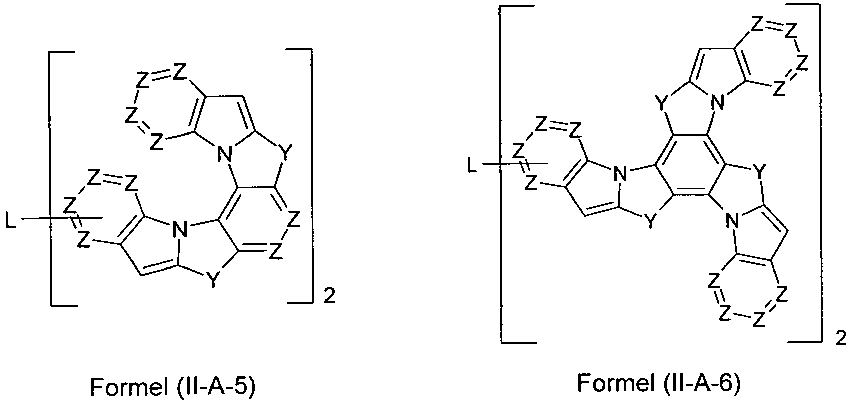 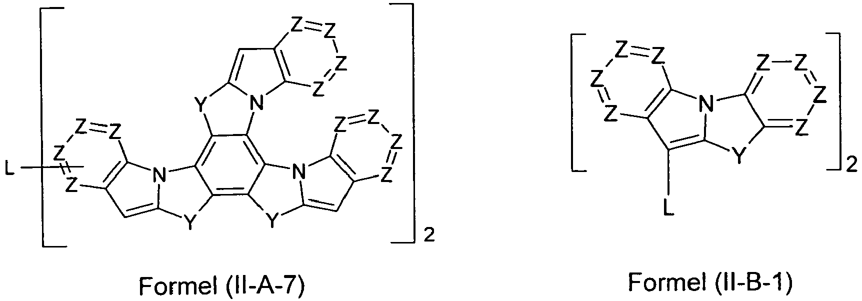 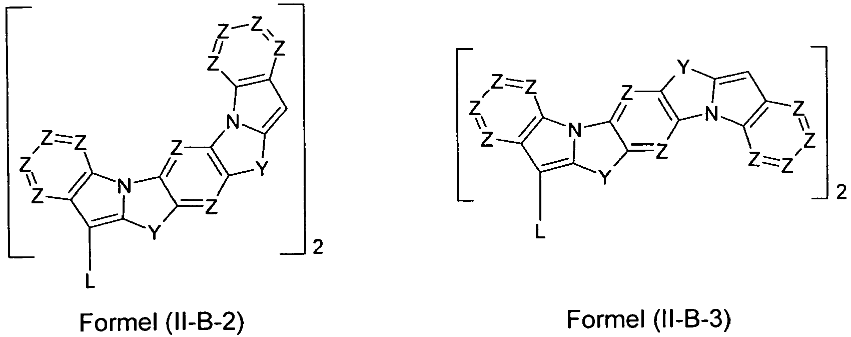 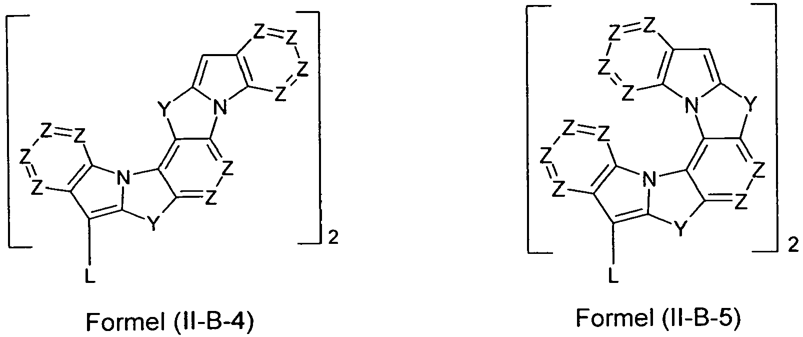 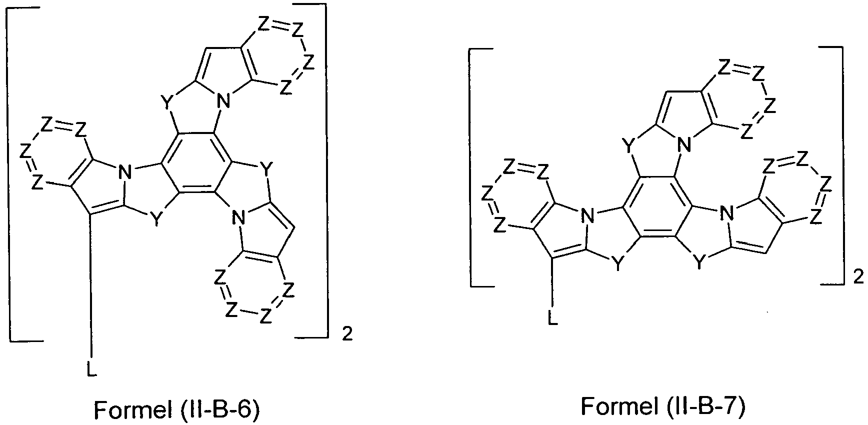 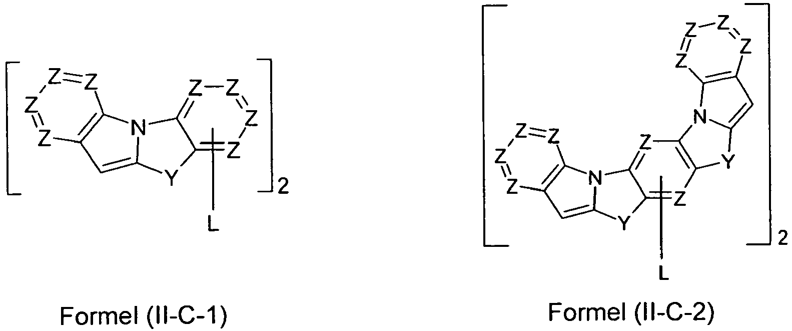 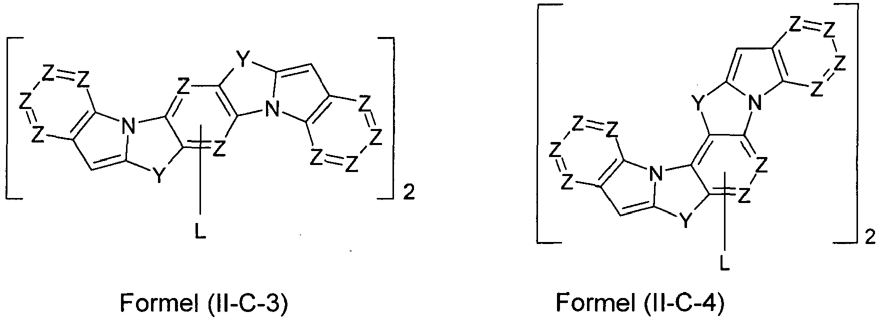 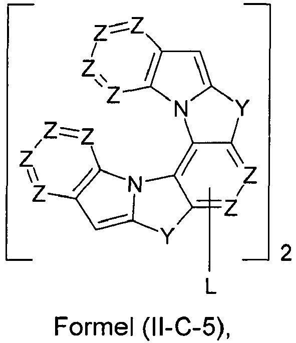 wobei die auftretenden Symbole wie oben definiert sind und diejenige Gruppe Z, an die die Gruppe L bindet, für ein Kohlenstoffatom steht, und wobei weiterhin die Verbindungen an allen unsubstituierten Positionen mit Resten R¹ substituiert sein können.

Für die Verbindungen der Formeln (II-A-1) bis (II-A-7), (II-B-1) bis (II-B-7) und (II-C-1) bis (II-C-5) gelten allgemein die oben angegebenen bevorzugten Ausführungsformen der variablen Gruppen.

Nochmals weiterhin ist es für diese Verbindungen bevorzugt, dass L eine Einfachbindung ist oder ausgewählt ist aus C=O, NR¹, O, S, Alkylengruppen mit 1 bis 10 C-Atomen oder Alkenylengruppen mit 2 bis 10 C-Atomen, wobei bei den genannten Gruppen eine oder mehrere CH₂-Gruppen durch C=O, NR¹, P(=O)(R¹), O oder S ersetzt sein können, oder Arylen- oder Heteroarylengruppen mit 5 bis 20 aromatischen Ringatomen, welche mit einem oder mehreren Resten R¹ substituiert sein können, oder aromatischen oder heteroaromatischen Ringsystemen der Formel (L-1).

Besonders bevorzugt sind Verbindungen der Formeln (II-A-1), (II-A-2), (II-A-3), (II-B-1), (II-B-2) und (II-B-3).

Nochmals stärker bevorzugt sind Verbindungen der folgenden Formeln 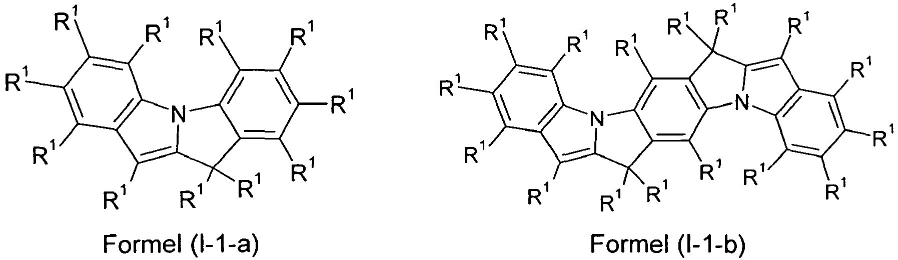 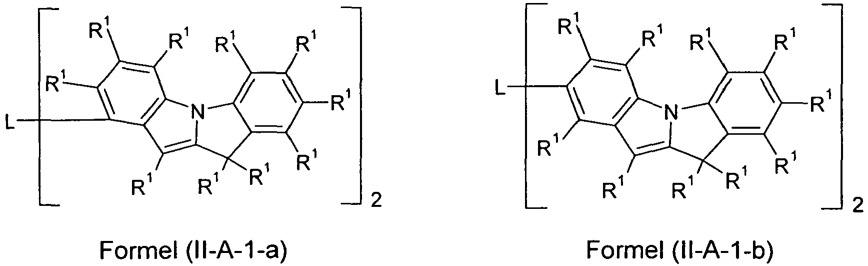 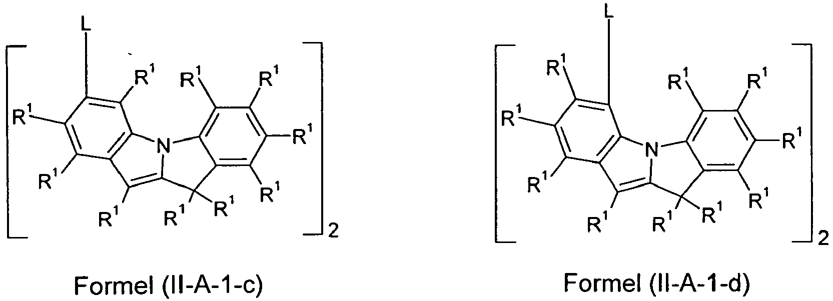 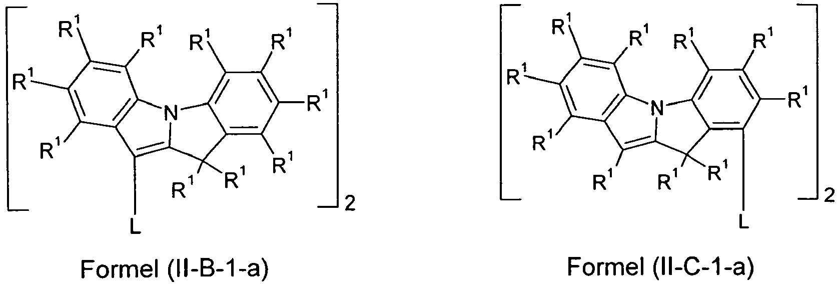 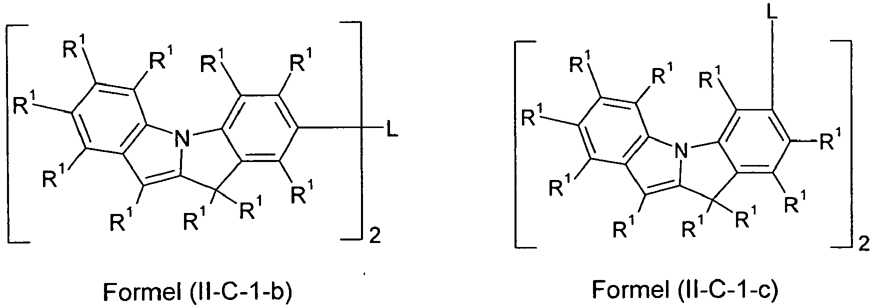 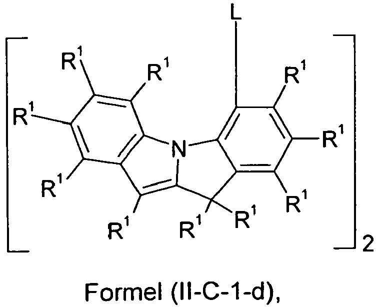 wobei R¹ und L wie oben definiert sind.

Für die Verbindungen der Formeln (I-1-a) bis (I-1-c), (II-A-1-a) bis (II-A-1-d), (II-B-1-a) und (II-C-1-a) bis (II-C-1-d) gelten allgemein die oben angegebenen bevorzugten Ausführungsformen der variablen Gruppen.

Insbesondere ist es für die genannten Verbindungen bevorzugt, dass sie als Substituent R¹ mindestens eine Gruppe tragen, welche ausgewählt ist aus den oben definierten Gruppen R¹-I bis R¹-III.

Nochmals weiterhin ist es für diese Verbindungen bevorzugt, dass L eine Einfachbindung ist oder ausgewählt ist aus C=O, NR¹, O, S, Alkylengruppen mit 1 bis 10 C-Atomen oder Alkenylengruppen mit 2 bis 10 C-Atomen, wobei bei den genannten Gruppen eine oder mehrere CH₂-Gruppen durch C=O, NR¹, P(=O)(R¹), O oder S ersetzt sein können, oder Arylen- oder Heteroarylengruppen mit 5 bis 20 aromatischen Ringatomen, welche mit einem oder mehreren Resten R¹ substituiert sein können, oder aromatischen oder heteroaromatischen Ringsystemen der Formel (L-1).

Besonders bevorzugt sind unter den genannten Verbindungen Verbindungen der Formeln (I-1-a), (I-1-b), (II-A-1-b), (II-B-1-a) und (II-C-1-b).

Beispiele für erfindungsgemäße Verbindungen sind in der folgenden Tabelle aufgeführt:

| | |
|---|---|
| 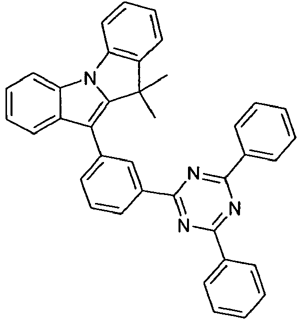 | 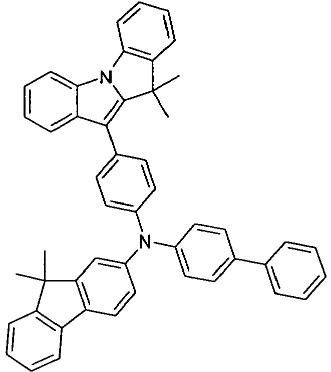 |
| 1 | 2 |
| 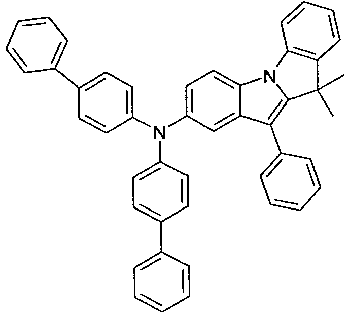 | 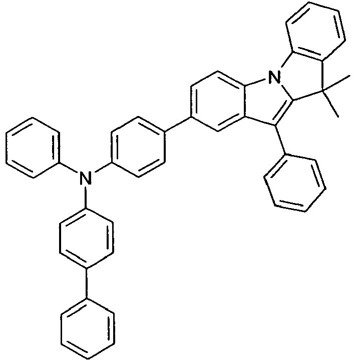 |
| 3 | 4 |
| 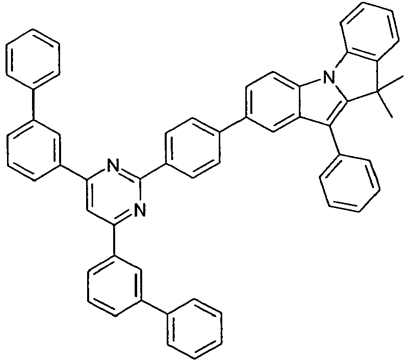 | 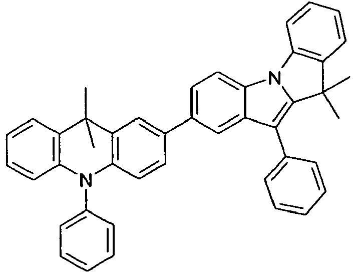 |
| 5 | 6 |
| 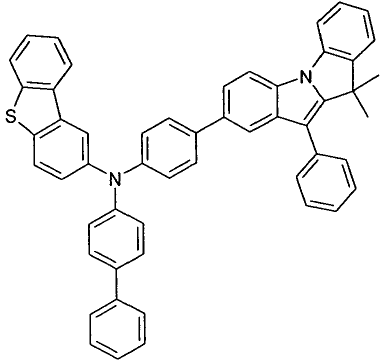 | 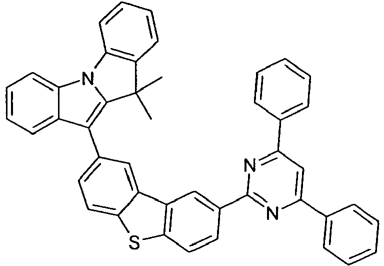 |
| 7 | 8 |
| 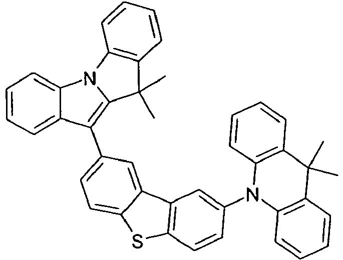 | 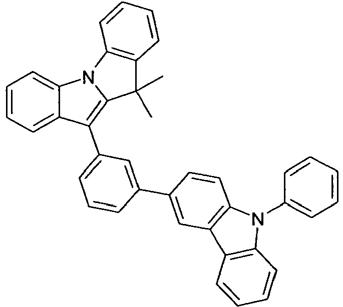 |
| 9 | 10 |
| 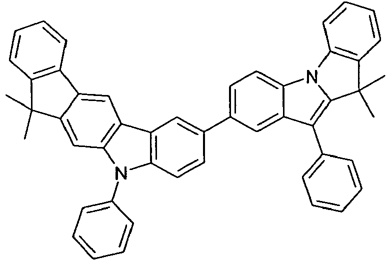 | 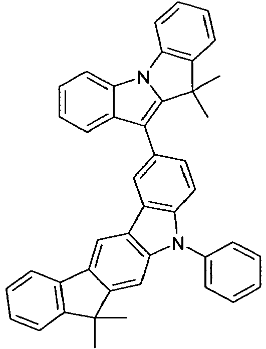 |
| 11 | 12 |
| 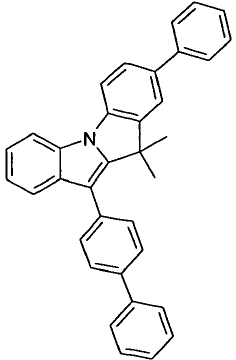 | 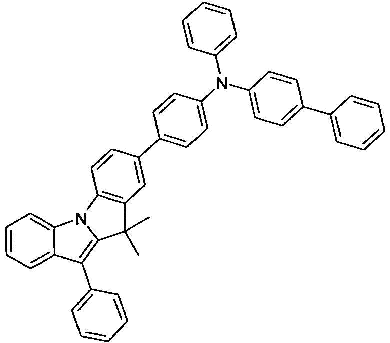 |
| 13 | 14 |
| 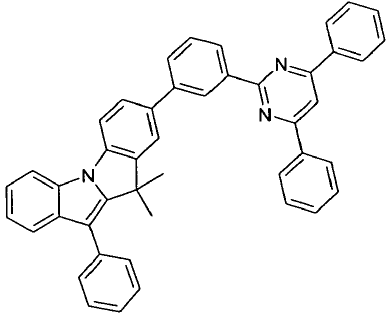 | 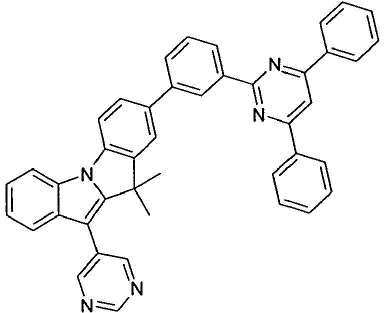 |
| 15 | 16 |
| 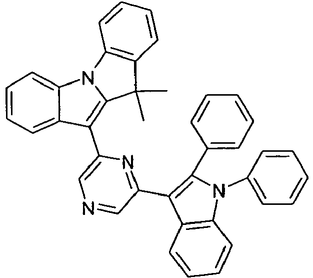 | 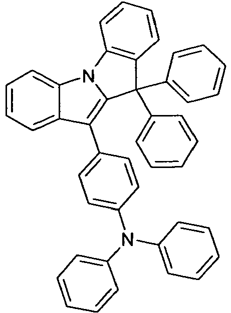 |
| 17 | 18 |
| 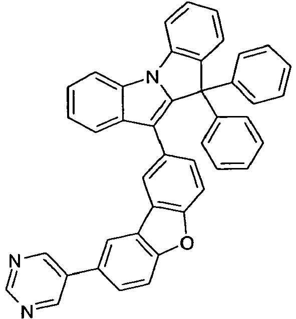 | 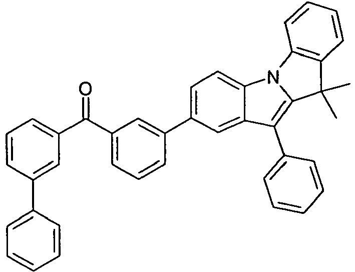 |
| 19 | 20 |
| 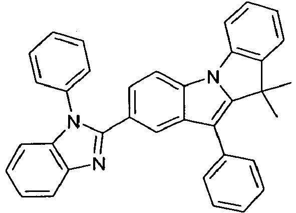 | 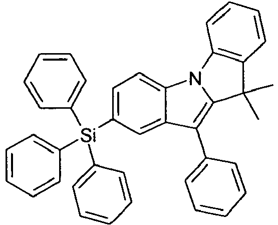 |
| 21 | 22 |
| 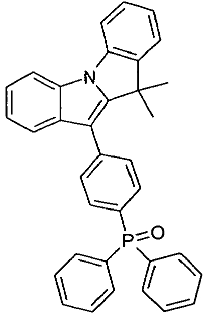 | 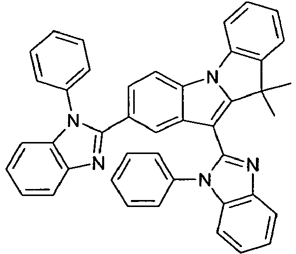 |
| 23 | 24 |
| 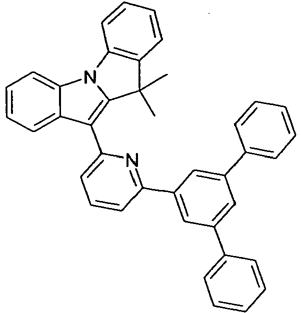 | 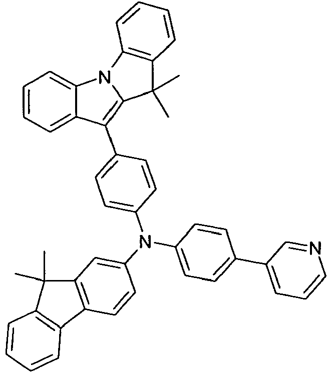 |
| 25 | 26 |
| 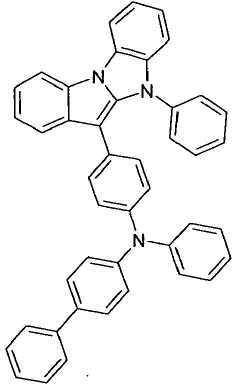 | 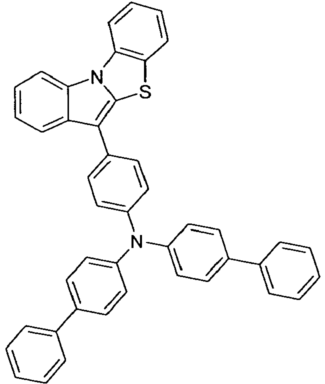 |
| 27 | 28 |
| 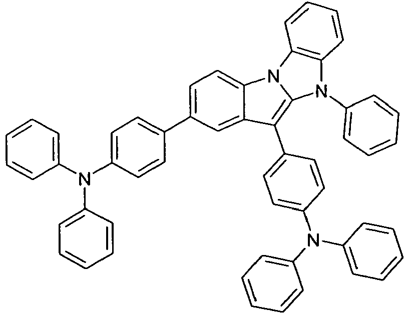 | 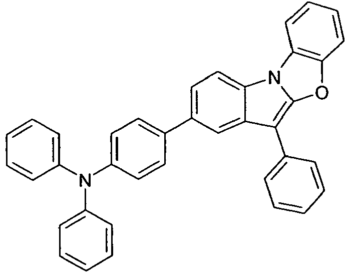 |
| 29 | 30 |
| 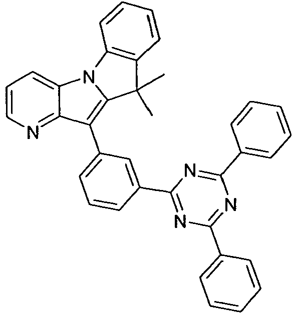 | 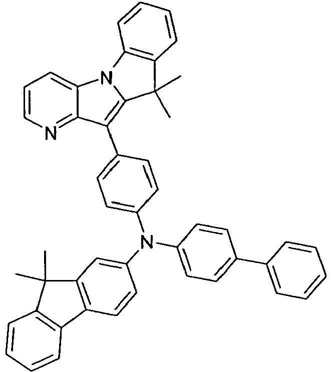 |
| 31 | 32 |
| 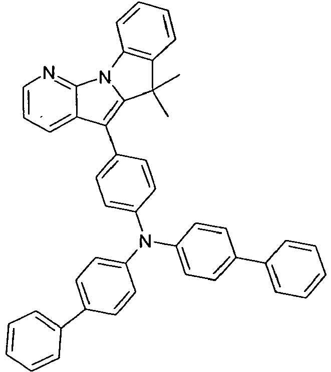 | 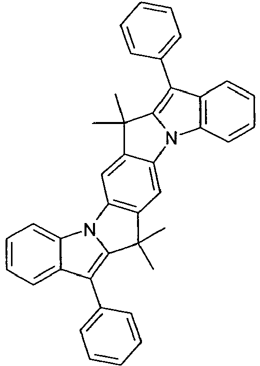 |
| 33 | 34 |
| 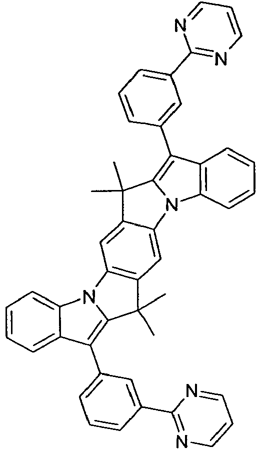 | 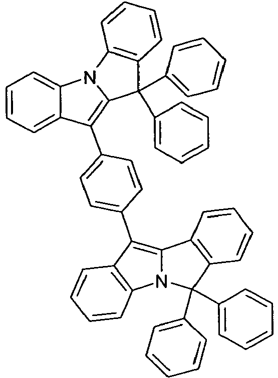 |
| 35 | 36 |
| 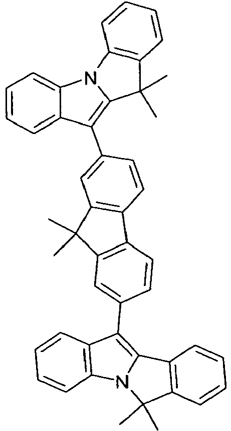 | 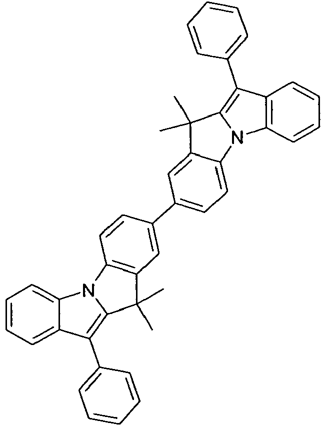 |
| 37 | 38 |
| 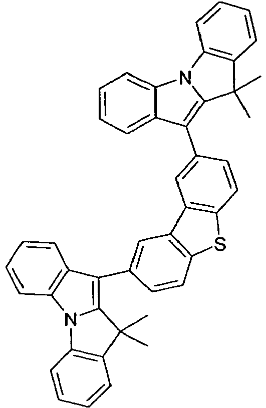 | 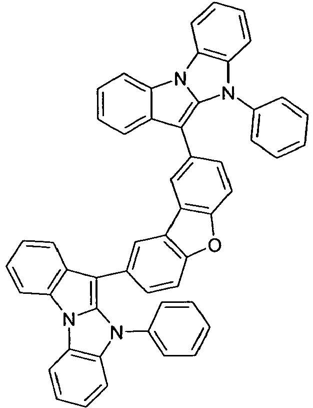 |
| 39 | 40 |
| 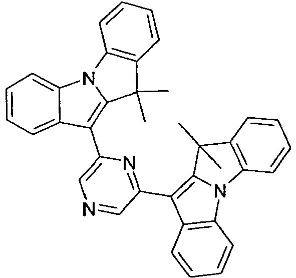 | 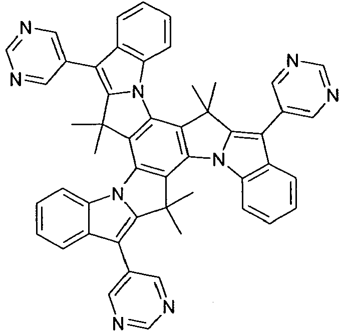 |
| 41 | 42 |
| 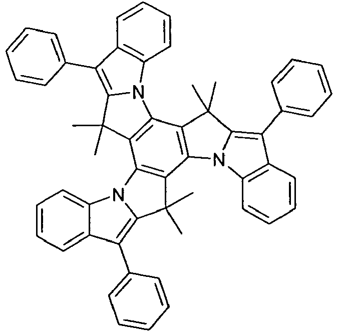 | |
| 43 | |

Gegenstand der Erfindung ist weiterhin eine Verbindung der Formel (I) oder (II), wobei die folgenden Verbindungen ausgenommen sind:

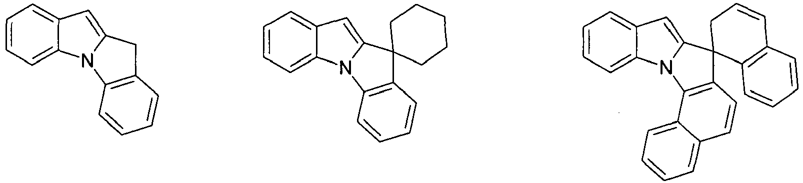

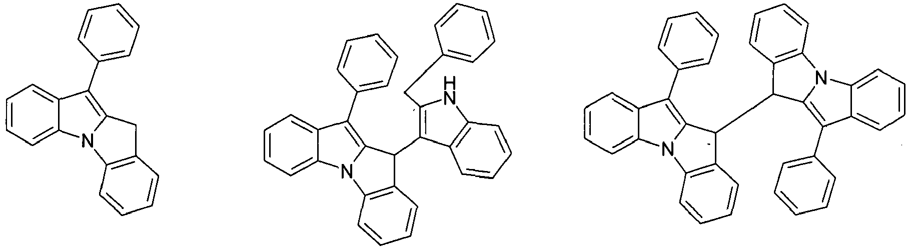

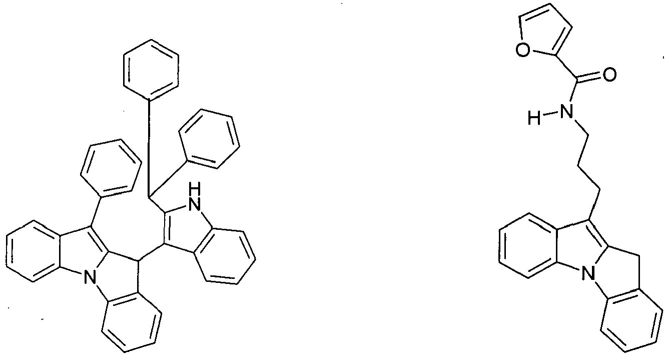

Alle Ausführungsformen von Verbindungen, welche oben im Zusammenhang mit Inhaltsstoffen der erfindungsgemäßen elektronische Vorrichtung als bevorzugt angegeben wurden, gelten auch in Bezug auf den Erfindungsgegenstand der Verbindungen selbst als bevorzugt.

Insbesondere gilt, dass der Index p, der die Anzahl der an L bindenden Molekülteile wiedergibt, bevorzugt gleich 2 oder 3 ist und besonders bevorzugt gleich 2 ist.

Gemäß einer bevorzugten Ausführungsform ist die Summe der Werte für n in Formel (I) gleich 0 oder 1, besonders bevorzugt ist sie gleich 0. Gemäß einer bevorzugten Ausführungsform ist die Summe der Werte für n pro Einheit in eckigen Klammern mit Index p für Formel (II) gleich 0 oder 1, besonders bevorzugt gleich 0.

Die erfindungsgemäßen Verbindungen können durch bekannte organischchemische Syntheseverfahren hergestellt werden. Dazu zählen beispielsweise die Ullmann-Kupplung, die Hartwig-Buchwald-Kupplung, die Suzuki-Kupplung sowie Bromierungen und Zyklisierungen.

Das folgende Schema 1 zeigt die Synthese der Grundkörper **A,** aus denen über Folgereaktionen erfindungsgemäße Verbindungen hergestellt werden können.

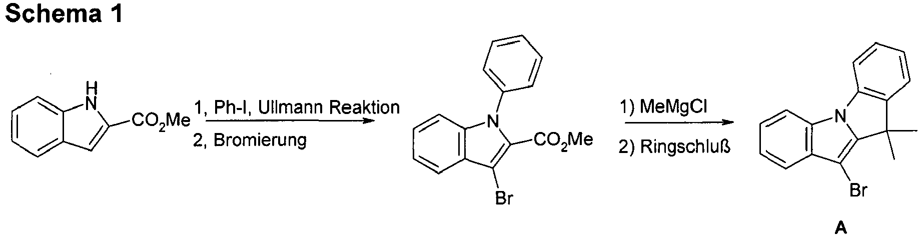

Zur Synthese der Grundkörper **A** wird an 1H-Indolo-2-carbonsäuremethylester zuerst in einer Ullmann-Reaktion lodbenzol gekuppelt. Die resultierende Verbindung wird dann bromiert mittels NBS. Die Umsetzung der resultierenden Verbindung mit Methylmagnesiumchlorid gefolgt von einer Ringschlussreaktion unter wasserentziehenden Bedingungen ergibt das entsprechende verbrückte Indolo-Phenyl-Derivat.

Schema 2 zeigt die Synthese des Grundkörpers **B.** Sie unterscheidet sich von der in Schema 1 gezeigten Synthese lediglich dadurch, dass statt

Iodbenzol 1,4-Diiodbenzol in der Ullmann-Kupplung mit zwei Äquivalenten 1H-Indolo-2-carbonsäure-methylester eingesetzt wird.

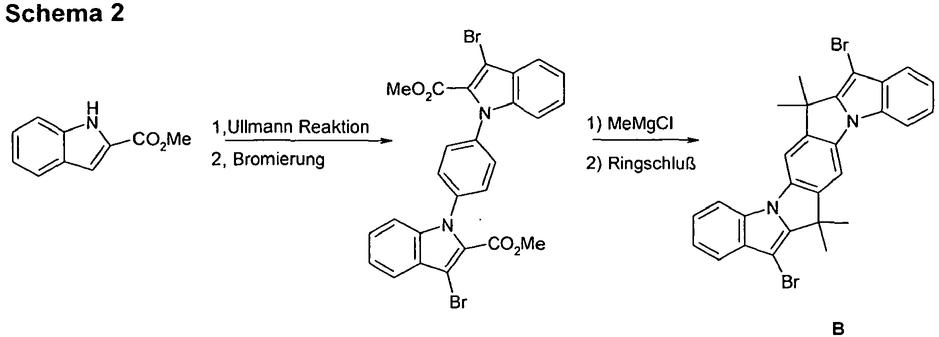

In Schema 3a werden exemplarisch für den Grundkörper **A** verschiedene Möglichkeiten gezeigt, durch Derivatisierungsreaktionen erfindungsgemäße Vebindungen der Formel (I) zu erhalten. Für die Grundkörper **B** kann analog verfahren werden.

In der ersten gezeigten Reaktion wird eine Suzuki-Reaktion mit einem mono-Boronsäure-funktionalisierten Arylderivat durchgeführt. In der zweiten gezeigten Reaktion wird durch eine Buchwald-Reaktion mit einem Diarylamin ein entsprechendes Diarylamin als Substituent am Indol eingeführt.

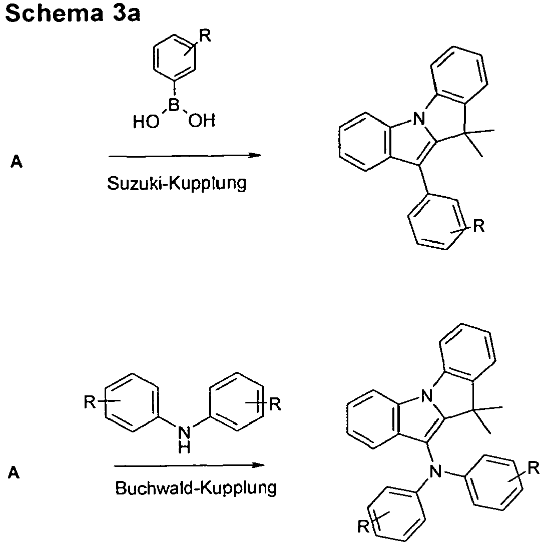

In Schema 3b wird exemplarisch gezeigt, wie aus Intermediaten des Grundkörpers **A** erfindungsgemäße Verbindungen der Formel (II) erhalten werden können. Hierzu wird ein Bis-Diboronsäure-funktionalisiertes Arylderivat, gezeigt am Beispiel von 4,4'-Diboronsäurebiphenyl, eingesetzt, so dass an jede der beiden Boronsäure-substituierten Positionen des Arylderivats jeweils ein Grundkörper **A** gekuppelt wird.

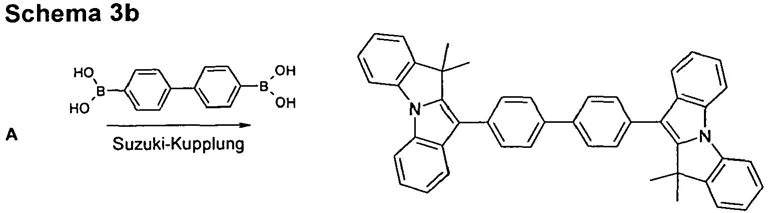

Weiterhin können in solchen Reaktionen auch andere divalente Arylgruppen in der Suzuki-Kupplung eingesetzt werden, beispielsweise Heteroarylgruppen wie Dibenzothiophen oder andere Arylgruppen wie Benzol, Fluoren oder Terphenyl.

In Schema 4a werden exemplarisch für den Grundkörper A weitere Möglichkeiten gezeigt, durch Derivatisierungsreaktionen erfindungsgemäße Verbindungen nach Formel (I) zu erhalten. Für die Grundkörper **B** kann analog verfahren werden. Als Intermediat wird ein Grundkörper **C** erhalten.

Zuerst wird dazu eine Monobromierung durchgeführt, um das entsprechende 5-Bromindol zu erhalten. Mittels Suzuki-Reaktion oder Buchwald-Reaktion wird anschließend eine Arylgruppe bzw. ein Diarylamin als Substituent an der Position 5 des Indolrings eingeführt.

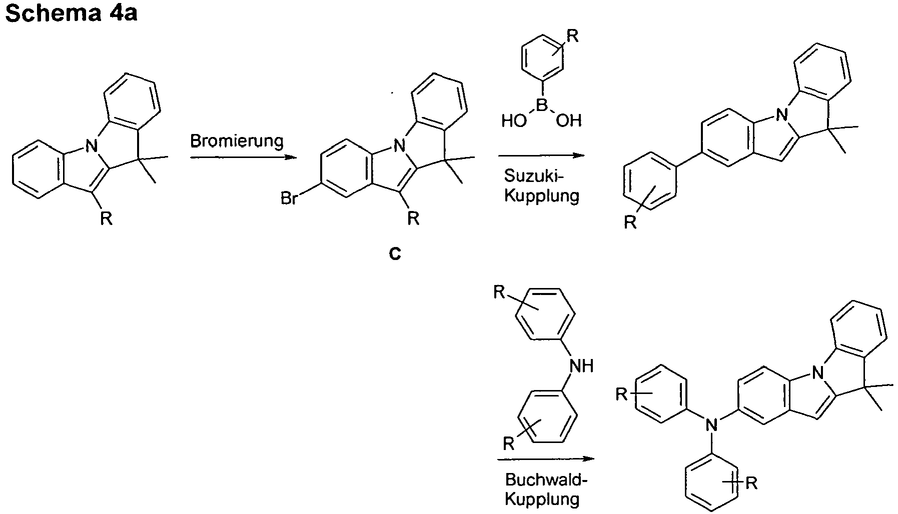

In Schema 4b wird exemplarisch gezeigt, wie auf analogem Weg zu Schema 4a durch Verwendung von bifunktionellen Arylderivaten in der Buchwald-Kupplung erfindungsgemäße Verbindungen nach Formel (II) erhalten werden können. Hierzu wird ein Bis-Diboronsäure-funktionalisiertes Arylderivat, gezeigt am Beispiel von 1,3-Diboronsäurebenzol, eingesetzt, an das zwei Gruppen des Grundkörpers C gekuppelt werden.

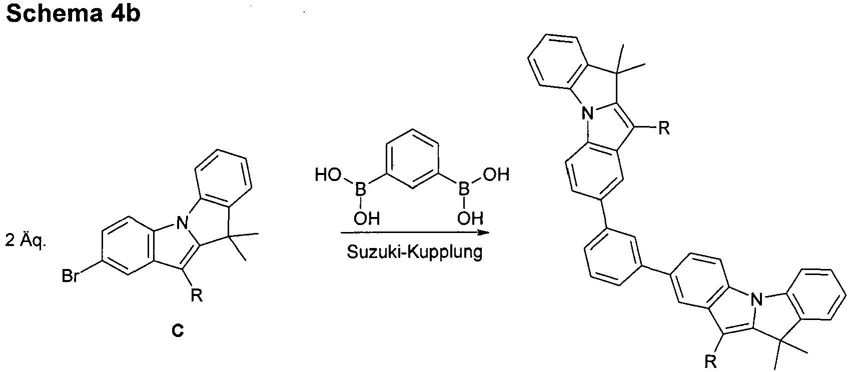

In Reaktionen gemäß Schema 4b können anstelle von Verbindungen des Grundkörpers **C** auch Verbindungen des Grundkörper **A** eingesetzt werden, um alternative Verbindungen der Formel (II) zu erhalten. Weiterhin können in solchen Reaktionen auch andere divalente Arylgruppen in der Buchwald-Kupplung eingesetzt werden, beispielsweise Heteroarylgruppen wie Dibenzothiophen oder andere Arylgruppen wie Benzol, Fluoren oder Terphenyl.

Die oben beschriebenen Synthesewege sollen lediglich als Beispiele dienen. Der Fachmann kann zur Synthese der erfindungsgemäßen Verbindungen auf alternative Syntheseverfahren zurückgreifen, wenn ihm dies unter den gegebenen Umständen vorteilhaft erscheint. Weiterhin kann er unter Heranziehung seines allgemeinen Fachwissens auf dem Gebiet der organischen Synthesechemie die gezeigten Synthesen erweitern und/oder modifizieren, um erfindungsgemäße Verbindungen herzustellen.

Gegenstand der Erfindung ist somit weiterhin ein Verfahren zur Herstellung einer Verbindung gemäß Formel (I) oder (II), dadurch gekennzeichnet, dass mindestens eine metallorganische Kupplungsreaktion zwischen einem Indolderivat und einer halogensubstituierten aromatischen oder heteroaromatischen Verbindung und mindestens eine Ringschlussreaktion zwischen dem Indolderivat und der gekuppelten aromatischen oder heteroaromatischen Verbindung umfasst sind.

Die oben beschriebenen Verbindungen gemäß Formel (I) oder (II), insbesondere Verbindungen, welche mit reaktiven Abgangsgruppen, wie Brom, Iod, Chlor, Boronsäure oder Boronsäureester, substituiert sind, können als Monomere zur Erzeugung von Oligomeren, Dendrimeren oder Polymeren Verwendung finden. Die Oligomerisation bzw. Polymerisation erfolgt dabei bevorzugt über die Halogenfunktionalität bzw. die Boronsäurefunktionalität.

Weiterer Gegenstand der Erfindung sind daher Oligomere, Polymere oder Dendrimere enthaltend eine oder mehrere Verbindungen gemäß Formel (I) oder (II), wobei die Bindung(en) zum Polymer, Oligomer oder Dendrimer an beliebigen, in Formel (I) oder (II) mit R¹ substituierten Positionen lokalisiert sein können. Je nach Verknüpfung der Verbindung gemäß Formel (I) oder (II) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein. In den linear verknüpften Strukturen können die Einheiten gemäß Formel (I) oder (II) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) oder (II) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein. Für die Wiederholeinheiten gemäß Formel (I) oder (II) in Oligomeren, Dendrimeren und Polymeren gelten dieselben Bevorzugungen wie oben für Verbindungen gemäß Formel (I) oder (II) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) oder (II) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 02/067343 A1 und WO 2005/026144 A1.

Für die Verarbeitung der Verbindungen gemäß Formel (I) oder (II) aus flüssiger Phase, beispielsweise durch Spin-Coating oder durch Druckverfahren, sind Formulierungen der Verbindungen erforderlich. Diese Formulierungen können beispielsweise Lösungen, Dispersionen oder Miniemulsionen sein.

Gegenstand der Erfindung ist daher weiterhin eine Formulierung, insbesondere eine Lösung, Dispersion oder Miniemulsion, enthaltend mindestens eine Verbindung gemäß Formel (I) oder (II) oder mindestens ein Polymer, Oligomer oder Dendrimer enthaltend mindestens eine Einheit gemäß Formel (I) oder (II) sowie mindestens ein Lösungsmittel, bevorzugt ein organisches Lösungsmittel. Wie solche Lösungen hergestellt werden können, ist dem Fachmann bekannt und beispielsweise in den Anmeldungen WO 2002/072714 und WO 2003/019694 und der darin zitierten Literatur beschrieben. Bevorzugt ist eine Formulierung enthaltend mindestens eine Verbindung gemäß Formel (I) oder (II), sowie mindestens ein Lösungsmittel.

Die Verbindungen gemäß Formel (I) oder (II) eignen sich für den Einsatz in elektronischen Vorrichtungen, insbesondere in organischen Elektrolumineszenzvorrichtungen (OLEDs). Abhängig von der Substitution werden die Verbindungen bevorzugt in bestimmten Funktionen und/oder Schichten eingesetzt.

Beispielsweise sind Verbindungen gemäß Formel (I) oder (II), welche als Substituent R¹ mindestens eine Gruppe R¹-I tragen, wie oben definiert, besonders zur Verwendung als Matrixmaterial für phosphoreszierende Dotanden, als Elektronentransportmaterial oder als Lochblockiermaterial geeignet. Bevorzugt umfasst dabei die Gruppe R¹-I mindestens eine elektronenarme Gruppe wie Sechsring-Heteroarylgruppen mit einem oder mehreren Stickstoffatomen oder Fünfring-Heteroarylgruppen mit zwei oder mehr Stickstoffatomen.

Weiterhin sind Verbindungen gemäß Formel (I) oder (II), welche als Substituent R¹ mindestens eine Gruppe R¹-II und/oder R¹-III tragen, wie oben definiert, besonders zur Verwendung als Lochtransportmaterialien oder zur Verwendung als fluoreszierende Dotanden geeignet. Bevorzugt umfasst die Gruppe R¹-II dabei ein aromatisches Ringsystem mit 12 bis 20 aromatischen Ringatomen.

Bevorzugt werden die Verbindungen gemäß Formel (I) oder (II) als Elektronentransportmaterial in einer Elektronentransportschicht, als Matrixmaterial in einer emittierenden Schicht oder als Lochtransportmaterial in einer Lochtransportschicht eingesetzt. Wenn die Verbindungen als Matrixmaterialien in einer emittierenden Schicht eingesetzt werden, umfasst die emittierende Schicht bevorzugt mindestens eine phosphoreszierende Emitterverbindung. Die Verbindungen können aber auch in anderen Schichten und/oder Funktionen eingesetzt werden, beispielsweise als fluoreszierende Dotanden in einer emittierenden Schicht oder als Loch- oder Elektronenblockiermaterialien.

Ein weiterer Gegenstand der Erfindung ist daher die Verwendung der Verbindungen gemäß Formel (I) oder (II) in elektronischen Vorrichtungen. Dabei sind die elektronischen Vorrichtungen bevorzugt ausgewählt aus der Gruppe bestehend aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und besonders bevorzugt ausgewählt aus organischen Elektrolumineszenzvorrichtungen (OLEDs).

Gegenstand der Erfindung ist, wie bereits oben erwähnt, eine elektronische Vorrichtung, enthaltend Anode, Kathode sowie mindestens eine organische Schicht, wobei die organische Schicht mindestens eine Verbindung der Formel (I) oder (II) enthält. Dabei ist die elektronische Vorrichtung bevorzugt ausgewählt aus den oben genannten Vorrichtungen und besonders bevorzugt eine organische Elektrolumineszenzvorrichtung.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer), Auskopplungsschichten und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt Die in den jeweiligen Schichten und Funktionen bevorzugt eingesetzten Verbindungen werden in späteren Abschnitten explizit offenbart.

Es ist erfindungsgemäß bevorzugt, wenn die Verbindung gemäß Formel (I) oder (II) in einer elektronischen Vorrichtung enthaltend einen oder mehrere phosphoreszierende Dotanden eingesetzt wird. Dabei kann die Verbindung in unterschiedlichen Schichten, bevorzugt in einer Elektronentransportschicht, einer Lochtransportschicht, einer Lochinjektionsschicht oder in der emittierenden Schicht verwendet werden. Die Verbindung gemäß Formel (I) oder (II) kann aber auch erfindungsgemäß in einer elektronischen Vorrichtung, enthaltend einen oder mehrere fluoreszierende Dotanden und keine phosphoreszierenden Dotanden, eingesetzt werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen phosphoreszierenden Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den erfindungsgemäßen Verbindungen in organischen Elektrolumineszenzvorrichtungen einsetzen. Weitere Beispiele für geeignete phosphoreszierende Dotanden können der in einem späteren Abschnitt folgenden Tabelle entnommen werden.

In einer bevorzugten Ausführungsform der vorliegenden Erfindung werden die Verbindungen der Formel (I) oder (II) als Matrixmaterial in Kombination mit einem oder mehreren Dotanden, vorzugsweise phosphoreszierenden Dotanden, eingesetzt. Die Verbindungen sind insbesondere dann zur Verwendung als Matrixmaterial geeignet, wenn sie eine oder mehrere Gruppen der Formel R¹-I enthalten, wie zum Beispiel Sechsring-Heteroarylgruppen mit einem oder mehreren Stickstoffatomen oder Fünfring-Heteroarylgruppen mit zwei oder mehr Stickstoffatomen.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%. Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß Formel (I) oder (II) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt.

Die Mixed-Matrix-Systeme können einen oder mehrere Dotanden umfassen. Die Dotandverbindung bzw. die Dotandverbindungen zusammen haben erfindungsgemäß einen Anteil von 0.1 bis 50.0 Vol.-% an der Gesamtmischung und bevorzugt einen Anteil von 0.5 bis 20.0 Vol.-% an der Gesamtmischung. Entsprechend haben die Matrixkomponenten zusammen einen Anteil von 50.0 bis 99.9 Vol-% an der Gesamtmischung und bevorzugt einen Anteil von 80.0 bis 99.5 Vol.-% an der Gesamtmischung.

Besonders geeignete Matrixmaterialien, welche in Kombination mit den erfindungsgemäßen Verbindungen als Matrixkomponenten eines Mixed-Matrix-Systems eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, Indenocarbazolderivate, z. B. gemäß WO 2010/136109 und WO 2011/000455, oder verbrückte Carbazole, z. B gemäß WO 2011/088877 und WO 2011/128017.

Bevorzugte phosphoreszierende Dotanden zur Verwendung in Mixed-Matrix-Systemen enthaltend die erfindungsgemäßen Verbindungen sind die in einer folgenden Tabelle aufgeführten phosphoreszierenden Dotanden.

In einer weiteren bevorzugten Ausführungsform der Erfindung werden die Verbindungen gemäß Formel (I) oder (II) als Lochtransportmaterial eingesetzt. Die Verbindungen werden dann bevorzugt in einer Lochtransportschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Die Verbindungen sind insbesondere dann zur Verwendung als Lochtransportmaterial geeignet, wenn sie mit einem oder mehreren aromatischen Ringsystemen mit 12 bis 20 aromatischen Ringatomen und/oder mit einer oder mehreren Arylaminogruppen substituiert sind.

Wird die Verbindung gemäß Formel (I) oder (II) als Lochtransportmaterial in einer Lochtransportschicht eingesetzt, so kann die Verbindung als Reinmaterial, d.h. in einem Anteil von 100 % in der Lochtransportschicht eingesetzt werden, oder sie kann in Kombination mit weiteren Verbindungen in der Lochtransportschicht eingesetzt werden.

In einer weiteren Ausführungsform der Erfindung werden die die Verbindungen gemäß Formel (I) oder (II) als fluoreszierende Dotanden in einer emittierenden Schicht eingesetzt. Die Verbindungen sind insbesondere dann zur Verwendung als fluoreszierende Dotanden geeignet, wenn sie mit einem oder mehreren aromatischen Systemen, bevorzugt aromatischen Systemen enthaltend 12 bis 20 aromatische Ringatome, substituiert sind. Die erfindungsgemäßen Verbindungen werden bevorzugt als grüne oder blaue Emitter verwendet.

Der Anteil der Verbindung gemäß Formel (I) oder (II) als Dotand in der Mischung der emittierenden Schicht beträgt in diesem Fall zwischen 0.1 und 50.0 Vol.-%, bevorzugt zwischen 0.5 und 20.0 Vol.-%, besonders bevorzugt zwischen 0.5 und 8.0 Vol.-%. Entsprechend beträgt der Anteil des Matrixmaterials zwischen 50.0 und 99.9 Vol.-%, bevorzugt zwischen 80.0 und 99.5 Vol.-%, besonders bevorzugt zwischen 92.0 und 99.5 Vol.-%.

Bevorzugte Matrixmaterialien zur Verwendung in Kombination mit den erfindungsgemäßen Verbindungen als fluoreszierende Dotanden sind in einem der folgenden Abschnitte aufgeführt. Sie entsprechen den als bevorzugt aufgeführten Matrixmaterialien für fluoreszierende Dotanden.

In einer weiteren Ausführungsform der Erfindung werden die Verbindungen als Elektronentransportmaterialien in einer Elektronentransportschicht einer organischen Elektrolumineszenzvorrichtung eingesetzt. Die Verbindungen sind insbesondere dann zur Verwendung als Elektronentransportmaterial geeignet, wenn sie eine oder mehrere Gruppen der Formel R¹-I enthalten, wie zum Beispiel Sechsring-Heteroarylgruppen mit einem oder mehreren Stickstoffatomen oder Fünfring-Heteroarylgruppen mit zwei oder mehr Stickstoffatomen.

Die organische Elektrolumineszenzvorrichtung kann auch mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren, wobei die verschiedenen Farben in dieser Ausführungsform der Erfindung zusammen weißes Licht ergeben. Insbesondere bevorzugt sind Dreischichtsysteme, also Systeme mit drei emittierenden Schichten, wobei eine oder mehrere dieser Schichten eine Verbindung gemäß Formel (I) oder (II) enthalten kann und wobei die drei Schichten blaue, grüne und orangefarbene oder rote Emission zeigen (für den prinzipiellen Aufbau siehe z. B. WO 2005/011013). Ebenso eignen sich in solchen Systemen für weiße Emission Emitter, welche breitbandige Emissionsbanden aufweisen und dadurch weiße Emission zeigen. Alternativ und/oder zusätzlich können die erfindungsgemäßen Verbindungen in solchen Systemen auch in einer Lochtransportschicht oder Elektronentransportschicht oder in einer anderen Schicht vorhanden sein.

Im Folgenden werden die in den elektronischen Vorrichtungen, enthaltend eine oder mehrere Verbindungen gemäß Formel (I) oder (II), bevorzugt eingesetzten weiteren Funktionsmaterialien aufgeführt.

Die in der folgenden Tabelle aufgeführten Verbindungen stellen besonders geeignete phosphoreszierende Dotanden dar.

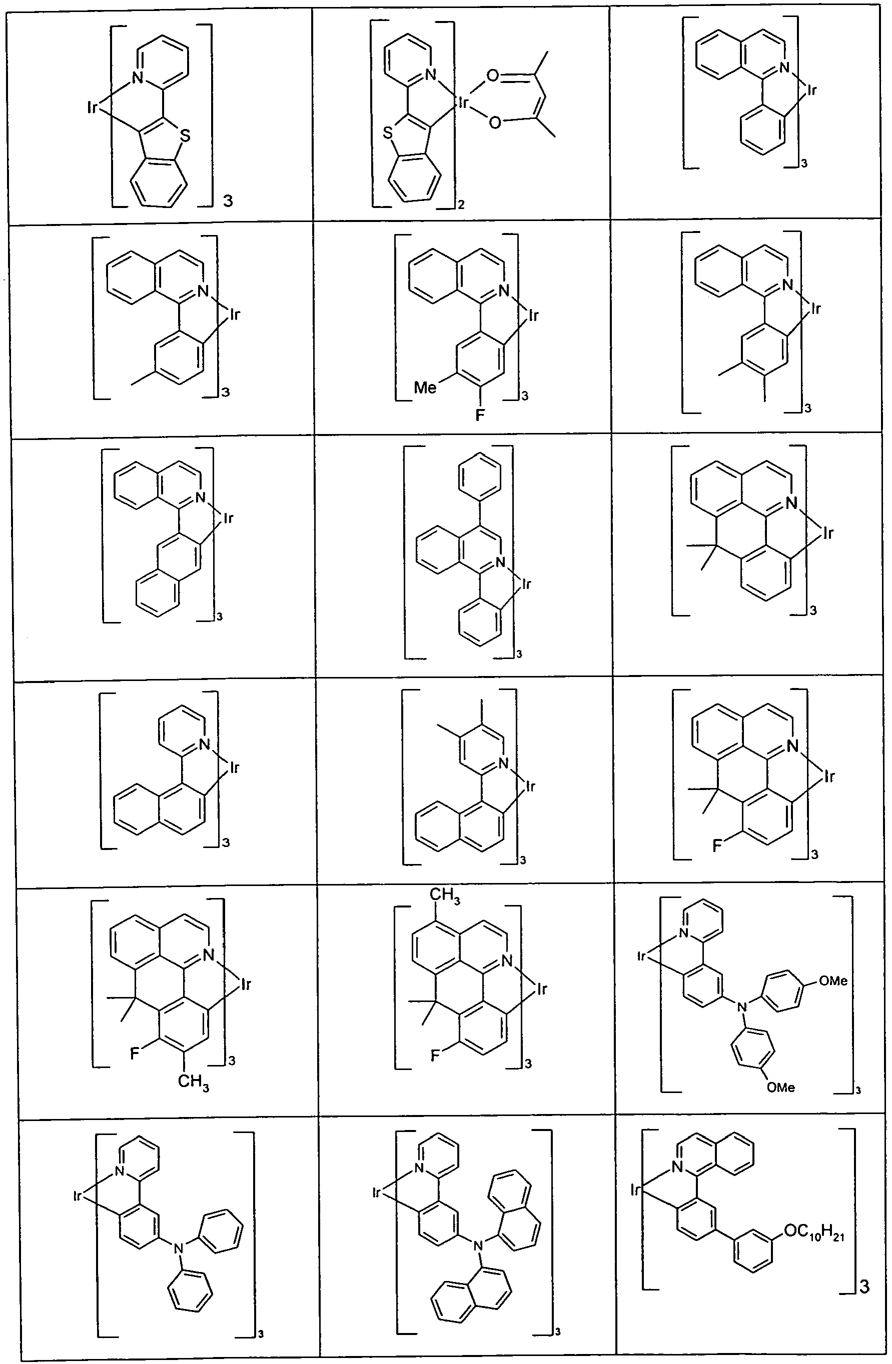

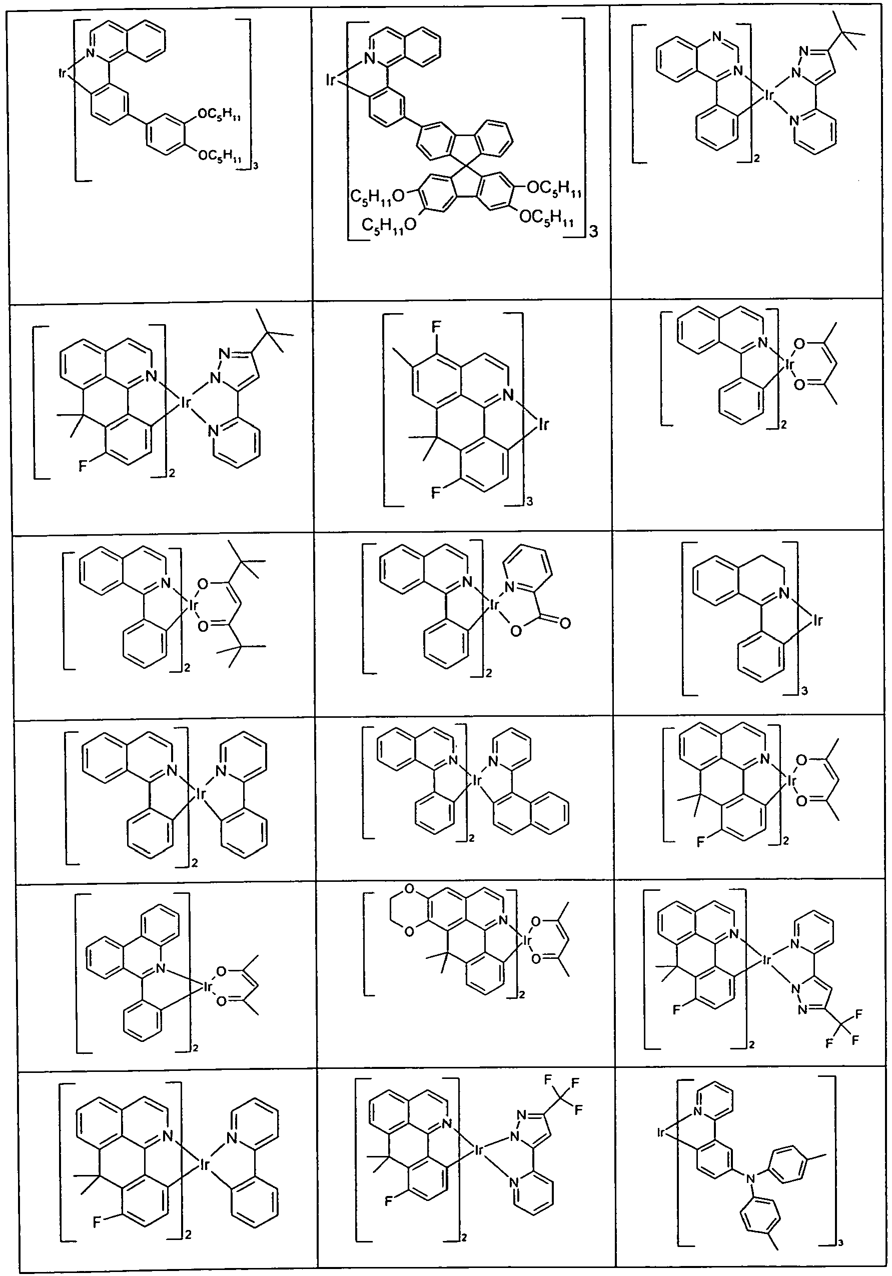

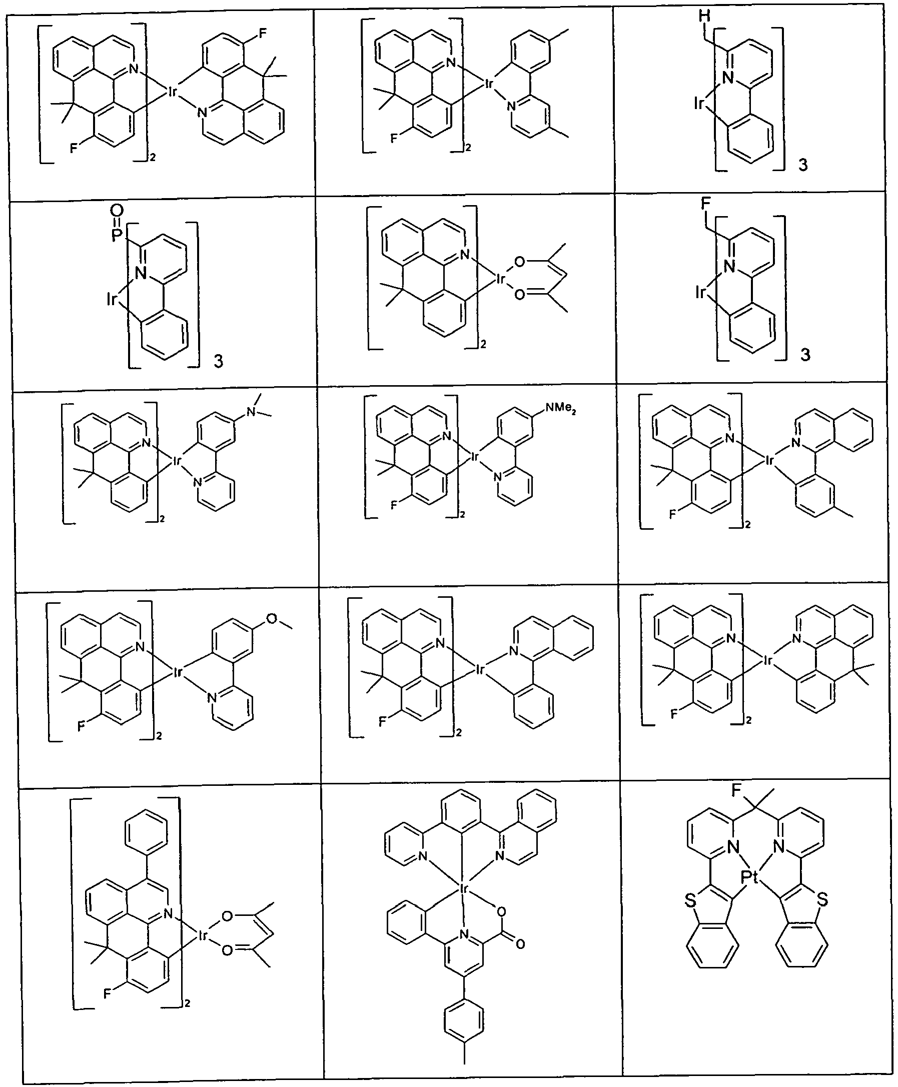

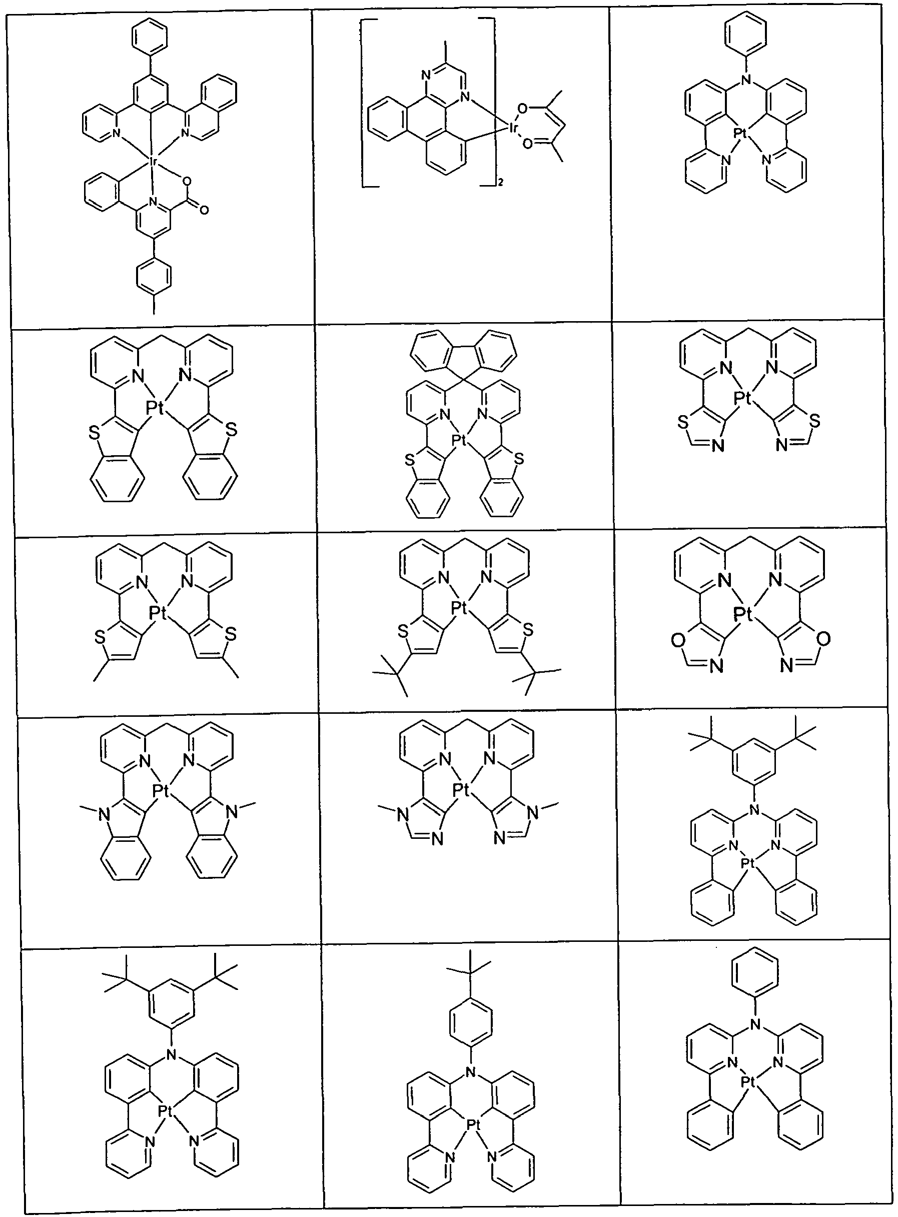

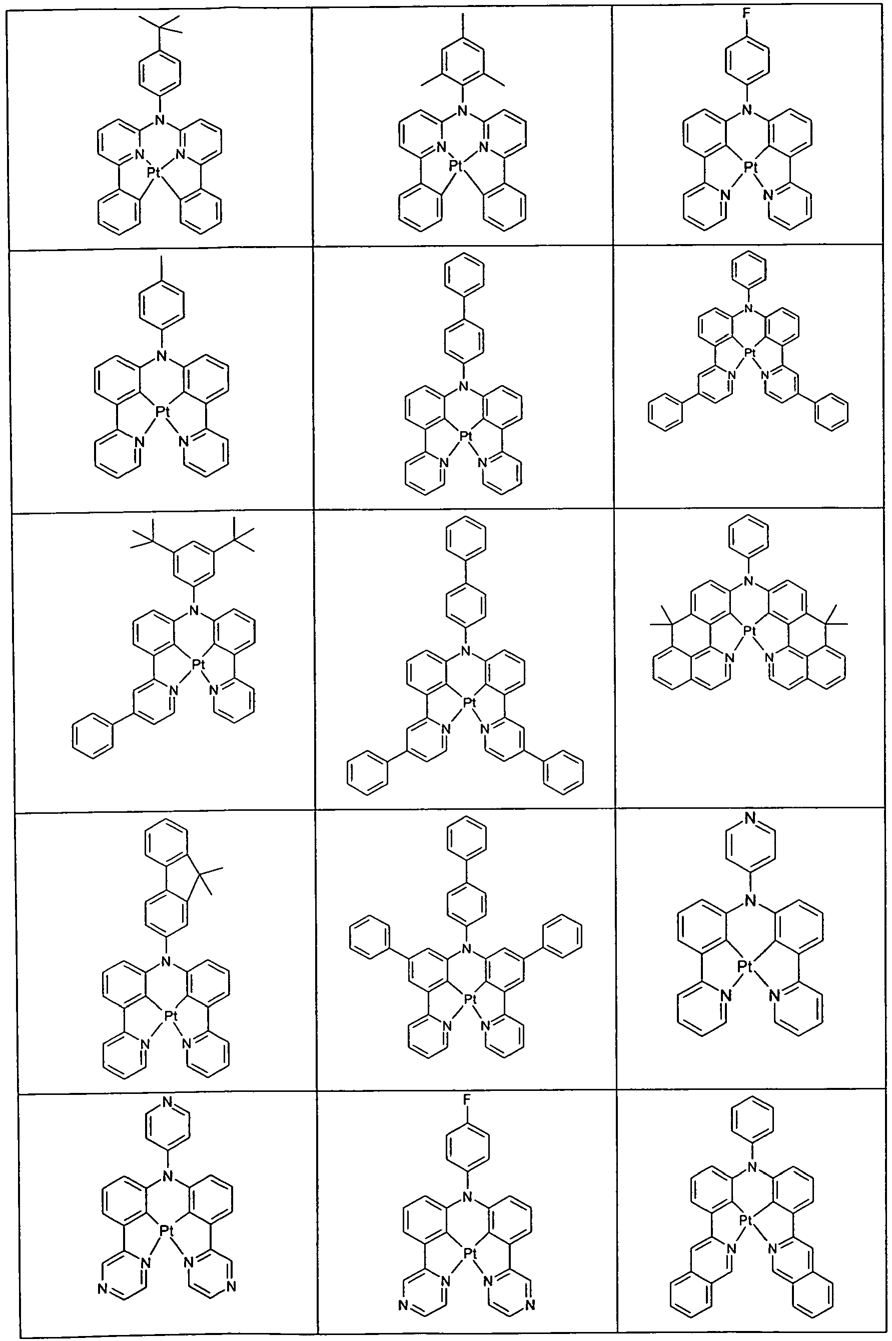

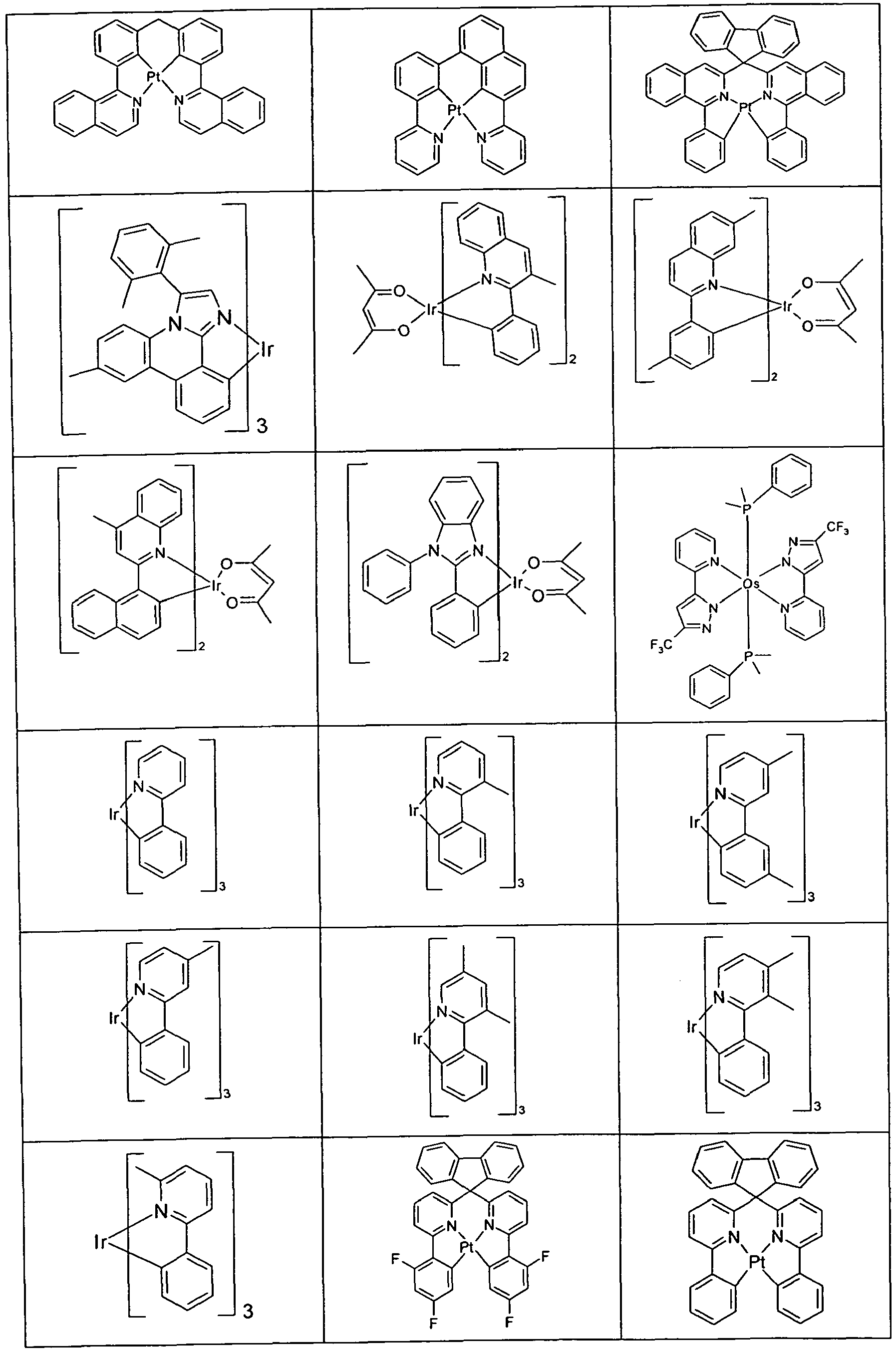

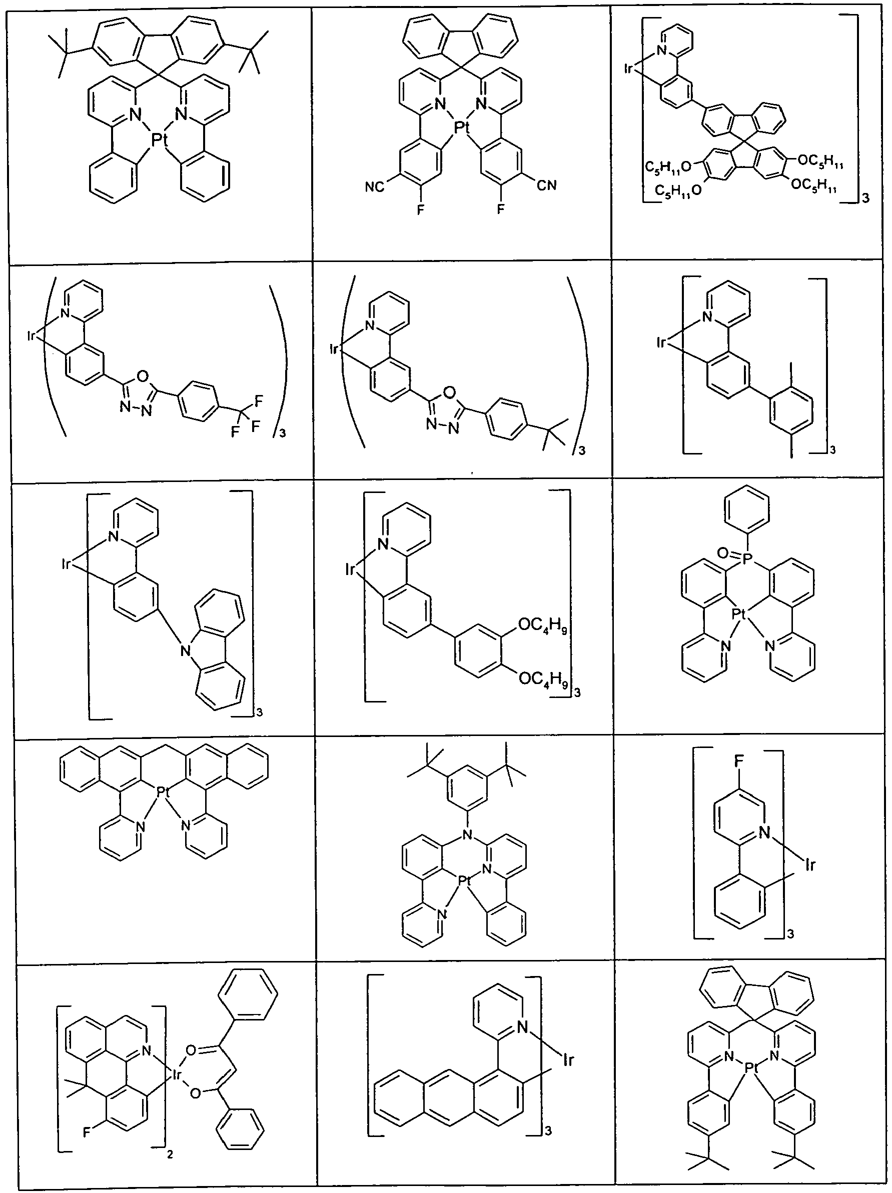

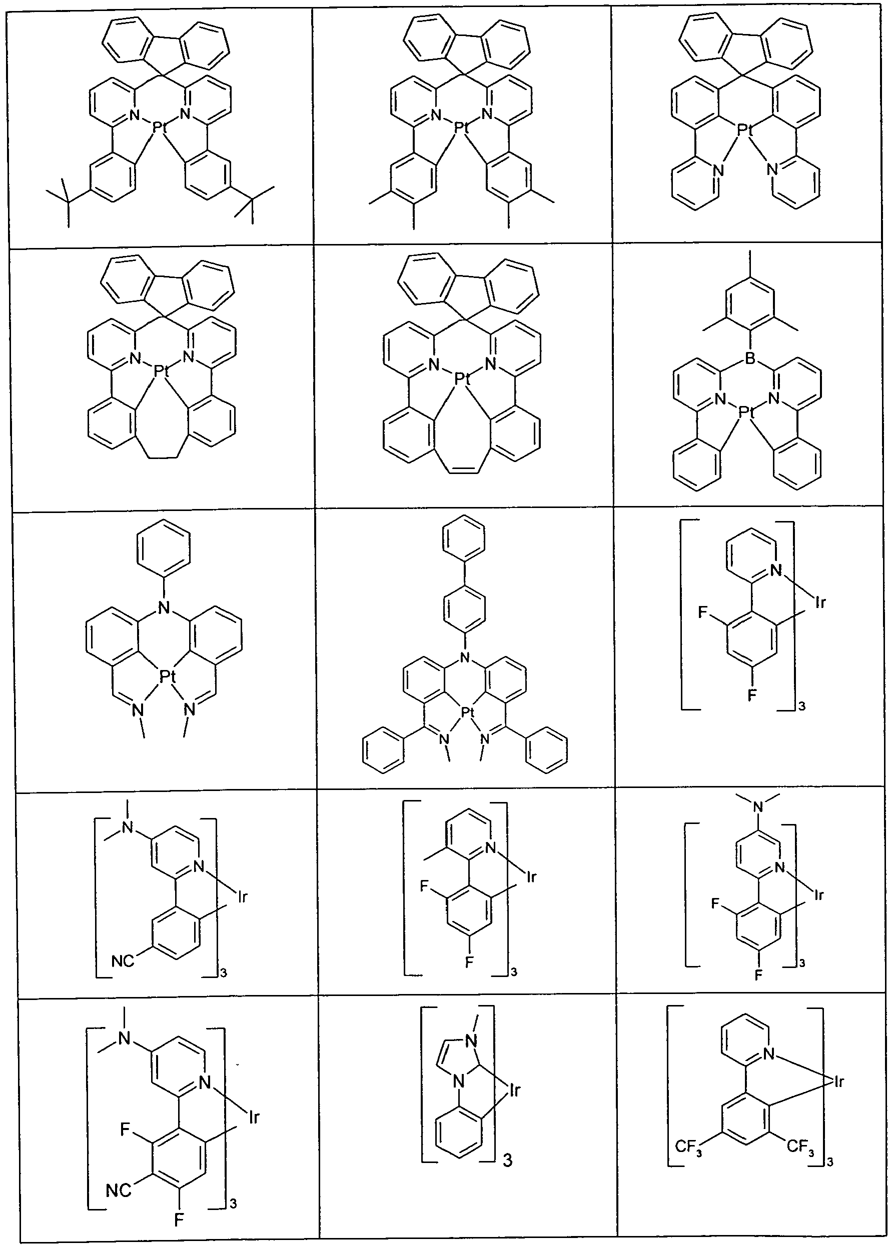

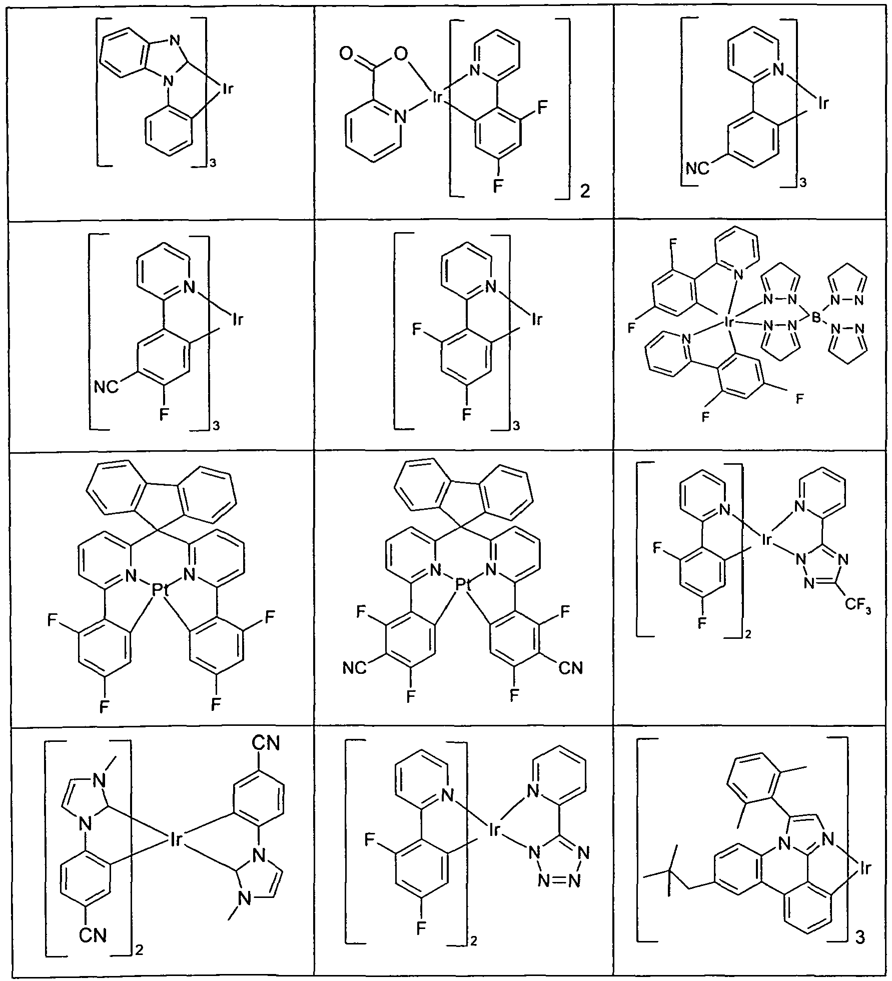

Bevorzugte fluoreszierende Dotanden sind ausgewählt aus der Klasse der Monostyrylamine, der Distyrylamine, der Tristyrylamine, der Tetrastyrylamine, der Styrylphosphine, der Styrylether und der Arylamine. Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält. Die Styrylgruppen sind besonders bevorzugt Stilbene, die auch noch weiter substituiert sein können. Entsprechende Styrylphosphine und Styrylether sind in Analogie zu den Aminen definiert. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte fluoreszierende Dotanden sind gewählt aus Indenofluorenaminen bzw. -diaminen, beispielsweise gemäß WO 2006/122630, Benzoindenofluorenaminen bzw. -diaminen, beispielsweise gemäß WO 2008/006449, und Dibenzoindenofluorenaminen bzw. -diaminen, beispielsweise gemäß WO 2007/140847. Beispiele für fluoreszierende Dotanden aus der Klasse der Styrylamine sind substituierte oder unsubstituierte Tristilbenamine oder die fluoreszierenden Dotanden, die in WO 2006/000388, WO 2006/058737, WO 2006/000389, WO 2007/065549 und WO 2007/115610 beschrieben sind. Weiterhin bevorzugt sind die in WO 2010/012328 offenbarten kondensierten Kohlenwasserstoffe. Weiterhin können auch die Verbindungen gemäß Formel (I) oder (II) als fluoreszierende Dotanden verwendet werden.

Geeignete fluoreszierende Dotanden sind weiterhin die in in JP 2006/001973, WO 2004/047499, WO 2006/098080, WO 2007/065678, US 2005/0260442 und WO 2004/092111 offenbarten Strukturen.

Als Matrixmaterialien, bevorzugt für fluoreszierende Dotanden, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Weiterhin sind als Matrixmaterialien die Verbindungen gemäß Formel (I) oder (II) bevorzugt. Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Geeignete Matrixmaterialien, bevorzugt für fluoreszierende Dotanden, sind beispielsweise in WO 2004/018587, WO 2008/006449, US 5935721, US 2005/0181232, JP 2000/273056, EP 681019, US 2004/0247937 und US 2005/0211958 offenbart.

Bevorzugte Matrixmaterialien für phosphoreszierende Dotanden sind Carbazolderivate (z. B. CBP (N,N-Biscarbazolylbiphenyl) oder Verbindungen gemäß WO 2005/039246, US 2005/0069729, JP 2004/288381, EP 1205527 oder WO 2008/086851), Triarylamine, Azacarbazole (z. B. gemäß EP 1617710, EP 1617711, EP 1731584, JP 2005/347160), Indolocarbazolderivate, z. B. gemäß WO 2007/063754 oder WO 2008/056746, Ketone (z. B. gemäß WO 2004/093207 oder WO 2010/006680), Phosphinoxide, Sulfoxide und Sulfone (z. B. gemäß WO 2005/003253), Oligophenylene, aromatische Amine (z. B. gemäß US 2005/0069729), bipolare Matrixmaterialien (z. B. gemäß WO 2007/137725), Silane (z. B. gemäß WO 2005/111172), Azaborole oder Boronester, z. B. gemäß WO 2006/117052, Triazinderivate, z. B. gemäß WO 2010/015306, WO 2007/063754 oder WO 2008/056746, Zinkkomplexe (z. B. gemäß WO 2009/062578) Aluminiumkomplexe (z. B. BAIq), Diazasilol- und Tetraazasilol-Derivate, z. B. gemäß WO 2010/054730, Indenocarbazolderivate, z. B. gemäß WO 2010/136109 und WO 2011/000455 oder Diazaphosphole, z. B. gemäß WO 2010/054730.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungenbeispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Ba/Ag, Ba/Al oder Mg/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink-Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) oder (II) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Die organischen Elektrolumineszenzvorrichtungen enthaltend eine oder mehrere erfindungsgemäße Verbindungen können in Displays, als Lichtquellen in Beleuchtungsanwendungen sowie als Lichtquellen in medizinischen und/oder kosmetischen Anwendungen (z.B. Lichttherapie) eingesetzt werden.

Bei Verwendung der Verbindungen gemäß Formel (I) oder (II) in einer organischen Elektrolumineszenzvorrichtung können einer oder mehrere der im Folgenden genannten Vorteile realisiert werden:
Die erfindungsgemäßen Verbindungen eignen sich sehr gut zur Verwendung als Matrixmaterialien für phosphoreszierende Dotanden sowie zur Verwendung als Elektronentransportmaterialien. Bei Verwendung der erfindungsgemäßen Verbindungen in diesen Funktionen werden gute Leistungseffizienzen, geringe Betriebsspannungen und gute Lebensdauern der organischen Elektrolumineszenzvorrichtungen erhalten.

Weiterhin zeichnen sich die erfindungsgemäßen Verbindungen durch eine hohe Oxidationsstabilität in Lösung aus, was sich vorteilhaft bei der Aufreinigung und Handhabung der Verbindungen sowie bei ihrer Verwendung in elektronischen Vorrichtungen auswirkt.

Ferner sind die erfindungsgemäßen Verbindungen temperaturstabil und können somit weitgehend zersetzungsfrei sublimiert werden. Die Aufreinigung der Verbindungen wird dadurch erleichtert, und die Verbindungen können in höherer Reinheit erhalten werden, was sich positiv auf die Leistungsdaten der elektronischen Vorrichtungen enthaltend die Materialien auswirkt. Insbesondere können dadurch Vorrichtungen mit längeren operativen Lebensdauern hergestellt werden.

Die Erfindung wird durch die nachfolgenden Anwendungsbeispiele näher erläutert, wobei die Erfindung nicht auf den Umfang der Beispiele beschränkt ist.

### Anwendungsbeispiele

### A) Synthesebeispiele

Die nachfolgenden Synthesen werden, sofern nicht anders angegeben, unter einer Schutzgasatmosphäre in getrockneten Lösungsmitteln durchgeführt. Die angegebenen Lösungsmittel und Reagenzien können kommerziell bezogen werden.

### Beispiel 1: 11-[3-(4,6-Diphenyl-[1,3,5]triazin-2-yl)-phenyl]-10,10-dimethyl-10H-indolo[1,2-a]indol

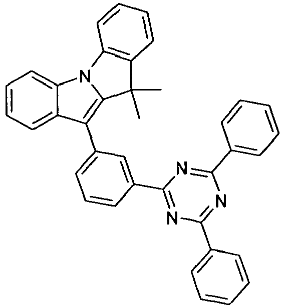

1. Stufe:
   50 g (285 mmol) 2-Methylindol-2-carboxylat, 116.4 g (571 mmol) Iodbenzol und 98.5 g K₂CO₃ (712.5 mmol) werden in 1L Toluol suspendiert. Zu dieser Suspension werden 15.8 g (114 mmol) Cul und 10.06 g N,N'-Dimethylendiamin (114 mmol) gegeben. Die Reaktionsmischung wird 48 h unter Rückfluss erhitzt. Nach Erkalten wird der Niederschlag über einen Faltenfilter abfiltriert. Die Reaktionslösung wird im Anschluss zwischen Essigester und Wasser verteilt, die organische Phase dreimal mit Wasser gewaschen, über Na₂SO₄ getrocknet und im Vakuum eingeengt. Das zurückgebliebene schwarzgrüne Öl wird mit Heptan:Toluol über Kieselgel filtriert. Der eingedampfte Filtrat-Rückstand wird anschließend aus Methanol umkristallisiert. Ausbeute: 57 g 1-Phenyl-1 H-indol-2-methylcarboxylat (80%)
2. Stufe:
   57 g (227 mmol) 1-Phenyl-1H-indol-2-methylcarboxylat werden in 500 mL Dimethylformamid vorgelegt. Anschließend tropft man unter Lichtausschluss bei 0 °C eine Lösung aus 40.4 g (227 mmol) NBS in 100 ml Dimethylformamid hinzu, lässt auf RT kommen und rührt 4 h weiter bei dieser Temperatur. Anschließend wird die Mischung auf Eiswasser gegossen. Das Präzipitat wird filtriert, mit Wasser gewaschen und anschließend mit Heptan gewaschen. Das Produkt wird mit Heptan heiß ausgerührt und abgesaugt. Ausbeute: 71.1 g, (95 %)
3. Stufe:
   57.48 g wasserfreies Cer(III)-chlorid (233 mmol) wird in 700 mL trockenem THF vorgelegt. Zu dieser Lösung werden 70 g (212 mmol) 1-Phenyl-3-brom-1H-indol-2-methylcarboxylat portionsweise zudosiert und 1 h gerührt. Die Reaktionsmischung wird abgekühlt und bei 5° C 212 mL (636 mmol) Methylmagnesiumchlorid-Lösung (3 mol/L in THF) innerhalb von 40 min zugetropft. Nach einer Stunde wird das Reaktionsgemisch vorsichtig auf Eis gegossen und dreimal mit Dichlormethan extrahiert. Die vereinigten organischen Phasen werden über Na₂SO₄ getrocknet und eingeengt. Der Rückstand wird aus Toluol umkristallisiert. Ausbeute: 65.8 g (94 %)
4. Stufe:
   157 g Polyphosphorsäure (1.6 mol) und 104 g Methansulfonsäure (1.1 mol) werden in 1 L CH₂Cl₂ vorgelegt. Zu dieser Lösung werden 65 g (197 mmol) 2-(3-Bromo-1-phenyl-1 H-indol-2-yl)-propan-2-ol in Dichlormethan-Lösung (150 mL) innerhalb von 30 min zugetropft und 1 h bei 50 °C gerührt. Nach dieser Zeit wird das Reaktionsgemisch abgekühlt, vorsichtig auf Eis gegossen und dreimal mit Dichlormethan extrahiert. Die vereinigten organischen Phasen werden über Na₂SO₄ getrocknet und eingeengt. Der Rückstand wird aus Toluol umkristallisiert. Ausbeute: 52.3 g 11-Bromo-10,10-dimethyl-10H-indolo[1,2-a]indol (85 %)
5. Stufe:
   20 g (57 mmol) 11-Bromo-10,10-dimethyl-10H-indolo[1,2-a]indol, 25 g (57 mmol) 2,4-Diphenyl-6-[3-(4,4,5,5-tetramethyl-[1,3,2]dioxaborolan-2-yl)-phenyl]-[1,3,5]triazin und 86 mL einer 2M Natriumcarbonatlösung werden in 500 ml Ethylenglycoldimethylether suspendiert. Zu dieser Suspension werden 1.66 g (1.43 mmol) Pd(PPh₃)₄ gegeben. Die Reaktionsmischung wird 5 h unter Rückfluss erhitzt. Nach Erkalten wird der ausgefallene Feststoff abgesaugt und mit Wasser und Ethanol gewaschen und getrocknet. Der Rückstand wird mit Toluol heiß extrahiert, aus Toluol umkristallisiert und abschließend im Hochvakuum sublimiert. Die Reinheit beträgt 99,9%. Ausbeute: 19.5 g (59 %).

### Beispiel 2: Bis-biphenyl-4-yl-[4-(11,11-dimethyl-11H-indolo[1,2-a]indol-10-yl)-phenyl]-amin

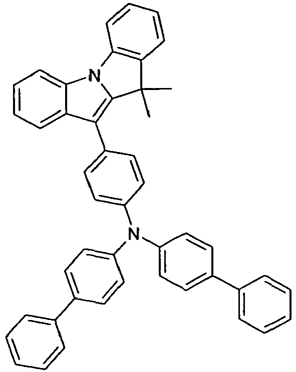

20 g (64 mmol) 11-Bromo-10,10-dimethyl-10H-indolo[1,2-a]indol (4. Stufe des Beispiels 1), 33.5 g (64 mmol) Bis-biphenyl-4-yl-[4-(4,4,5,5-tetramethyl-[1,3,2]dioxaborolan-2-yl)-phenyl]-amin und 96 mL einer 2M Natriumcarbonatlösung werden in 500 ml Ethylenglycoldimethylether suspendiert. Zu dieser Suspension werden 1.66 g (1.43 mmol) Pd(PPh₃)₄ gegeben. Die Reaktionsmischung wird 10 h unter Rückfluss erhitzt. Nach Erkalten wird der ausgefallene Festsstoff abgesaugt, mit Wasser und Ethanol gewaschen und getrocknet. Der Rückstand wird mit Toluol heiß extrahiert, aus Toluol umkristallisiert und anschließend im Hochvakuum sublimiert. Die Reinheit beträgt 99.9 %. Ausbeute: 26 g (65 %).

### Beispiel 3: Bis-10,10'-[(4-4'-Biphenyl)-11,11-dimethyl-11H-indolo[1,2-a]indol

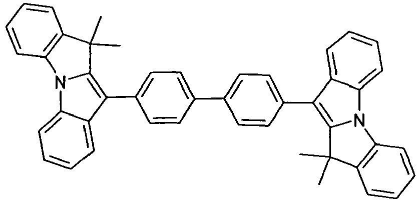

30 g (96 mmol) 11-Bromo-10,10-dimethyl-10H-indolo[1,2-a]indol (4. Stufe des Beispiels 1), 11.6 g (48 mmol) 4,4'-Biphenyl-diboronsäure und 145 mL einer 2M Natriumcarbonatlösung werden in 600 ml Ethylenglycoldimethylether suspendiert. Zu dieser Suspension werden 2.77 g (2.4 mmol) Pd(PPh₃)₄ gegeben. Die Reaktionsmischung wird 12 h unter Rückfluss erhitzt. Nach Erkalten wird der ausgefallene Feststoff abgesaugt, mit Wasser und Ethanol gewaschen und getrocknet. Der Rückstand wird mit Toluol heiß extrahiert, aus Toluol umkristallisiert und anschließend im Hochvakuum sublimiert. Die Reinheit beträgt 99.9 %. Ausbeute: 35.6 g (60 %).

### Beispiel 4

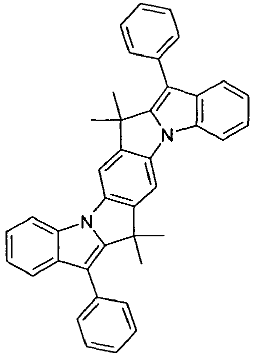

1. Stufe:
   50 g (285 mmol) 2-Methylindol-2-carboxylat, 47 g (143 mmol) 1,4-Diiodbenzol und 98.5 g K₂CO₃ (712.5 mmol) werden in 1 L Toluol suspendiert. Zu dieser Suspension werden 15.8 g (114 mmol) Cul und 10.06 g N,N'-Dimethylendiamin (114 mmol) gegeben. Die Reaktionsmischung wird 48 h unter Rückfluss erhitzt. Nach Erkalten wird der Niederschlag über einen Faltenfilter abfiltriert. Die Reaktionslösung wird im Anschluss zwischen Essigester und Wasser verteilt, die organische Phase wird dreimal mit Wasser gewaschen, über Na₂SO₄ getrocknet und einrotiert. Das zurückgebliebene schwarzgrüne Öl wird mit Heptan:Toluol über Kieselgel filtriert. Der eingedampfte Filtrat-Rückstand wird aus Methanol umkristallisiert. Ausbeute: 48.5 g 1,4-Di-(1H-indol-2-methylcarboxylat-1-yl)benzol (80%).
2. Stufe:
   45 g (106 mmol) 1,4-Di-(1H-indol-2-methylcarboxylat-1-yl)benzol wird in 500 mL Dimethylformamid vorgelegt. Anschließend tropft man unter Lichtausschluss bei 0 °C eine Lösung aus 37.5 g (212 mmol) NBS in 100 ml Dimethylformamid hinzu, lässt auf RT kommen und rührt 4 h weiter bei dieser Temperatur. Anschließend wird die Mischung auf Eiswasser gegossen. Das Präzipitat wird filtriert, mit Wasser gewaschen und anschließend mit Heptan gewaschen. Das Produkt wird mit Heptan heiß ausgerührt und abgesaugt. Ausbeute: 55.5 g (90 %).
3. Stufe:
   30.9 g wasserfreies Cer(III)-chlorid (172 mmol) wird in 700 mL trockenem THF vorgelegt. Zu dieser Lösung werden 50 g (85.9 mmol) 1,4-Di-(1H-indol-3-brom-2-methylcarboxylat-1-yl)benzol portionsweise zudosiert und es wird 1 h gerührt. Die Reaktionsmischung wird abgekühlt und es wird bei 5 °C 200 mL (601 mmol) Methylmagnesiumchlorid-Lösung (3 mol/L in THF) innerhalb von 40 min zugetropft. Nach einer Stunde wird das Reaktionsgemisch vorsichtig auf Eis gegossen und dreimal mit Dichlormethan extrahiert. Die vereinigten organischen Phasen werden über Na₂SO₄ getrocknet und eingeengt. Der Rückstand wird aus Toluol umkristallisiert. Ausbeute: 47 g (95 %).
4. Stufe:
   26 g Polyphosphorsäure (270 mmol) und 25.9 g Methansulfonsäure (270 mmol) werden in 500 mL CH₂Cl₂ vorgelegt. Zu dieser Lösung werden 45 g (77.3 mmol) der Verbindung aus Stufe 3 in Dichlormethan-Lösung (150 mL) innerhalb von 30 min zugetropft und es wird 1 h bei 50 °C gerührt. Nach dieser Zeit wird das Reaktionsgemisch abgekühlt, vorsichtig auf Eis gegossen und dreimal mit Dichlormethan extrahiert. Die vereinigten organischen Phasen werden über Na₂SO₄ getrocknet und eingeengt. Der Rückstand wird aus Toluol umkristallisiert. Ausbeute: 39.2 g (90 %).
5. Stufe:
   35 g (62 mmol) der Verbindung aus der 4. Stufe, 15 g (124 mmol) Phenylboronsäure und 124 mL einer 2M Natriumcarbonatlösung werden in 700 ml Ethylenglycoldimethylether suspendiert. Zu dieser Suspension werden 3.58 g (3.1 mmol) Pd(PPh₃)₄ gegeben. Die Reaktionsmischung wird 5 h unter Rückfluss erhitzt. Nach Erkalten wird der ausgefallene Feststoff abgesaugt, mit Wasser und Ethanol gewaschen und getrocknet. Der Rückstand wird mit Toluol heiß extrahiert, aus Toluol umkristallisiert und anschließend im Hochvakuum sublimiert. Die Reinheit beträgt 99.9 %. Ausbeute: 21.8 g (65 %).

### B) Device-Beispiele

Die Herstellung der erfindungsgemäßen OLEDs erfolgt nach einem allgemeinen Verfahren gemäß WO 2004/058911, das auf die hier beschriebenen Gegebenheiten (Schichtdickenvariation, Materialien) angepasst wird.

In den folgenden Beispielen E1 bis E9 (siehe Tabellen 1 und 2) werden die Daten verschiedener OLEDs vorgestellt. Glasplättchen, die mit strukturiertem ITO (Indium Zinn Oxid) der Dicke 150 nm beschichtet sind, werden zur verbesserten Prozessierung mit 20 nm PEDOT beschichtet (Poly(3,4-ethylendioxy-2,5-thiophen), aus Wasser aufgeschleudert; bezogen von H. C. Starck, Goslar, Deutschland). Diese beschichteten Glasplättchen bilden die Substrate, auf welche die OLEDs aufgebracht werden. Die OLEDs haben prinzipiell folgenden Schichtaufbau: Substrat / Optionale Lochinjektionsschicht (HIL) / Lochtransportschicht (HTL) / Optionale Zwischenschicht (IL) / Elektronenblockerschicht (EBL) / Emissionsschicht (EML) / Optionale Lochblockierschicht (HBL) / Elektronentransportschicht (ETL) / Optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:**3**:TEG1 (70%:20%:10%) bedeutet hierbei, dass das Material IC1 in einem Volumenanteil von 70%, **3** in einem Anteil von 20% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent in Abhängigkeit der Leuchtdichte, berechnet aus Strom-Spannungs-Leuchtdichte-Kennlinien (IUL-Kennlinien) unter Annahme einer lambertschen Abstrahlcharakteristik) bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Die Daten der OLEDs sind in Tabelle 2 zusammengefasst. Je nach Substitutionsmuster lassen sich die erfindungsgemäßen Materialien in unterschiedlichen Schichten und Funktionen einsetzen. Dabei sind gute bis sehr gute Werte für Effizienz und Spannung erreichbar.

Gemäß einer bevorzugten Ausführungsform werden die Verbindungen als Matrixmaterialien für rot und für grün phosphoreszierende Emitter verwendet (Verbindungsbeispiele **1, 3** und **4**). Device-Beispiele hierzu sind E1-E4, E7 und E9. Hierbei stellen E1 und E2 Beispiele dar, in denen die erfindungsgemäßen Verbindungen als einzige Matrixmaterialien eingesetzt werden, und E3, E4, E7 und E9 stellen Beispiele dar, in denen die erfindungsgemäßen Verbindungen in Kombination mit einem weiteren Matrixmaterial eingesetzt werden (Mixed-Matrix-Systeme).

Weiterhin können die erfindungsgemäßen Verbindungen vorteilhaft als Lochtransportmaterialien eingesetzt werden, wie anhand des Verbindungsbeispiels **2** in den Devicebeispielen E5 und E6 gezeigt wird.

Nochmals weiterhin können die erfindungsgemäßen Verbindungen als Elektronentransportmaterialien eingesetzt werden, wie anhand des Verbindungsbeispiels **1** im Devicebeispiel E5 gezeigt wird.

**Tabelle 1: Aufbau der OLEDs**

| Bsp | HIL Dicke | HTL Dicke | IL Dicke | EBL Dicke | EML Dicke | HBL Dicke | ETL Dicke | EIL Dicke |
|---|---|---|---|---|---|---|---|---|
| E1 | --- | SpA1 20nm | --- | NPB 20nm | **1**:TER1 (85%:15%) 30nm | --- | Alq₃ 20nm | LiF 1nm |
| E2 | --- | SpA1 70nm | HATC N 5nm | BPA1 90nm | **1**:TEG1 (90%:10%) 30nm | --- | ST2:LiQ (50%:50%) 40nm | --- |
| E3 | --- | SpA1 70nm | HATC N 5nm | BPA1 90nm | ST1:**3**:TEG1 (30%:60%:10%) 30nm | IC1 10nm | ST2:LiQ (50%:50%) 30nm | --- |
| E4 | --- | SpA1 70nm | HATC N 5nm | BPA1 90nm | IC1:**3**:TEG1 (30%:60%:10%) 30nm | --- | ST2:LiQ (50%:50%) 40nm | --- |
| E5 | HATCN 5nm | SpA1 140nm | --- | **2** 20nm | M1:D1 (95%:5%) 20nm | --- | ST1:LiQ (50%:50%) 30nm | --- |
| E6 | --- | SpA1 70nm | HATC N 5nm | **2** 90nm | IC1:TEG1 (90%:10%) 30nm | ST1 10nm | ST1:LiQ (50%:50%) 30nm | - |
| E7 | --- | SpA1 20nm | --- | NPB 20nm | ST1:**2**:TER1 (70%:15%:15%) 30nm | --- | Alq₃ 20nm | LiF 1nm |
| E8 | --- | SpA1 70nm | HATC N 5nm | BPA1 90nm | ST1:TEG1 (90%:10%) 30nm | --- | **1** 40nm | LiQ 3nm |
| E9 | --- | SpA1 70nm | HATC N 5nm | BPA1 90nm | IC1:**4**:TEG1 (70%:20%:10%) 30nm | IC1 10nm | ST2:LiQ (50%:50%) 30nm | --- |

**Tabelle 2: Daten der OLEDs**

| Bsp. | U1000 (V) | SE1000 (cd/A) | LE1000 (Im/W) | EQE 1000 | CIE x/y |
|---|---|---|---|---|---|
| E1 | 4.7 | 7.7 | 5.1 | 12.8% | 0.69/0.31 |
| E2 | 3.7 | 51 | 43 | 14.1% | 0.36/0.60 |
| E3 | 3.6 | 53 | 46 | 14.8% | 0.36/0.60 |
| E4 | 3.8 | 50 | 41 | 13.9% | 0.36/0.60 |
| E5 | 4.2 | 7.5 | 5.6 | 6.1% | 0.14/0.15 |
| E6 | 3.5 | 55 | 50 | 15.3% | 0.36/0.60 |
| E7 | 4.7 | 6.5 | 4.3 | 10.8% | 0.39/0.61 |
| E8 | 4.3 | 48 | 35 | 13.2% | 0.36/0.60 |
| E9 | 3.5 | 56 | 50 | 15.6% | 0.37/0.61 |

**Tabelle 3: Strukturformel der Materialien für die OLEDs**

| | |
|---|---|
| 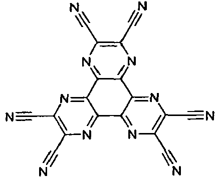 | 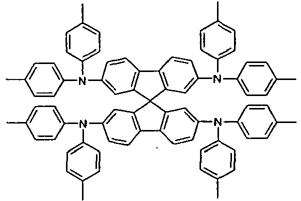 |
| HATCN | SpA1 |
| 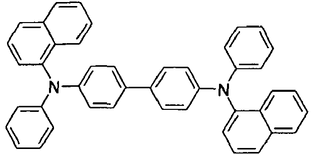 | 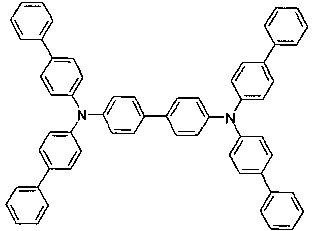 |
| NPB | BPA1 |
| 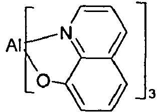 | 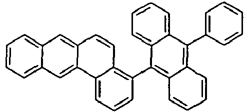 |
| Alq₃ | M1 |
| 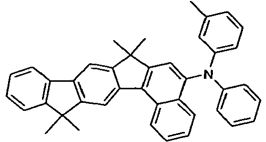 | 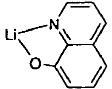 |
| D1 | LiQ |
| 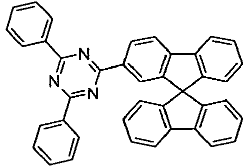 | 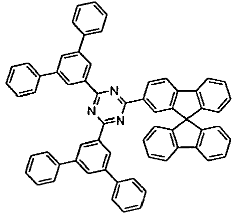 |
| ST1 | ST2 |
| 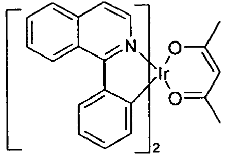 | 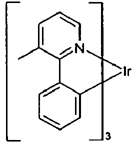 |
| TER1 | TEG1 |
| 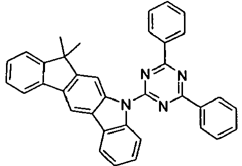 | |
| IC1 | |
| 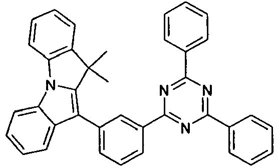 | 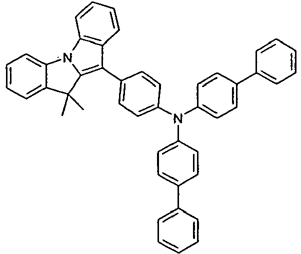 |
| **1** | **2** |
| 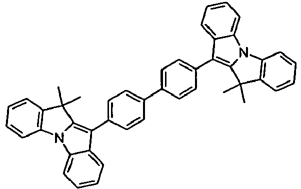 | 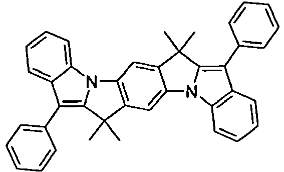 |
| **3** | **4** |

## Patentansprüche

1. Elektronische Vorrichtung, enthaltend Anode, Kathode sowie mindestens eine organische Schicht, wobei die organische Schicht mindestens eine Verbindung der Formel (I) oder (II) enthält 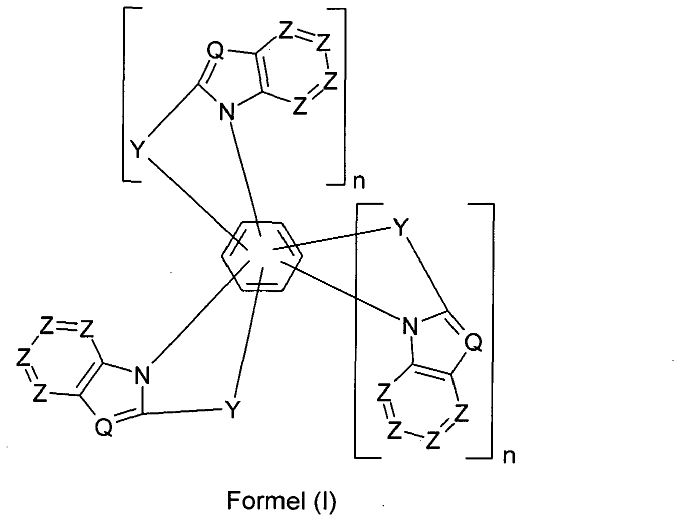 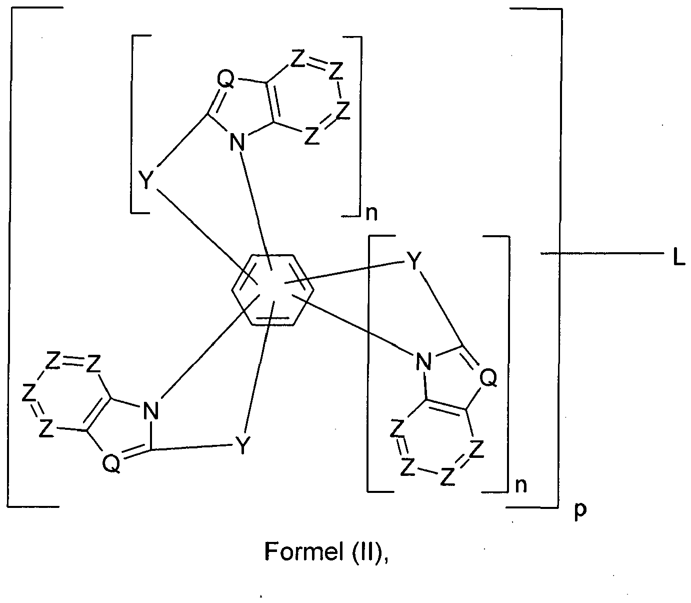 wobei gilt:
Y ist gleich C(R¹)₂;
Q, Z sind gleich CR¹;
L ist ausgewählt aus C=O, C=NR¹, Si(R¹)₂, NR¹, P(=O)(R¹), O, S, SO, SO₂, Alkylengruppen mit 1 bis 20 C-Atomen oder Alkenylen- oder Alkinylengruppen mit 2 bis 20 C-Atomen, wobei bei den genannten Gruppen eine oder mehrere CH₂-Gruppen durch Si(R¹)₂, O, S, C=O, C=NR¹, C(=O)O, (C=O)NR¹, NR¹, P(=O)(R¹), SO oder SO₂ ersetzt sein können und wobei ein oder mehrere H-Atome in den genannten Gruppen durch D, F, Cl, Br, I, CN oder NO₂ ersetzt sein können, und aromatischen oder heteroaromatischen Ringsystemen mit 5 bis 60 aromatischen Ringatomen, die jeweils durch einen oder mehrere Reste R¹ substituiert sein können, und beliebigen Kombinationen aus 1, 2, 3, 4 oder 5 gleichen oder verschiedenen Gruppen ausgewählt aus den oben genannten Gruppen; oder L ist eine Einfachbindung, wobei p in diesem Fall gleich 2 ist;
R¹ ist bei jedem Auftreten gleich oder verschieden H, D, F, Cl, Br, I, B(OR²)₂, CHO, C(=O)R², CR²=C(R²)₂, CN, C(=O)OR², C(=O)N(R²)₂, Si(R²)₃, N(R²)₂, NO₂, P(=O)(R²)₂, OSO₂R², OR², S(=O)R², S(=O)₂R², eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch -R²C=CR²-, -C=C-, Si(R²)₂, Ge(R²)₂, Sn(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, eine Aryloxy- oder Heteroaryloxygruppe, mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die durch einen oder mehrere Reste R² substituiert sein kann und wobei in der Alkylgruppe auch einzelne H-Atome und/oder CH₂-Gruppen durch die oben genannten Gruppen ersetzt sein können, wobei zwei oder mehr Reste R¹ miteinander verknüpft sein können und einen Ring oder ein Ringsystem bilden können;
R² ist bei jedem Auftreten gleich oder verschieden H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C=C-, Si(R³)₂, Ge(R³)₂, Sn(R³)₂, C=O, C=S, C=Se, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, eine Aryloxy- oder Heteroaryloxygruppe mit 5 bis 60 aromatischen Ringatomen, die durch einen oder mehrere Reste R³ substituiert sein kann, oder eine Aralkyl- oder Heteroaralkylgruppe mit 5 bis 60 aromatischen Ringatomen, die durch einen oder mehrere Reste R³ substituiert sein kann und wobei in der Alkylgruppe auch einzelne H-Atome und/oder CH₂-Gruppen durch die oben genannten Gruppen ersetzt sein können, wobei zwei oder mehr Reste R² miteinander verknüpft sein können und einen Ring oder ein Ringsystem bilden können;
R³ ist bei jedem Auftreten gleich oder verschieden H, D, F oder ein aliphatischer, aromatischer und/oder heteroaromatischer organischer Rest mit 1 bis 20 C-Atomen, in dem auch ein oder mehrere H-Atome durch D oder F ersetzt sein können; dabei können zwei oder mehr Substituenten R³ auch miteinander verknüpft sein und einen Ring oder ein Ringsystem bilden;
n ist bei jedem Auftreten gleich oder verschieden 0 oder 1, wobei für den Fall n=0 die in Klammern stehende Gruppe nicht vorliegt und an den zentralen Benzolring gegebenenfalls stattdessen Gruppen R¹ gebunden sind;
p ist gleich 2, 3, 4, 5 oder 6; und
wobei die Gruppe Y und das Stickstoffatom an beliebigen benachbarten Positionen am aromatischen Sechsring binden können;
wobei in Formel (II) die an L bindenden Molekülteile in eckigen Klammern mit Index p gleich oder verschieden sein können; und
wobei in Formel (II) die Gruppe L an einer beliebigen Position des in eckigen Klammern gesetzten Molekülteils mit Index p gebunden sein kann.

2. Elektronische Vorrichtung nach Anspruch 1, **dadurch gekennzeichnet, dass** p gleich 2 ist.

3. Elektronische Vorrichtung nach Anspruch 1 oder 2, **dadurch gekennzeichnet, dass** die Summe der Werte für n in Formel (I) und die Summe der Werte für n pro Einheit in eckigen Klammern mit Index p in Formel (II) gleich 0 ist.

4. Elektronische Vorrichtung nach einem oder mehreren der Ansprüche 1 bis 3, **dadurch gekennzeichnet, dass** L ausgewählt ist aus einer Einfachbindung, wobei in diesem Fall p = 2 ist, oder aus C=O, NR¹, O oder S, wobei in diesen Fällen p=2 ist, oder aus Alkylengruppen mit 1 bis 10 C-Atomen, Alkenylengruppen mit 2 bis 10 C-Atomen, wobei bei den genannten Gruppen eine oder mehrere CH₂-Gruppen durch C=O, NR¹, P(=O)(R¹), O oder S ersetzt sein können, und Arylen- oder Heteroarylengruppen mit 5 bis 20 aromatischen Ringatomen, welche mit einem oder mehreren Resten R¹ substituiert sein können, oder aus divalenten aromatischen oder heteroaromatischen Ringsystemen der Formel (L-1) 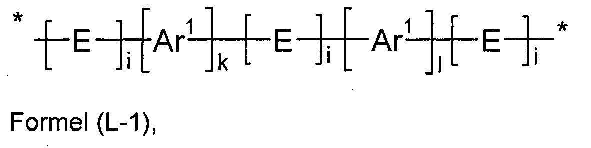 wobei p in diesem Fall gleich 2 ist und wobei weiterhin gilt:
Ar¹ ist bei jedem Auftreten gleich oder verschieden eine Aryl- oder Heteroarylgruppe mit 5 bis 20 aromatischen Ringatomen, welche jeweils mit einem oder mehreren Resten R¹ substituiert sein kann;
E ist bei jedem Auftreten gleich oder verschieden eine Einfachbindung, C=O, NAr¹, P(=O)(R¹), O, S, SO oder SO₂;
i ist bei jedem Auftreten gleich oder verschieden 0 oder 1;
k,l sind bei jedem Auftreten gleich oder verschieden 0, 1, 2 oder 3, wobei die Summe der Werte von k und l größer als 0 ist; und
wobei weiterhin die Gruppen Ar¹ untereinander über eine oder mehrere divalente Gruppen T verbunden sein können, wobei
T bei jedem Auftreten gleich oder verschieden ausgewählt ist aus einer Einfachbindung, BR¹, C(R¹)₂, C=O, C=S, C=NR¹, C=C(R¹)₂, CR¹=CR¹, Si(R¹)₂, NR¹, PR¹, P(=O)R¹, O, S, S=O und S(=O)₂; und
die Symbole * Bindungen der Gruppe L an den Rest der Verbindung markieren.

5. Elektronische Vorrichtung nach einem oder mehreren der Ansprüche 1 bis 4, **dadurch gekennzeichnet, dass** die Verbindung der Formel (I) ausgewählt sind aus den Formeln 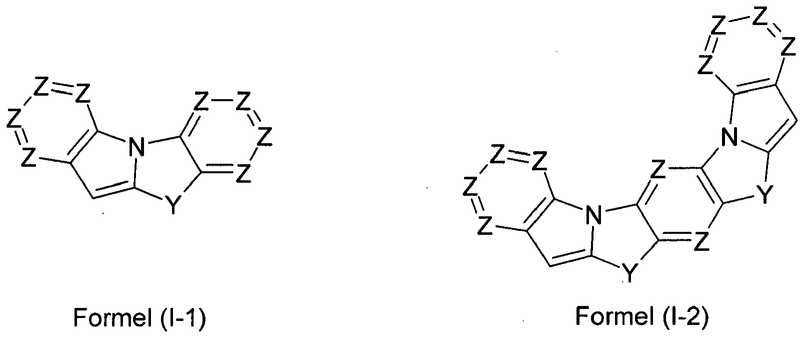 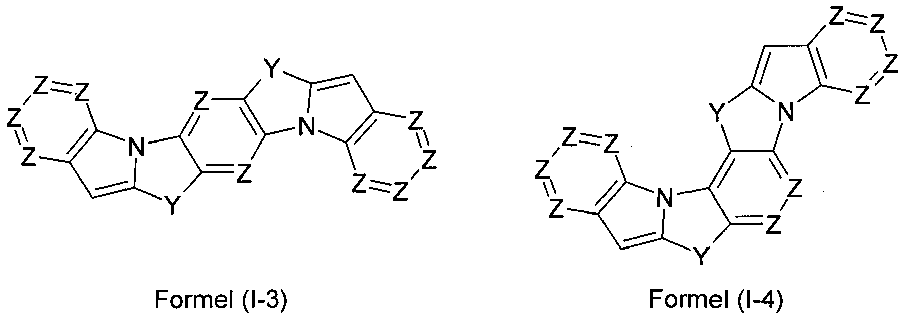 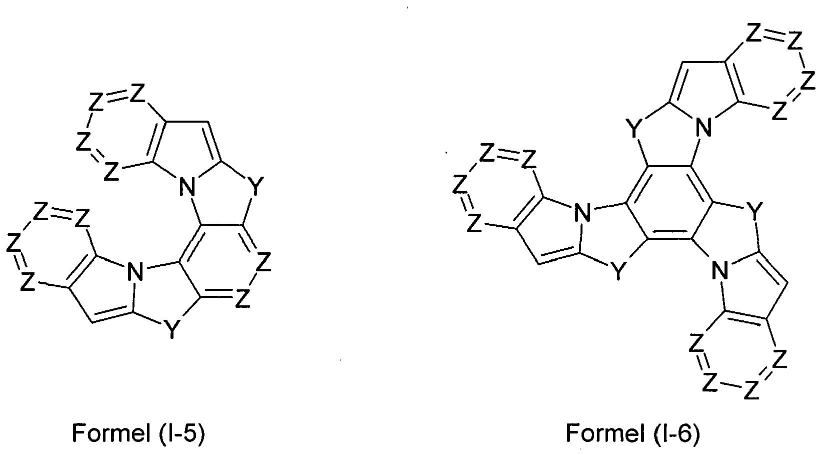 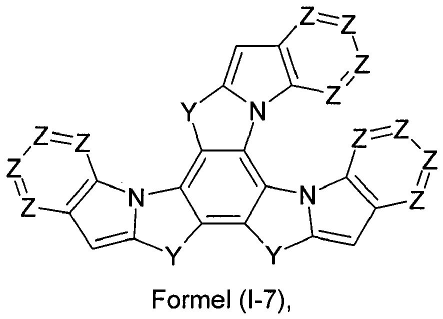 wobei die auftretenden Symbole wie in Anspruch 1 definiert sind und weiterhin die Verbindungen an allen unsubstituierten Positionen mit Resten R¹ substituiert sein können.

6. Elektronische Vorrichtung nach einem oder mehreren der Ansprüche 1 bis 5, **dadurch gekennzeichnet, dass** die Verbindung der Formel (II) ausgewählt sind aus den Formeln 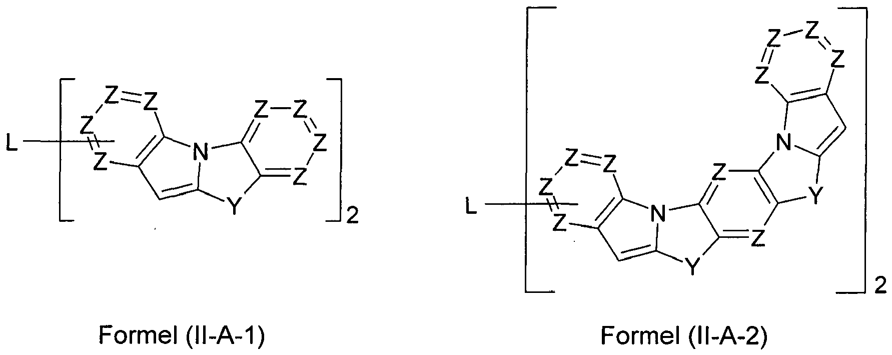 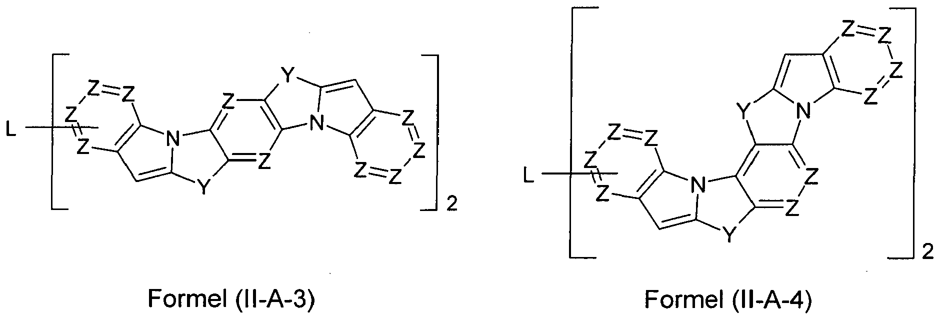 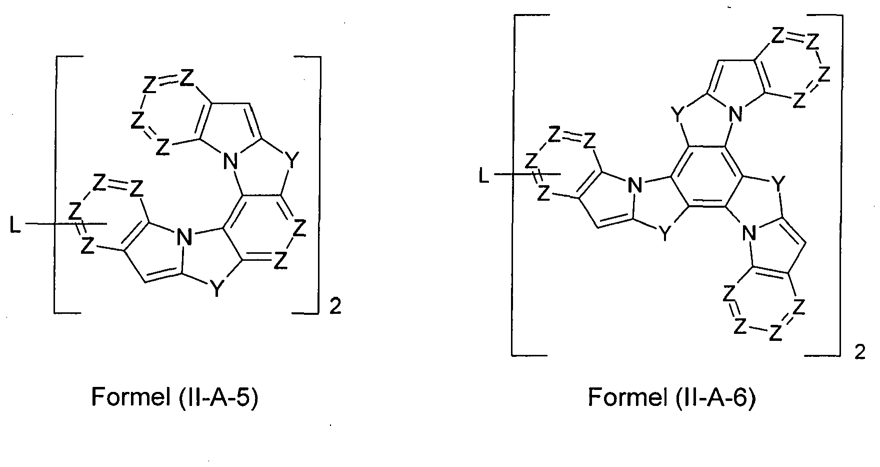 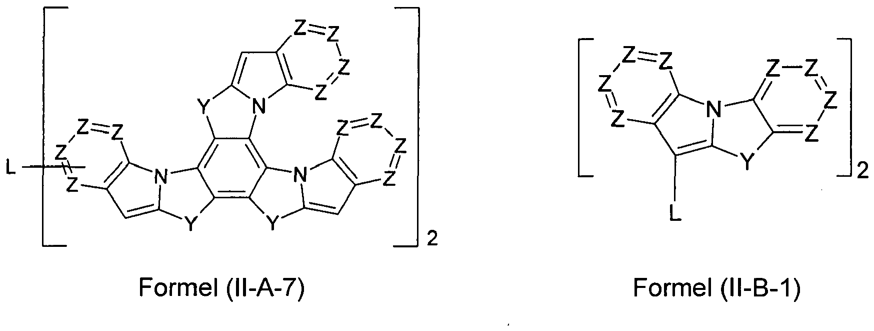 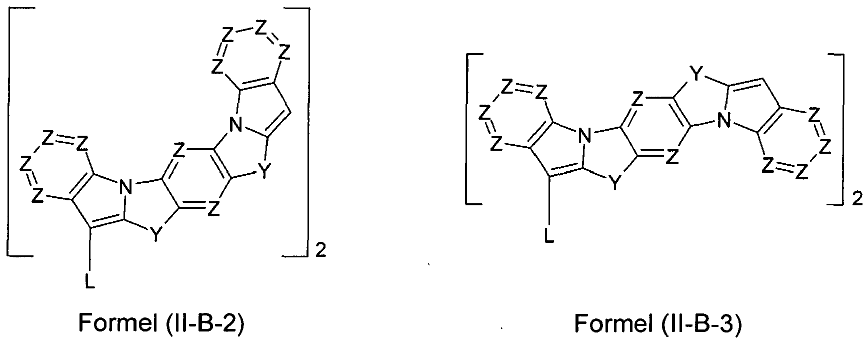 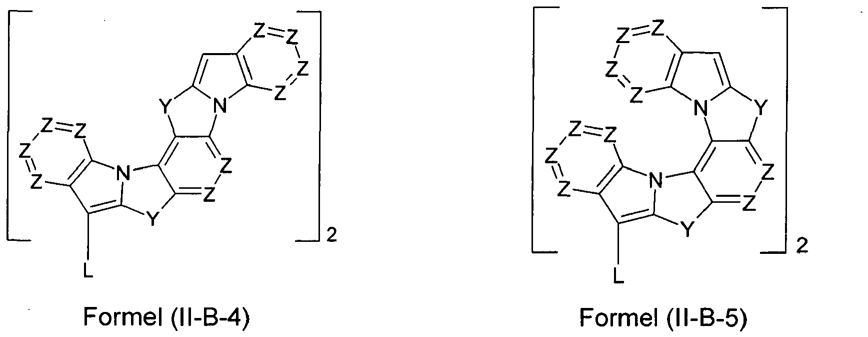 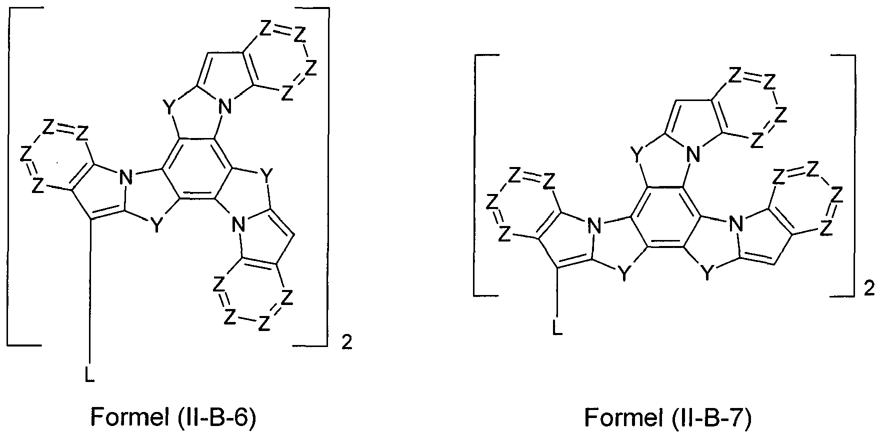 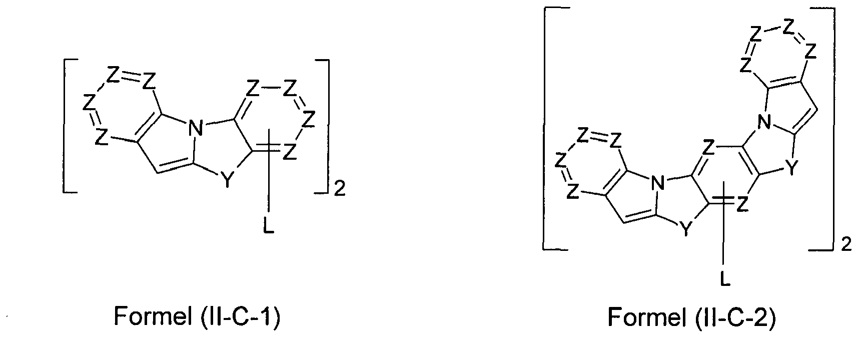 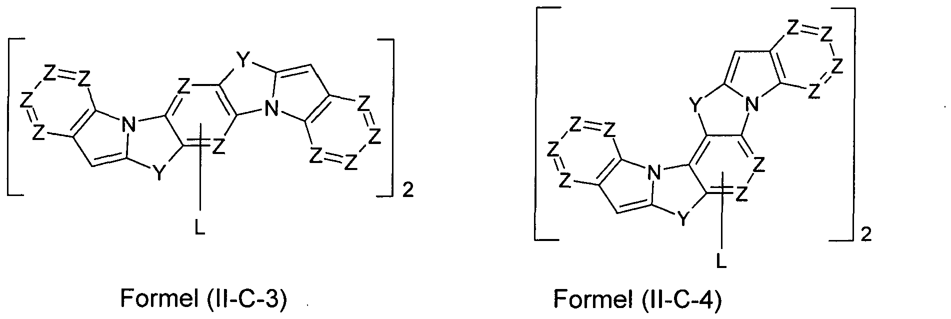 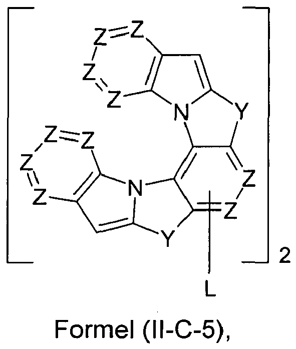 wobei die auftretenden Symbole wie in Anspruch 1 definiert sind und diejenige Gruppe Z, an die die Gruppe L bindet, für ein Kohlenstoffatom steht, und wobei weiterhin die Verbindungen an allen unsubstituierten Positionen mit Resten R¹ substituiert sein können.

7. Elektronische Vorrichtung nach einem oder mehreren der Ansprüche 1 bis 6, **dadurch gekennzeichnet, dass** die Vorrichtung ausgewählt ist aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und organischen Elektrolumineszenzvorrichtungen (OLEDs).

8. Elektronische Vorrichtung nach einem oder mehreren der Ansprüche 1 bis 7, gewählt aus organischen Elektrolumineszenzvorrichtungen, **dadurch gekennzeichnet, dass** die Verbindung gemäß Formel (I) oder (II) als Lochtransportmaterial in einer Lochtransportschicht oder Lochinjektionsschicht vorhanden ist und/oder als Matrixmaterial in einer emittierenden Schicht vorhanden ist und/oder als Elektronentransportmaterial in einer Elektronentransportschicht vorhanden ist.

9. Verbindung gemäß Formel (I) oder (II) des Anspruchs 1, wobei die auftretenden Symbole und Indices wie in Anspruch 1 definiert sind, und die folgenden Verbindungen ausgenommen sind:

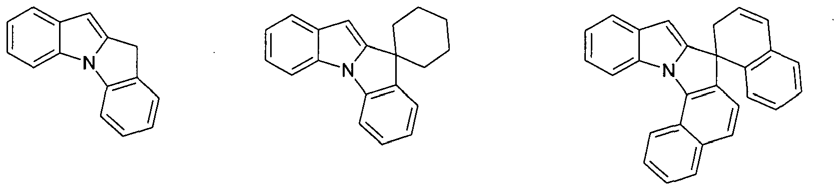

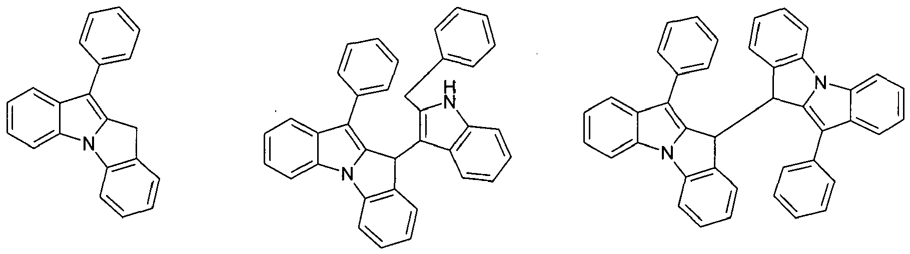

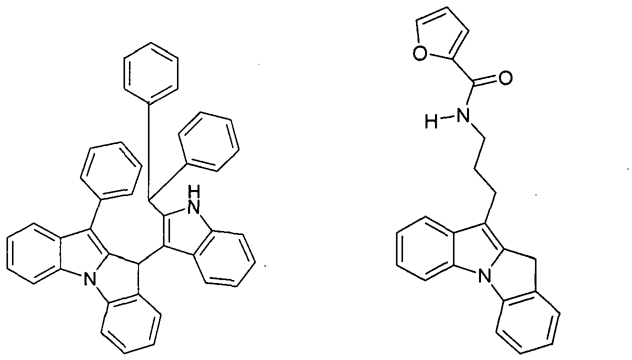

10. Oligomer, Polymer oder Dendrimer enthaltend eine oder mehrere Verbindungen nach Anspruch 9, wobei die Bindung(en) zum Polymer, Oligomer oder Dendrimer an beliebigen, in Formel (I) oder (II) mit R¹ substituierten Positionen lokalisiert sein können.

11. Formulierung enthaltend mindestens eine Verbindung nach Anspruch 9 oder mindestens ein Polymer, Oligomer oder Dendrimer nach Anspruch 10 sowie mindestens ein Lösungsmittel.

## Claims

1. Electronic device, comprising anode, cathode and at least one organic layer, where the organic layer comprises at least one compound of the formula (I) or (II) 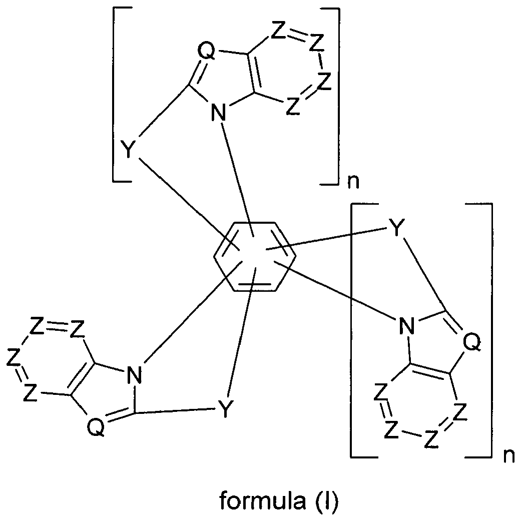 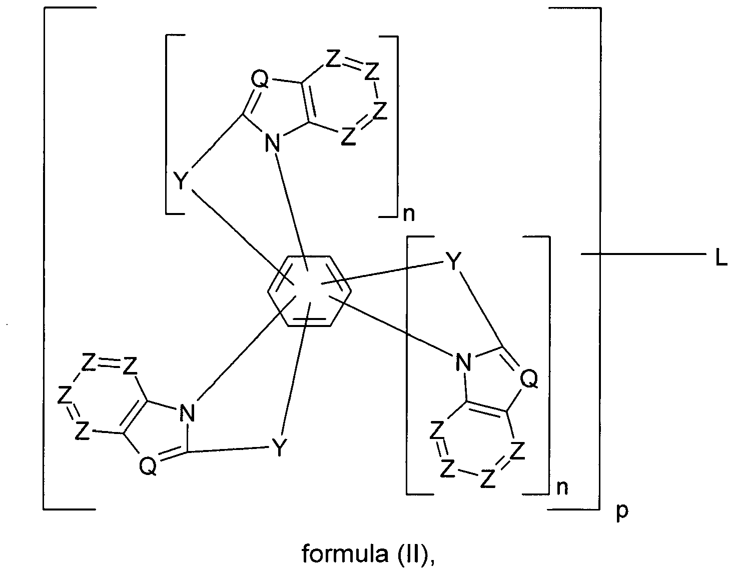 where:
Y is equal to C(R¹)₂;
Q, Z are equal to CR¹;
L is selected from C=O, C=NR¹, Si(R¹)₂, NR¹, P(=O)(R¹), O, S, SO, SO₂, alkylene groups having 1 to 20 C atoms or alkenylene or alkynylene groups having 2 to 20 C atoms, where one or more CH₂ groups in the said groups may be replaced by Si(R¹)₂, O, S, C=O, C=NR¹, C(=O)O, (C=O)NR¹, NR¹, P(=O)(R¹), SO or SO₂ and where one or more H atoms in the said groups may be replaced by D, F, Cl, Br, I, CN or NO₂, and aromatic or heteroaromatic ring systems having 5 to 60 aromatic ring atoms, which may in each case be substituted by one or more radicals R¹, and any desired combinations of 1, 2, 3, 4 or 5 identical or different groups selected from the above-mentioned groups; or L is a single bond, where p in this case is equal to 2;
R¹ is on each occurrence, identically or differently, H, D, F, Cl, Br, I, B(OR²)₂, CHO, C(=O)R², CR²=C(R²)₂, CN, C(=O)OR², C(=O)N(R²)₂, Si(R²)₃, N(R²)₂, NO₂, P(=O)(R²)₂, OSO₂R², OR², S(=O)R², S(=O)₂R², a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R² and where one or more CH₂ groups in the above-mentioned groups may be replaced by -R²C=CR²-, -C≡C-, Si(R²)₂, Ge(R²)₂, Sn(R²)₂, C=O, C=S, C=Se, C=NR², -C(=O)O-, -C(=O)NR²-, NR², P(=O)(R²), -O-, -S-, SO or SO₂ and where one or more H atoms in the above-mentioned groups may be replaced by D, F, Cl, Br, I, CN or NO₂, or an aromatic or heteroaromatic ring system having 5 to 60 aromatic ring atoms, which may in each case be substituted by one or more radicals R², an aryloxy or heteroaryloxy group having 5 to 60 aromatic ring atoms, which may be substituted by one or more radicals R², or an aralkyl or heteroaralkyl group having 5 to 60 aromatic ring atoms, which may be substituted by one or more radicals R² and where, in addition, individual H atoms and/or CH₂ groups in the alkyl group may be replaced by the above-mentioned groups, where two or more radicals R¹ may be linked to one another and may form a ring or a ring system;
R² is on each occurrence, identically or differently, H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C=C-, Si(R³)₂, Ge(R³)₂, Sn(R³)₂, C=O, C=S, C=Se, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I, CN or NO₂, or an aromatic or heteroaromatic ring system having 5 to 60 aromatic ring atoms, which may in each case be substituted by one or more radicals R³, an aryloxy or heteroaryloxy group having 5 to 60 aromatic ring atoms, which may be substituted by one or more radicals R³, or an aralkyl or heteroaralkyl group having 5 to 60 aromatic ring atoms, which may be substituted by one or more radicals R and where, in addition, individual H atoms and/or CH₂ groups in the alkyl group may be replaced by the above-mentioned groups, where two or more radicals R² may be linked to one another and may form a ring or a ring system;
R³ is on each occurrence, identically or differently, H, D, F or an aliphatic, aromatic and/or heteroaromatic organic radical having 1 to 20 C atoms, in which, in addition, one or more H atoms may be replaced by D or F; two or more substituents R³ here may also be linked to one another and form a ring or a ring system;
n is on each occurrence, identically or differently, 0 or 1, where, in the case n=0, the group in brackets is not present and optionally groups R¹ are instead bonded to the central benzene ring;
p is equal to 2, 3, 4, 5 or 6; and
where the group Y and the nitrogen atom may be bonded to the aromatic six-membered ring at any desired adjacent positions;
where, in formula (II), the moieties in square brackets with index p which are bonded to L may be identical or different; and
where, in formula (II), the group L may be bonded at any desired position of the moiety in square brackets with index p.

2. Electronic device according to Claim 1, **characterised in that** p is equal to 2.

3. Electronic device according to Claim 1 or 2, **characterised in that** the sum of the values for n in formula (I) and the sum of the values for n per unit in square brackets with index p in formula (II) is equal to 0.

4. Electronic device according to one or more of Claims 1 to 3, **characterised in that** L is selected from a single bond, where in this case p = 2, or from C=O, NR¹, O or S, where in these cases p=2, or from alkylene groups having 1 to 10 C atoms, alkenylene groups having 2 to 10 C atoms, where one or more CH₂ groups in the said groups may be replaced by C=O, NR¹, P(=O)(R¹), O or S, and arylene or heteroarylene groups having 5 to 20 aromatic ring atoms, which may be substituted by one or more radicals R¹, or from divalent aromatic or heteroaromatic ring systems of the formula (L-1) 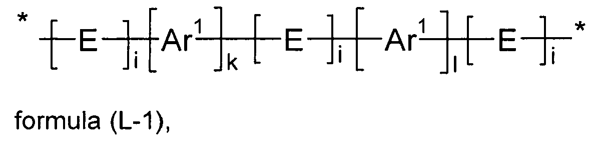 where p in this case is equal to 2 and where furthermore:
Ar¹ is on each occurrence, identically or differently, an aryl or heteroaryl group having 5 to 20 aromatic ring atoms, which may in each case be substituted by one or more radicals R¹;
E is on each occurrence, identically or differently, a single bond, C=O, NAr¹, P(=O)(R¹), O, S, SO or SO₂;
i is on each occurrence, identically or differently, 0 or 1;
k,l are on each occurrence, identically or differently, 0, 1, 2 or 3, where the sum of the values of k and l is greater than 0; and
where furthermore the groups Ar¹ may be connected to one another via one or more divalent groups T, where
T is selected on each occurrence, identically or differently, from a single bond, BR¹, C(R¹)₂, C=O, C=S, C=NR¹, C=C(R¹)₂, CR¹=CR¹, Si(R¹)₂, NR¹, PR¹, P(=O)R¹, O, S, S=O and S(=O)₂; and
the symbols * mark bonds from the group L to the remainder of the compound.

5. Electronic device according to one or more of Claims 1 to 4, **characterised in that** the compound of the formula (I) is selected from the formulae 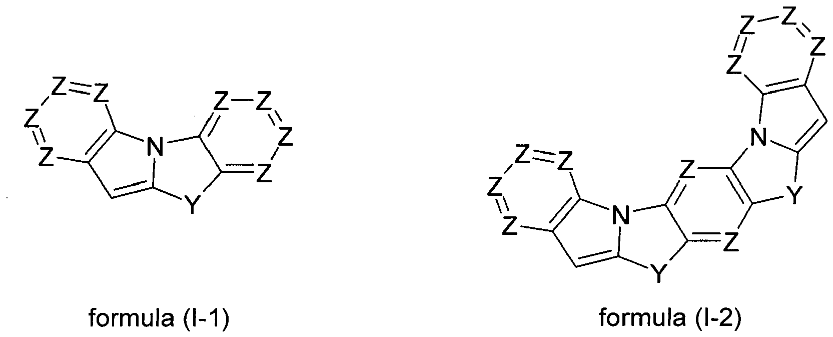 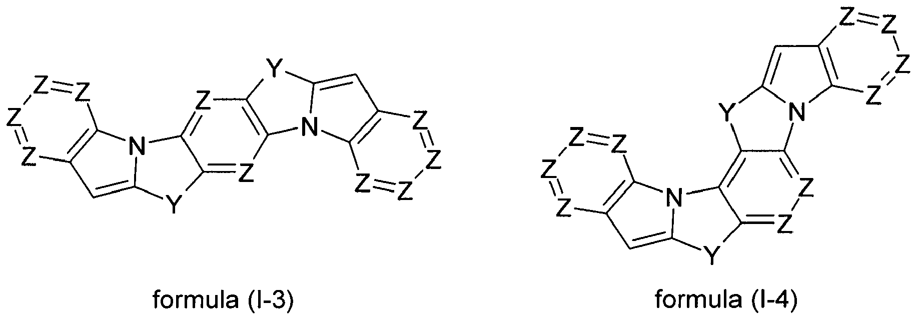 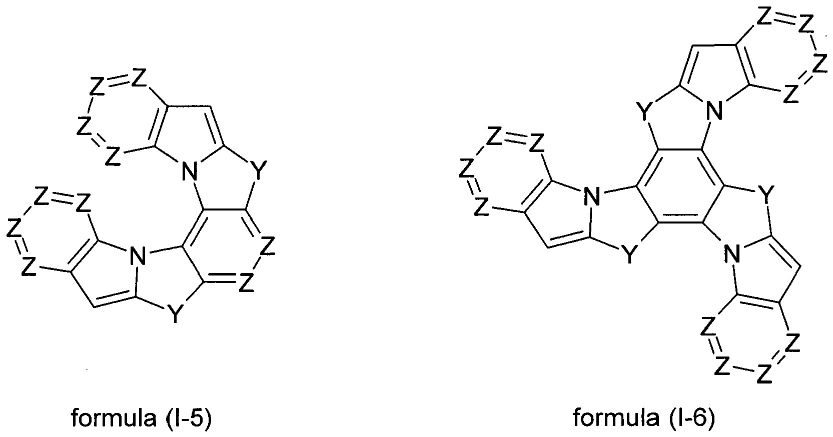 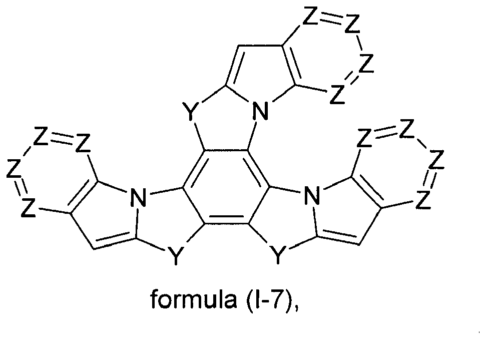 where the symbols occurring are as defined in Claim 1 and furthermore the compounds may be substituted by radicals R¹ at all unsubstituted positions.

6. Electronic device according to one or more of Claims 1 to 5, **characterised in that** the compound of the formula (II) is selected from the formulae 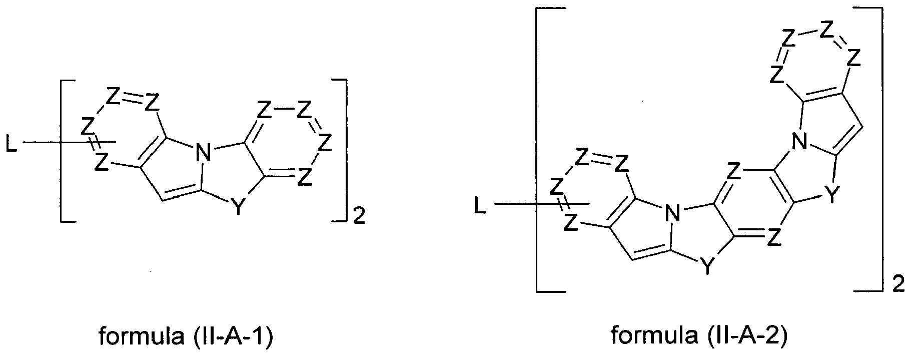 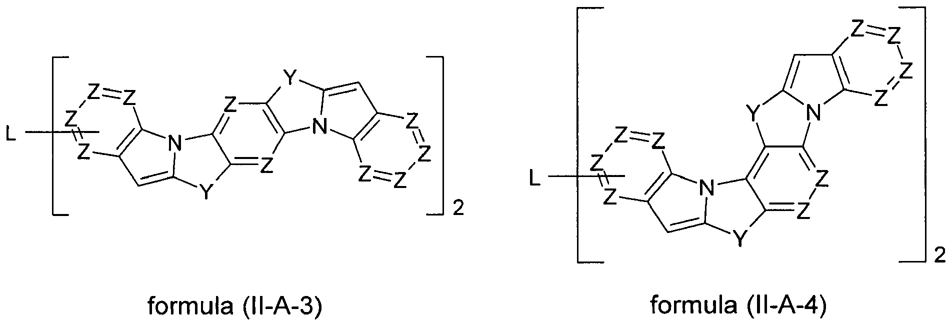 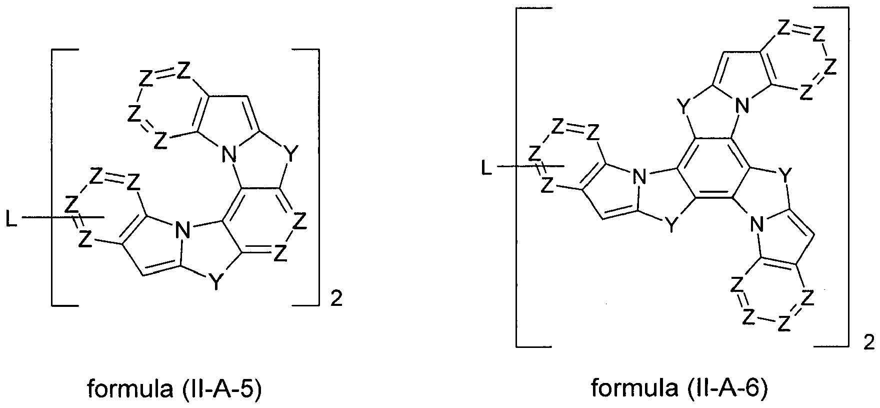 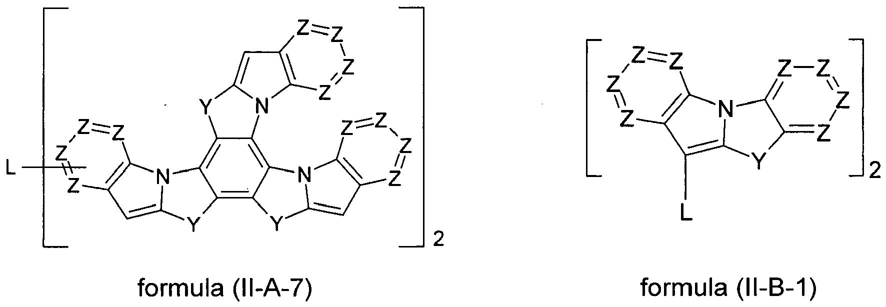 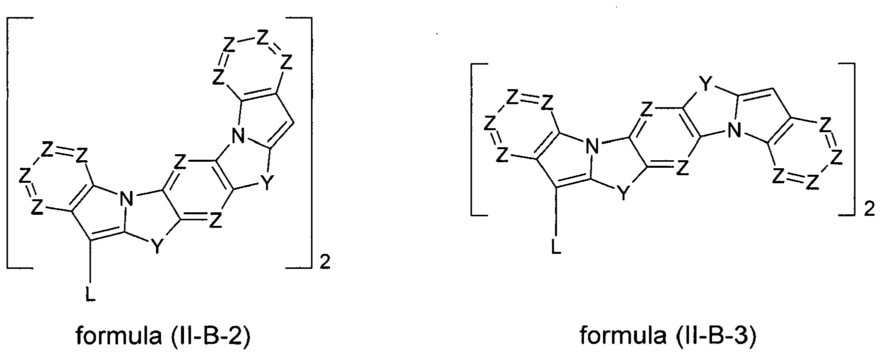 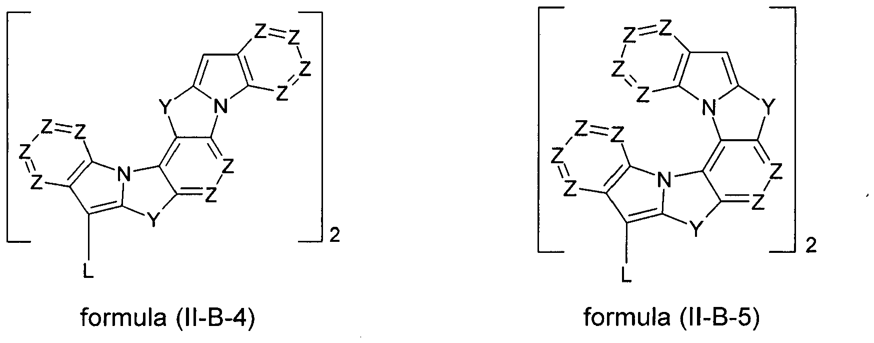 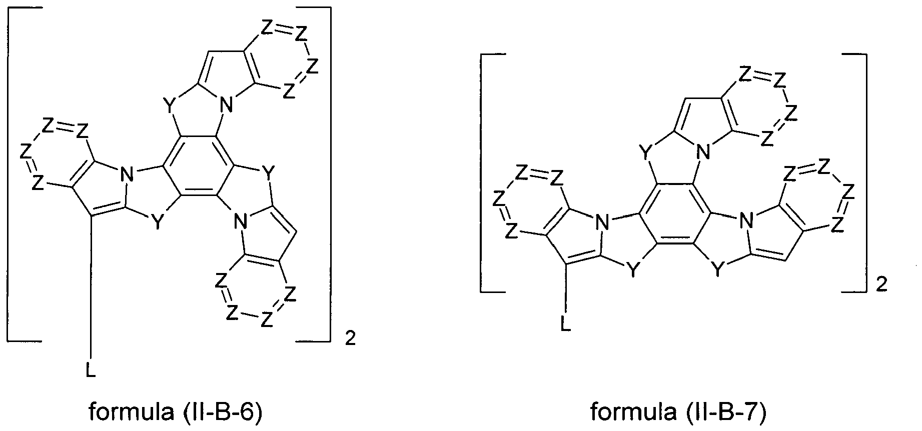 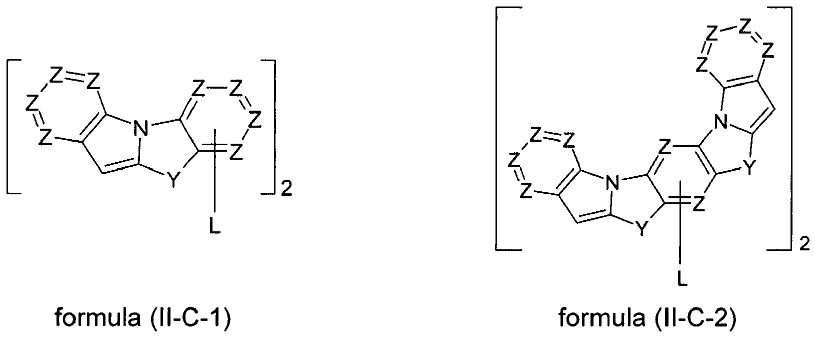 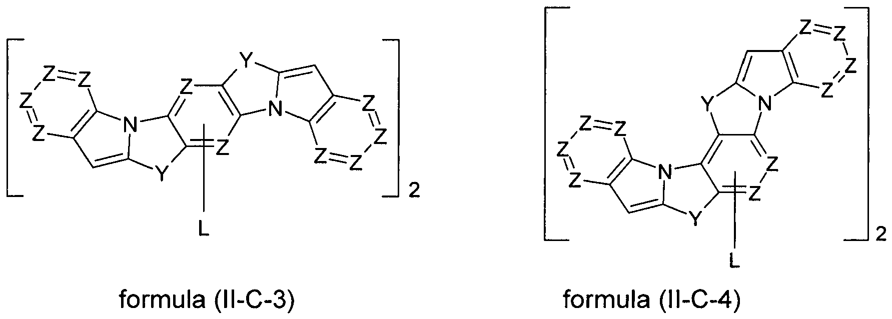 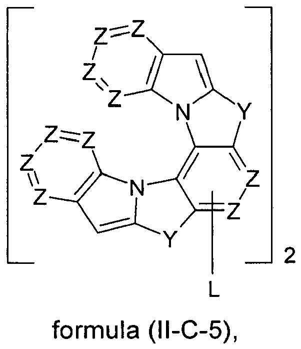 where the symbols occurring are as defined in Claim 1 and the group Z to which the group L is bonded stands for a carbon atom, and where furthermore the compounds may be substituted by radicals R¹ at all unsubstituted positions.

7. Electronic device according to one or more of Claims 1 to 6, **characterised in that** the device is selected from organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs), organic laser diodes (O-lasers) and organic electroluminescent devices (OLEDs).

8. Electronic device according to one or more of Claims 1 to 7, selected from organic electroluminescent devices, **characterised in that** the compound of the formula (I) or (II) is present as hole-transport material in a hole-transport layer or hole-injection layer and/or is present as matrix material in an emitting layer and/or is present as electron-transport material in an electron-transport layer.

9. Compound of the formula (I) or (II) of Claim 1, where the symbols and indices occurring are as defined in Claim 1, and the following compounds are excluded:

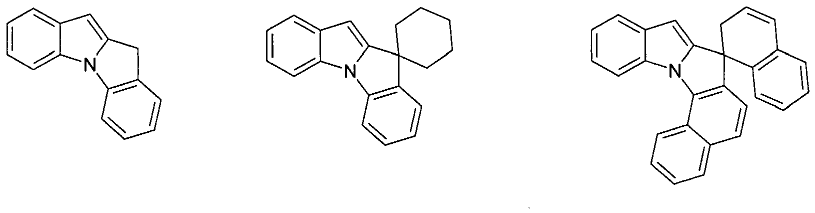

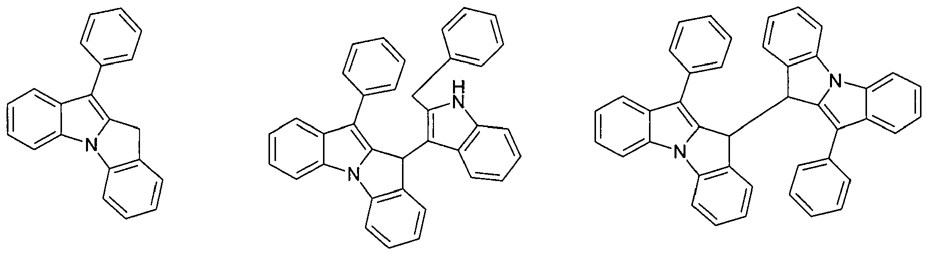

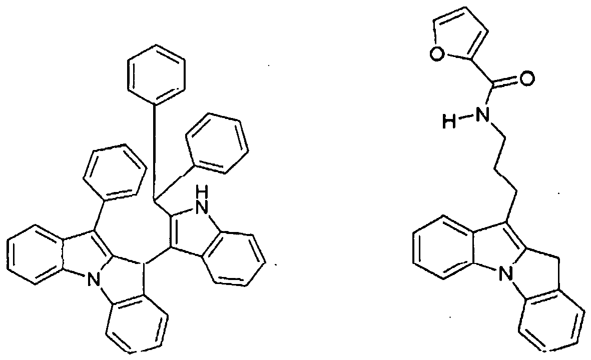

10. Oligomer, polymer or dendrimer containing one or more compounds according to Claim 9, where the bond(s) to the polymer, oligomer or dendrimer may be localised at any desired positions in formula (I) or (II) that are substituted by R¹.

11. Formulation comprising at least one compound according to Claim 9 or at least one polymer, oligomer or dendrimer according to Claim 10 and at least one solvent.

## Revendications

1. Dispositif électronique, comprenant une anode, une cathode et au moins une couche organique, dans lequel la couche organique comprend au moins un composé de la formule (I) ou (II) : 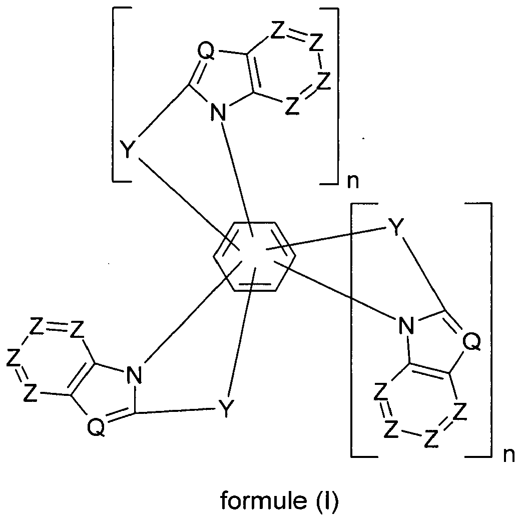 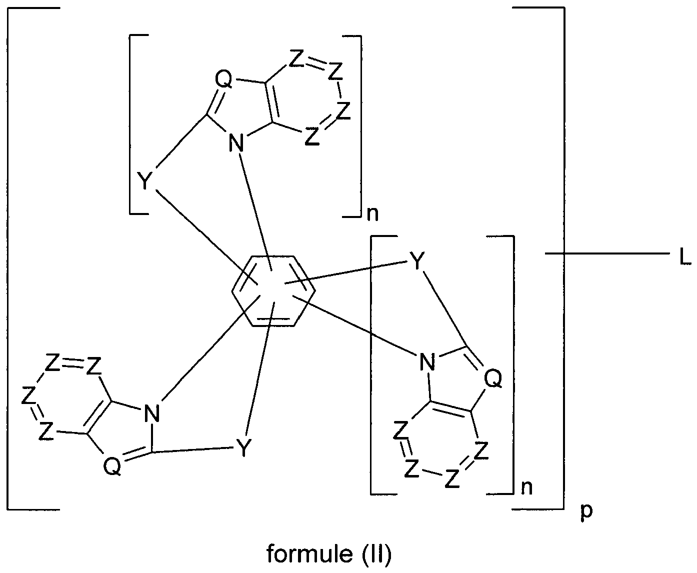 dans lesquelles :
Y est égal à C(R¹)₂;
Q, Z sont égaux à CR^{1;}
L est choisi parmi C=O, C=NR¹, Si(R¹)₂, NR¹, P(=O)(R¹), O, S, SO, SO₂, des groups alkylène comportant 1 à 20 atome(s) de C ou des groupes alkénylène ou alkynylène comportant 2 à 20 atomes de C, où un ou plusieurs groupe(s) CH₂ dans lesdits groupes peut/peuvent être remplacé(s) par Si(R¹)₂, O, S, C=O, C=NR¹, C(=O)O, (C=O)NR¹, NR¹, P(=O)(R¹), SO ou SO₂ et où un ou plusieurs atome(s) de H dans lesdits groupes peut/peuvent être remplacé(s) par D, F, Cl, Br, I, CN ou NO₂, et des systèmes de cycle aromatique ou hétéro-aromatique comportant 5 à 60 atomes de cycle aromatique, lesquels peuvent dans chaque cas être substitués par un radical ou plusieurs radicaux R¹, et n'importe quelles combinaisons souhaitées de 1, 2, 3, 4 ou 5 groupes identiques ou différents choisis parmi les groupes mentionnés ci avant; ou L est une liaison simple, où p dans ce cas est égal à 2 ;
R¹ est, pour chaque occurrence, de manière identique ou différente, H, D, F, Cl, Br, I, B(OR²)₂, CHO, C(=O)R², CR²=C(R²)₂, CN, C(=O)OR², C(=O)N(R²)₂, Si(R²)₃, N(R²)₂, NO₂, P(=O)(R²)₂, OSO₂R², OR², S(=O)R², S(=O)₂R², un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R² et où un ou plusieurs groupe(s) CH₂ dans les groupes mentionnés ci avant peut/peuvent être remplacé(s) par -R²C=CR²-, -C≡C-, Si(R²)₂, Ge(R²)₂, Sn(R²)₂, C=O, C=S, C=Se, C=NR², -C(=O)O-, -C(=O)NR²-, NR, P(=O)(R²), -O-, -S-, SO ou SO₂ et où un ou plusieurs atome(s) de H dans les groupes mentionnés ci avant peut/peuvent être remplacé(s) par D, F, Cl, Br, I, CN ou NO₂, ou un système de cycle aromatique ou hétéroaromatique comportant 5 à 60 atomes de cycle aromatique, lequel peut dans chaque cas être substitué par un radical ou plusieurs radicaux R², un groupe aryloxy ou hétéroaryloxy comportant 5 à 60 atomes de cycle aromatique, lequel peut être substitué par un radical ou plusieurs radicaux R², ou un groupe aralkyle ou hétéroaralkyle comportant 5 à 60 atomes de cycle aromatique, lequel peut être substitué par un radical ou plusieurs radicaux R² et où, en outre, des atomes de H individuels et/ou des groupes CH₂ individuels dans le groupe alkyle peuvent être remplacés par les groupes mentionnés ci avant, où deux radicaux R¹ ou plus peuvent être liés l'un à l'autre ou les uns aux autres et peuvent former un cycle ou un système de cycle ;
R² est, pour chaque occurrence, de manière identique ou différente, H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R³ et où un ou plusieurs groupe(s) CH₂ dans les groupes mentionnés ci avant peut/peuvent être remplacé(s) par -R³C=CR³-, -C≡C-, Si(R³)₂, Ge(R³)₂, Sn(R³)₂, C=O, C=S, C=Se, C=NR³, -C(=O)O-, -C(=O)NR³-, NR³, P(=O)(R³), -O-, -S-, SO ou SO₂ et où un ou plusieurs atome(s) de H dans les groupes mentionnés ci avant peut/peuvent être remplacé(s) par D, F, Cl, Br, I, CN ou NO₂, ou un système de cycle aromatique ou hétéroaromatique comportant 5 à 60 atomes de cycle aromatique, lequel peut dans chaque cas être substitué par un radical ou plusieurs radicaux R³, un groupe aryloxy ou hétéroaryloxy comportant 5 à 60 atomes de cycle aromatique, lequel peut être substitué par un radical ou plusieurs radicaux R³, ou un groupe aralkyle ou hétéroaralkyle comportant 5 à 60 atomes de cycle aromatique, lequel peut être substitué par un radical ou plusieurs radicaux R et où, en outre, des atomes de H individuels et/ou des groupes CH₂ individuels dans le groupe alkyle peuvent être remplacés par les groupes mentionnés ci avant, où deux radicaux R² ou plus peuvent être liés l'un à l'autre ou les uns aux autres et peuvent former un cycle ou un système de cycle ;
R³ est, pour chaque occurrence, de manière identique ou différente, H, D, F ou un radical aliphatique, aromatique et/ou hétéroaromatique comportant 1 à 20 atome(s) de C, où, en outre, un ou plusieurs atome(s) de H peut/peuvent être remplacé(s) par D ou F ; deux substituants R³ ou plus peuvent ici également être liés l'un à l'autre ou les uns aux autres et former un cycle ou un système de cycle ;
n est, pour chaque occurrence, de manière identique ou différente, 0 ou 1, où, dans le cas n=0, le groupe entre crochets n'est pas présent et en option, des groupes R¹ sont en lieu et place reliés au cycle benzène central ;
p est égal à 2, 3, 4, 5 ou 6 ; et
où le groupe Y et l'atome d'azote peuvent être reliés au cycle aromatique à six éléments au niveau de n'importe quelles positions adjacentes souhaitées ;
où, dans la formule (II), les moitiés entre crochets avec l'indice p qui sont reliées à L peuvent être identiques ou différentes ; et
où, dans la formule (II), le groupe L peut être relié au niveau de n'importe quelle position souhaitée de la moitié entre crochets avec l'indice p.

2. Dispositif électronique selon la revendication 1, **caractérisé en ce que** p est égal à 2.

3. Dispositif électronique selon la revendication 1 ou 2, **caractérisé en ce que** la somme des valeurs pour n dans la formule (I) et la somme des valeurs pour n par unité entre crochets avec l'indice p dans la formule (II) sont égales à 0.

4. Dispositif électronique selon une ou plusieurs des revendications 1 à 3, **caractérisé en ce que** L est choisi parmi une liaison simple, où dans ce cas p = 2, ou parmi C=O, NR¹, O ou S, où dans ces cas p=2, ou parmi des groupes alkylène comportant 1 à 10 atome(s) de C, des groupes alkénylène comportant 2 à 10 atomes de C, où un ou plusieurs groupe(s) CH₂ dans lesdits groupes peut/peuvent être remplacé(s) par C=O, NR¹, P(=O)(R¹), O ou S, et des groupes arylène ou hétéroarylène comportant 5 à 20 atomes de cycle aromatique, lesquels peuvent être substitués par un radical ou plusieurs radicaux R¹, ou parmi des systèmes de cycle aromatique ou hétéroaromatique divalents de la formule (L-1) : 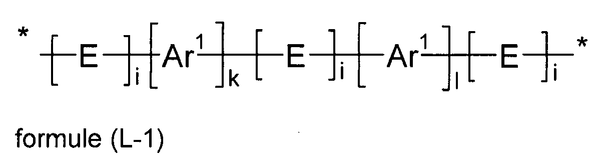 où p dans ce cas est égal à 2 et où en outre :
Ar¹ est, pour chaque occurrence, de manière identique ou différente, un groupe aryle ou hétéroaryle comportant 5 à 20 atomes de cycle aromatique, lequel peut dans chaque cas être substitué par un radical ou plusieurs radicaux R^{1 ;}
E est, pour chaque occurrence, de manière identique ou différente, une liaison simple, C=O, NAr¹, P(=O)(R¹), O, S, SO ou SO₂ ;
i est, pour chaque occurrence, de manière identique ou différente, 0 ou 1 ;
k, l sont, pour chaque occurrence, de manière identique ou différente, 0, 1, 2 ou 3, où la somme des valeurs de k et de l est supérieure à 0 ; et
où en outre les groupes Ar¹ peuvent être connectés les uns aux autres via un ou plusieurs groupe(s) divalent(s) T, où
T est choisi, pour chaque occurrence, de manière identique ou différente, parmi une liaison simple, BR¹, C(R¹)₂, C=O, C=S, C=NR¹, C=C(R¹)₂, CR¹=CR¹, Si(R¹)₂, NR¹, PR¹, P(=O)R¹, O, S, S=O et S(=O)₂ ; et
les symboles * identifient des liens depuis le groupe L jusqu'au reste du composé.

5. Dispositif électronique selon une ou plusieurs des revendications 1 à 4, **caractérisé en ce que** le composé de la formule (I) est choisi parmi les formules : 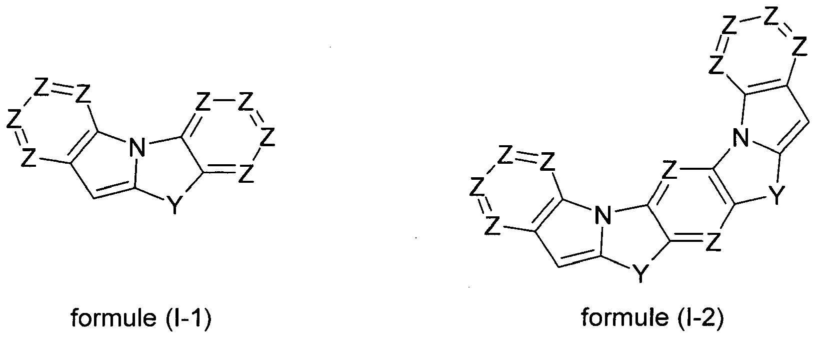 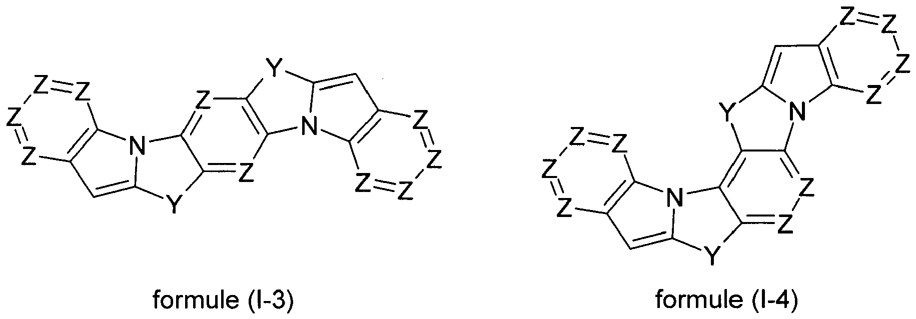 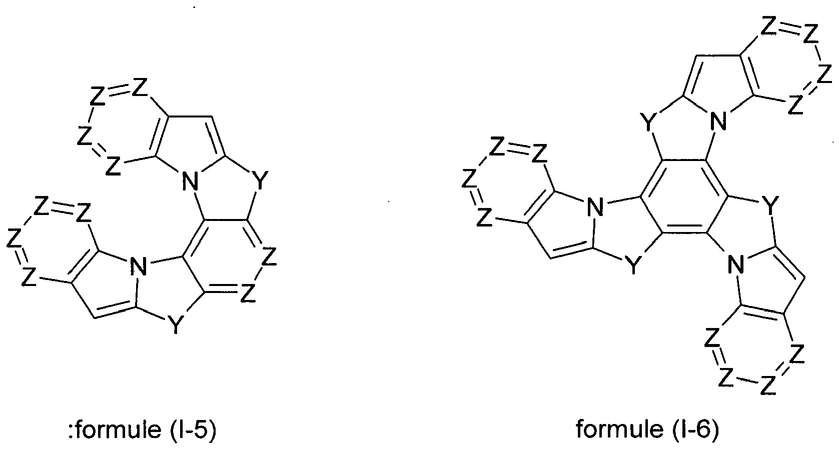 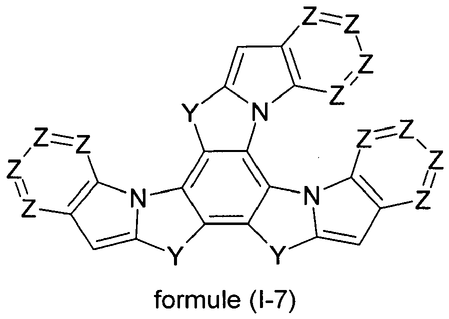 où les symboles rencontrés sont comme défini selon la revendication 1 et en outre, les composés peuvent être substitués par des radicaux R¹ au niveau de toutes les positions non substituées.

6. Dispositif électronique selon une ou plusieurs des revendications 1 à 5, **caractérisé en ce que** le composé de la formule (II) est choisi parmi les formules : 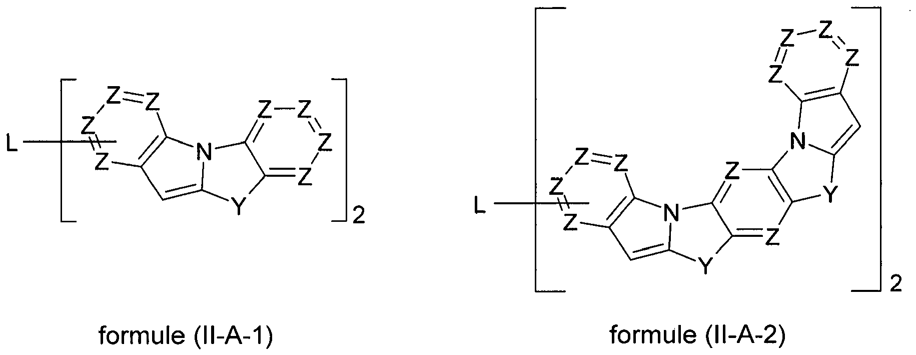 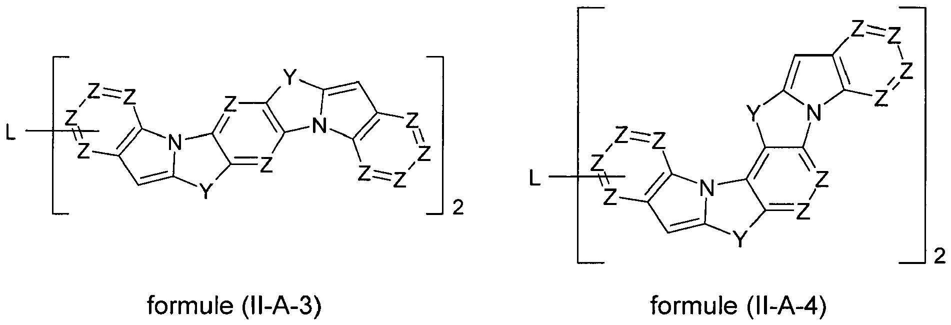 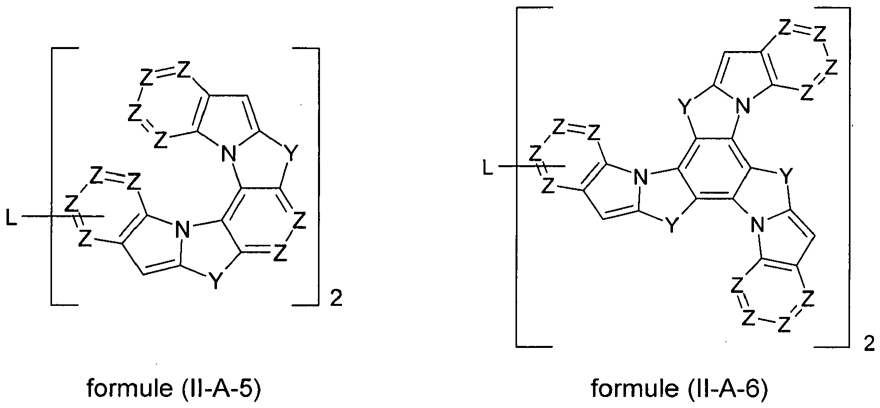 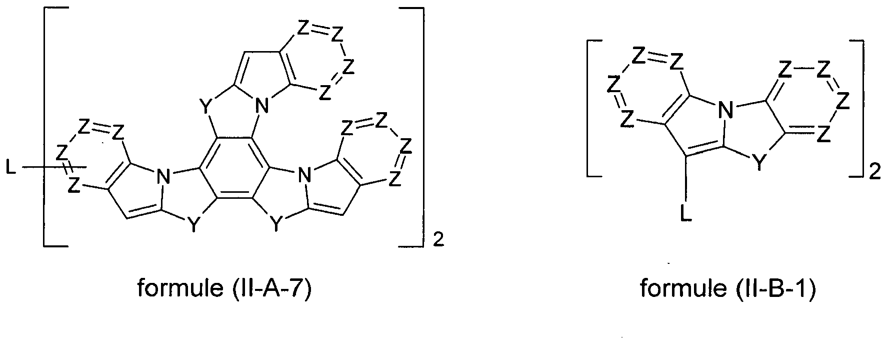 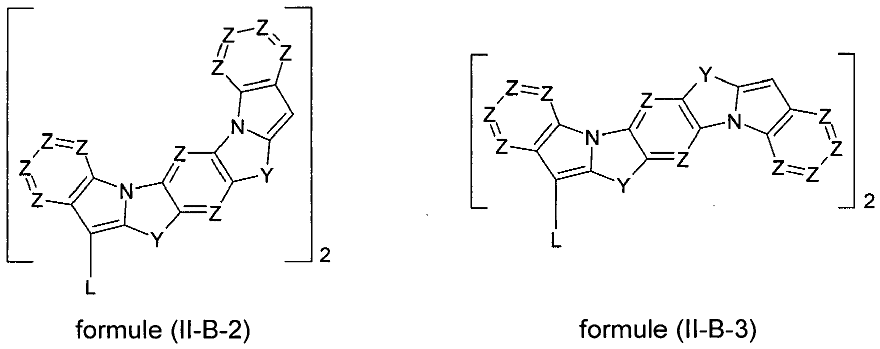 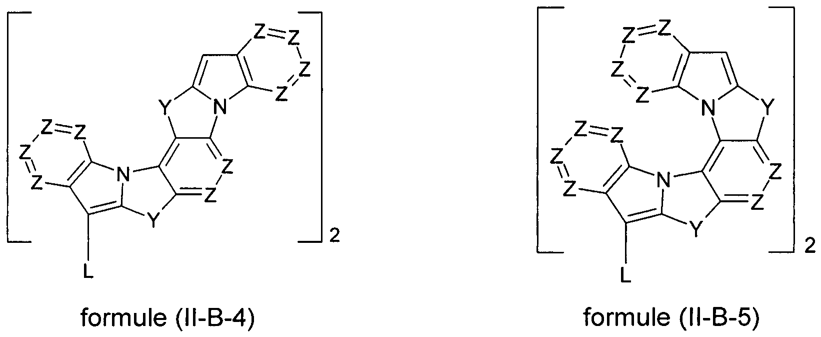 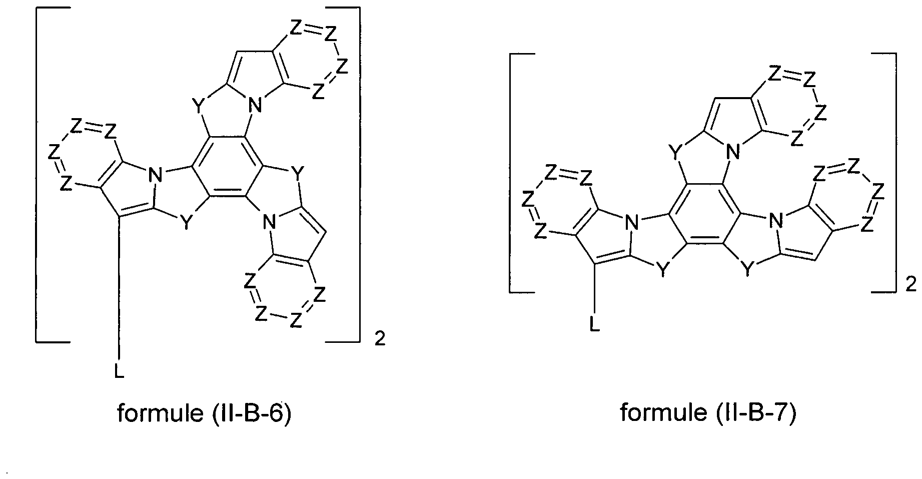 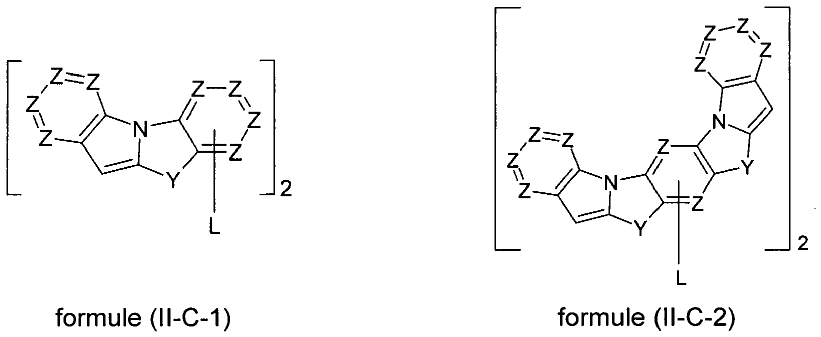 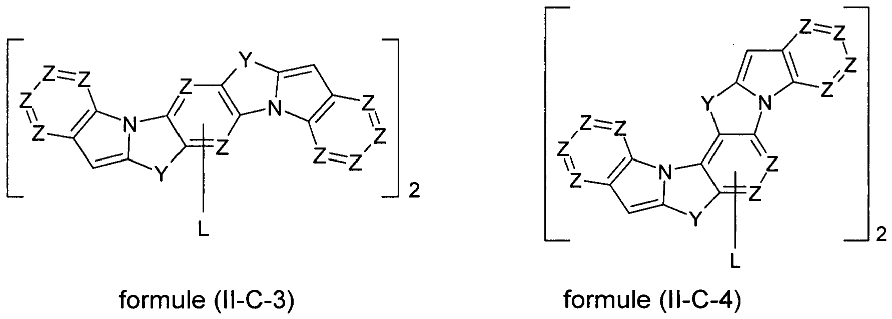 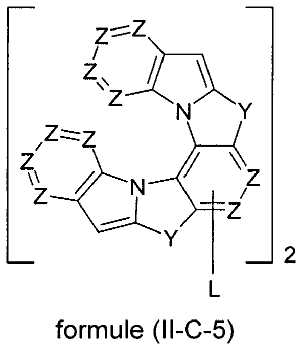 dans lesquelles les symboles rencontrés sont comme défini selon la revendication 1 et le groupe Z auquel le groupe L est relié représente un atome de carbone, et où en outre les composés peuvent être substitués par des radicaux R¹ au niveau de toutes les positions non substituées.

7. Dispositif électronique selon une ou plusieurs des revendications 1 à 6, **caractérisé en ce que** le dispositif est choisi parmi des circuits intégrés organiques (O-IC), des transistors à effet de champ organiques (O-FET), des transistors à film mince organiques (O-TFT), des transistors à émission de lumière organiques (O-LET), des cellules solaires organiques (O-SC), des détecteurs optiques organiques, des photorécepteurs organiques, des dispositifs à extinction de champ organiques (O-FQD), des cellules électrochimiques à émission de lumière (LEC), des diodes laser organiques (O-laser) et des dispositifs électroluminescents organiques (OLED).

8. Dispositif électronique selon une ou plusieurs des revendications 1 à 7, choisi parmi des dispositifs électroluminescents organiques, **caractérisé en ce que** le composé de la formule (I) ou (II) est présent en tant que matériau de transport de trous dans une couche de transport de trous ou une couche d'injection de trous et/ou est présent en tant que matériau de matrice dans une couche d'émission et/ou est présent en tant que matériau de transport d'électrons dans une couche de transport d'électrons.

9. Composé de la formule (I) ou (II) selon la revendication 1, dans lequel les symboles et indices rencontrés sont comme défini selon la revendication 1, et les composés qui suivent sont exclus :

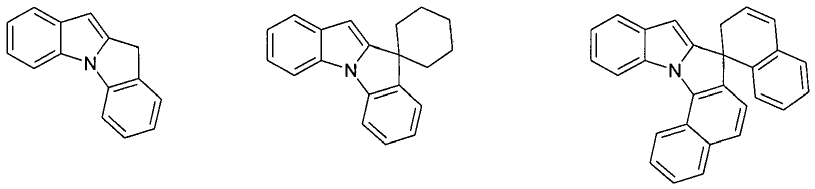

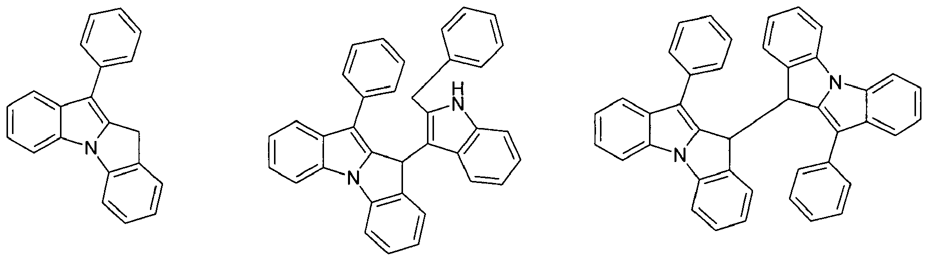

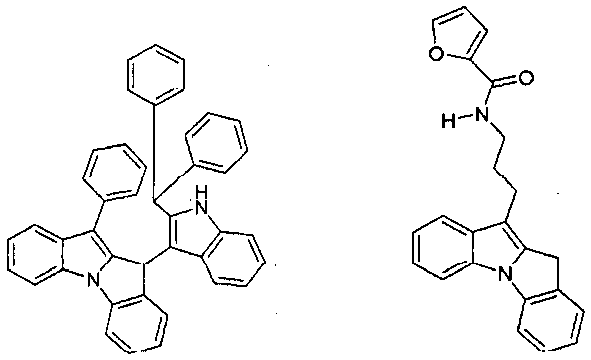

10. Oligomère, polymère ou dendrimère contenant un ou plusieurs composés selon la revendication 9, où le/les lien(s) sur le polymère, l'oligomère ou le dendrimère peut/peuvent être localisé(s) au niveau de n'importe quelles position(s souhaitées dans la formule (I) ou (II) qui sont substituées par R¹.

11. Formulation comprenant au moins un composé selon la revendication 9 ou au moins un polymère, un oligomère ou un dendrimère selon la revendication 10 et au moins un solvant.
